(19) Europäisches Patentamt
European Patent Office
Office européen des brevets

(11) EP 4 647 428 A1

(12) # EUROPEAN PATENT APPLICATION

published in accordance with Art. 153(4) EPC

(43) Date of publication:
**12.11.2025 Bulletin 2025/46**

(21) Application number: **24738574.3**

(22) Date of filing: **05.01.2024**

(51) International Patent Classification (IPC):
***C07D 401/12*** (2006.01) ***C07D 211/22*** (2006.01)
***C07D 211/96*** (2006.01) ***A61K 31/44*** (2006.01)
***A61K 31/454*** (2006.01) ***A61P 35/00*** (2006.01)
***A61P 11/00*** (2006.01) ***A61P 1/16*** (2006.01)

(52) Cooperative Patent Classification (CPC):
**A61K 31/44; A61K 31/454; A61P 1/16; A61P 11/00; A61P 35/00; C07D 211/22; C07D 211/96; C07D 401/12**

(86) International application number:
**PCT/CN2024/070917**

(87) International publication number:
**WO 2024/146643 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.01.2023 CN 202310015931**
**05.01.2023 CN 202310015933**

(71) Applicant: **Jiangsu Mingsheng Jutai Biotechnology Co. Ltd**
**Nantong, Jiangsu 226126 (CN)**

(72) Inventor: **ZHU, Wenhua**
**Nantong, Jiangsu 226126 (CN)**

(74) Representative: **Lavoix**
**Bayerstraße 83**
**80335 München (DE)**

(54) **COMPOUND CONTAINING 2-METHYL-2-(3-(PIPERIDIN-3-YL) PHENOXY)-2-METHYLPROPANAMIDE SKELETON**

(57) Provided in the present invention is a compound containing a 2-methyl-2-(3-(piperidin-3-yl)phenoxy)-2-methylpropanamide skeleton. Specifically, provided in the present invention is a compound of formula I or formula II, or a pharmaceutically acceptable salt thereof. The compound of formula I or formula II of the present invention has an excellent ability to inhibit BCL9/β-catenin interaction.

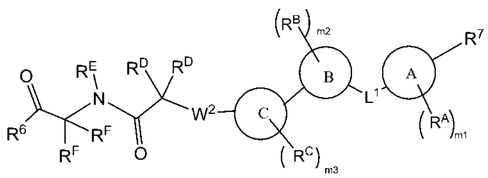

(I)

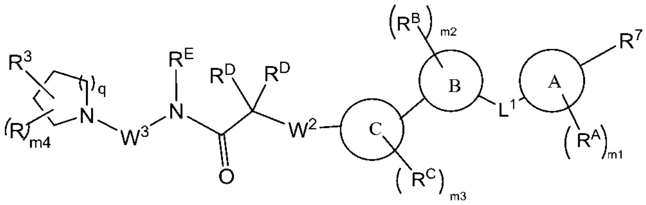

(II)

EP 4 647 428 A1

## Description

### Technical Field

The present invention belongs to the field of medicine, and specifically relates to a compound containing a 2-methyl-2-(3-(piperidin-3-yl)phenoxy)-2-methylpropionamide skeleton and its use in targeting the BCL9 (B-cell lymphoma 9)/β-catenin interaction .

### BACKGROUND

Wnt /β-catenin signaling is crucial for normal embryonic development and throughout life. Furthermore, aberrant Wnt signaling is associated with various diseases, particularly cancer. Recent studies have shown that directly targeting the β-catenin/B-cell lymphoma 9 (BCL9) protein-protein interaction (PPI) is a promising strategy for blocking the Wnt pathway. Advances in understanding the co-crystal complex and mechanism of action of the β-catenin/BCL9 interaction have facilitated the discovery of inhibitors, but only a few inhibitors have been reported.

Canonical Wnt signaling is a highly conserved developmental signaling pathway that regulates cell proliferation, differentiation, and survival. β-catenin is generally considered a key effector of Wnt signaling. In the absence of Wnt activation ( Wntoff ), the cytoplasmic pool of β-catenin binds to glycogen synthase kinase 3β (GSK3β), casein kinase 1α (CK1α), the scaffolding protein AXIN, and the tumor suppressor adenomatous polyposis coli (APC), regulating phosphorylation, followed by proteasomal degradation of β-catenin. β-catenin recruits coactivators, including BCL9 or B-cell lymphoma 9-like (B9L), Pygo , and CREB-binding protein (CBP), to promote the transcription of cell proliferation, migration, and survival genes, such as cyclin D1, c- Myc , survivin, and LEF1. These Wnt target genes are closely associated with the development and progression of many types of cancer , including colorectal cancer, breast cancer, lung cancer, hepatocellular carcinoma, leukemia, and multiple myeloma.

Advances in using the β-catenin/BCL9 complex, along with advances in reliable biochemical assays and drug discovery strategies, have provided further insights into the interaction, potentially leading to the discovery of new anticancer drugs. To date, several different types of β-catenin/BCL9 PPI inhibitors have been reported. These can be broadly categorized into two main groups: peptide inhibitors and non-peptide small molecule inhibitors. However, the search for β-catenin/BCL9 PPI inhibitors, particularly non-peptide small molecule inhibitors, remains in its early stages of research.

In summary, there is an urgent need in this field to develop a small molecule compound that targets the BCL9 (B-cell lymphoma 9)/β-catenin interaction with high targeting affinity and good cell membrane permeability.

### SUMMARY OF THE INVENTION

The purpose of the present invention is to provide a new class of small molecule compounds that target the BCL9/β-catenin interaction.

In the first aspect of the present invention, a compound or a pharmaceutically acceptable salt thereof, or an isomer, solvate, crystal form or prodrug thereof is provided, wherein the compound is as shown in Formula I

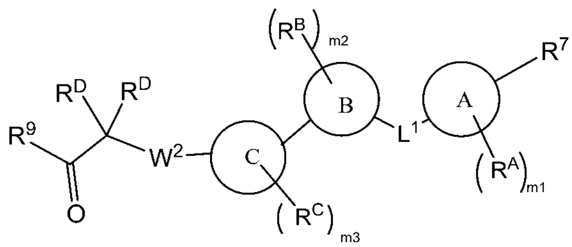

(I)

wherein

$R^9$ is

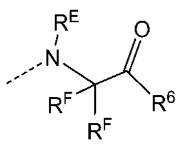

or

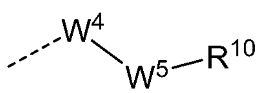

;

$W^4$ and $W^5$ are each independently selected from the group consisting of none, -O-, -S-, -C(O)-, -S(O)-, $-S(O)_2-$, $-N(R^{s1})-$, $-C(R^{s2})_2-$;
$R^{10}$ is H, OH, $R^6$, or is unsubstituted or substituted with one or more $R^H$ and is selected from the group consisting of $C_{1-6}$alkyl, C3-10 cycloalkyl, 4- to 10-membered heterocycloalkyl, C3-10 cycloalkenyl, 4- to 10-membered heterocycloalkenyl, $C_{6-10}$ aryl, and 5- to 10-membered heteroaryl;
RH is selected from the group consisting of R, optionally substituted $C_{1-6}$haloalkyl ;
$R^E$ is selected from the group consisting of H, optionally substituted $C_{1-4}$alkyl, optionally substituted C3-10 cycloalkyl, and optionally substituted 4- to 10-membered heterocycloalkyl;
RF are each independently selected from the group consisting of H, optionally substituted $C_{1-4}$ alkyl , optionally substituted $_C$3-10 cycloalkyl, optionally substituted 4 to 10 membered heterocycloalkyl; or two RFs and the carbon atom to which they are attached together form an optionally substituted C3-6 cycloalkyl or an optionally substituted 4 to 6 membered heterocyclyl;
$R^6$ is an optionally substituted group selected from the group consisting of $-OR^2$, $C_{3-12}$ cycloalkyl, a 4- to 10-membered heterocycloalkyl group connected to the rest of the group via a carbon atom on the ring, and $-NR^4$ $R^5$ ;
$R^2$ is selected from the group consisting of H, optionally substituted $C_{1-6}$alkyl, optionally substituted C3-10 cycloalkyl, optionally substituted 4 to 8-membered heterocycloalkyl, optionally substituted $C_{6-10}$ aryl, optionally substituted 5 to 10-membered heteroaryl, optionally substituted C3-10 cycloalkenyl, optionally substituted 4 to 10-membered heterocycloalkenyl;
R4 and R5 are each independently H, $_{C1-6}$ alkyl, C3-10 cycloalkyl, $_{4\text{- to }8\text{-}}$ membered heterocycloalkyl, $C_{6-10}$ aryl, 5- to 10-membered heteroaryl, C3-10 cycloalkenyl, or 4- to 10-membered heterocycloalkenyl, which is optionally substituted or substituted by one or more (e.g., 1, 2, or 3) R3 ; or , $^{R4}$ and R5, together with the nitrogen atom to which they are attached, form a ring optionally substituted or substituted by one or more (e.g., 1, 2, or 3) R3, which is selected from the group consisting of 4- to 10-membered heterocycloalkyl , 4- $_{to}$ 10 $^-$ membered heterocycloalkenyl, or 5- to 10-membered heteroaryl.
$R^3$ is each independently selected from the group consisting of R, $-OR^{31}$ , $-C_{1-4}$alkylene-$OR^{31}$ , $-N(R^{32})R^{33}$, $-C_{1-4}$alkylene-$N(R^{31})R^{32}$;
$R^{31}$ is selected from the group consisting of H, optionally substituted $C_{1-4}$alkyl, $R^{34}$, $-C_{1-4}$alkylene-$R^{34}$ ;
$R^{32}$ is selected from the group consisting of H, optionally substituted $C_{1-4}$alkyl;
$R^{33}$ is selected from the group consisting of H, optionally substituted $C_{1-4}$alkyl, $R^{34}$ , $-C_{1-4}$alkylene-$R^{34}$ ;
$R^{34}$ is selected from the group consisting of C3-10 cycloalkyl, 4- to 8-membered heterocycloalkyl, $C_{6-10}$ aryl, 5- to 10-membered heteroaryl, C3-10 cycloalkenyl, and 4- to 10-membered heterocycloalkenyl; wherein the cycloalkyl, heterocycloalkyl, aryl, heteroaryl, cycloalkenyl, and heterocycloalkenyl groups are optionally substituted with one or more groups selected from the group consisting of $-NH_2$ , R;
$R^D$ are each independently selected from the group consisting of H, optionally substituted $C_{1-4}$alkyl; or, two $R^D{}_S$ and the carbon atom to which they are attached together form a group selected from the group consisting of optionally substituted C3-10 cycloalkyl, optionally substituted 4 to 10 membered heterocycloalkyl;
W2 is selected from the group consisting $^{of}$ -O-, -S-, and $-N(R^{s1})-$;
Ring C is an optionally substituted ring selected from the group consisting of $C_{6-10}$ aryl, and 5- to 10-membered heteroaryl;
m3=0, 1, 2, 3, or 4;
Each RC is independently $R^{C1}$ or $R^{s1}$ ;
Each $R^{C1}$ is independently selected from the group consisting of halogen, optionally substituted $C_{1-6}$alkyl, optionally substituted $C_{1-6}$haloalkyl, hydroxy, and optionally substituted $C_{1-6}$alkoxy, optionally substituted $C_{1-6}$haloalkoxy;
Ring B is an optionally substituted ring selected from the group consisting of $C_{3-12}$ cycloalkyl, 4-to 12-membered heterocycloalkyl, C3-10 cycloalkenyl, and 4- to 10-membered heterocycloalkyl;
Each RB is independently $R^{B1}$, $R^{s1}$ $_{or}$ $R^{s2}$ ;
each $R^{B1}$ is independently selected from the group consisting of halogen, hydroxy, cyano, optionally substituted $C_{1-6}$alkyl, optionally substituted $C_{1-6}$alkoxy, optionally substituted $C_{1-6}$alkylthio, optionally substituted C3-10 cycloalkyl, optionally substituted 4- to 10-membered heterocycloalkyl, optionally substituted C3-10 cycloalkenyl, optionally substituted 4- to 10-membered heterocycloalkenyl, optionally substituted $C_{6-10}$ aryl, and optionally substituted 5- to 10-membered heteroaryl;
m2=0, 1, 2, 3, or 4;
$L^1$ is $-X-(W^1)_{n1}-$;
X is selected from the group consisting of -C(O)-, -S(O)-, and $-S(O)_2-$;
Each $W^1$ is independently selected from the group consisting of none, -O-, -S-, -C(O)-, -S(O)-, $-S(O)_2-$, $-N(R^1)-$, $-N(R^{s1})-$, $-CH(R^8)-$, $-C(R^{s2})_2-$;
Subscript n1 = 0, 1, 2, or 3;
Ring A is an optionally substituted ring selected from the group consisting of $C_{6-10}$ aryl; a 5- to 10-membered

heteroaryl; a $C_{6-10\ aryl\ substituted\ by\ a}$ C3-10 cycloalkyl, a 4- to 10-membered heterocycloalkyl, a C3-10 cycloalkenyl, a 4-to 10-membered heterocycloalkenyl, a $C_{6-10}$ aryl or a 5-to 10-membered heteroaryl ; a 5- to 10-membered heteroaryl substituted by a C3-10 cycloalkyl, a 4- to 10-membered heterocycloalkyl, a C3-10 cycloalkenyl, a 4- to 10-membered heterocycloalkenyl, a $C_{6-10}$ aryl or a 5- to 10-membered heteroaryl; a $C_{6-10}$ aryl fused to $_{a}$ C3-10 cycloalkyl, a 4- to 10-membered heterocycloalkyl , $_{a}$ C3-10 cycloalkenyl, a 4- to 10-membered heterocycloalkenyl, a $C_{6-10}$ aryl or a 5- to 10-membered heteroaryl ; 3-10 -membered cycloalkyl, 4- to 10-membered heterocycloalkyl, C3-10 cycloalkenyl, 4- to 10-membered heterocycloalkenyl, $C_{6-10}$ aryl, or 5- to 10-membered heteroaryl-fused 5- to 10-membered heteroaryl;

m1=0, 1, 2, 3, or 4;

each $R^A$ is independently $R^{A1}$ , $R^{s1}$ or $R^{s2}$;

each $R^{A1}$ is independently selected from the group consisting of halogen, optionally substituted $C_{1-6}$alkyl, optionally substituted $C_{1-6}$haloalkyl, optionally substituted $C_{1-6}$alkoxy, optionally substituted $C_{1-6}$alkylthio, optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl; or, when two $R^{A1}$ are located on adjacent ring atoms, the two $R^{A1}$ and the ring atoms adjacent thereto together form a ring selected from the group consisting of optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl;

Each of $R^1$ and $R^8$ is independently selected from the group consisting of H, optionally substituted $C_{1-6}$alkyl, optionally substituted C $_{3-6}$ cycloalkyl, halogen, optionally substituted $C_{1-6}$haloalkyl, optionally substituted $C_{1-6alkoxy}$ , optionally substituted $C_{1-6}$haloalkoxy (-$OC_{1-6}$haloalkyl), optionally substituted $C_{1-6}$alkyl-$OC_{1-6}$alkylene, optionally substituted $C_{1-6}$haloalkyl-$OC_{1-6}$alkylene, optionally substituted $C_{1-6}$haloalkyl-$SC_{1-6}$alkylene, optionally substituted $C_{1-6}$aminoalkyl, optionally substituted C3-10 cycloalkyl, optionally substituted 4 to 10 membered heterocycloalkyl, optionally substituted $C_{6-10}$ aryl, optionally substituted 5 to 10 membered heteroaryl, optionally substituted C3-10 cycloalkenyl, optionally substituted 4 to 10 membered heterocycloalkenyl, optionally substituted C3-10 cycloalkyl-C $_{R1}$ or $^{R8}$, together with $R^{s1}$ or $R^{s2}$ on ring $_{A}$ , form $^{an}$ optionally substituted $_{C4-10}$ cycloalkyl or $_{4-10}$ heterocycloalkyl ;

$R^7$ is an optionally substituted group selected from the group consisting of none, $C_{1-6}$alkyl, C3-10 cycloalkyl, 4 to 10-membered heterocycloalkyl, C3-10 cycloalkenyl, 4 to 10-membered heterocycloalkenyl, $C_{6-10}$ aryl, and 5 to 10-membered heteroaryl;

$R^{s1}$ are each independently selected from the group consisting of H, optionally substituted $C_{1-4}$alkyl, optionally substituted C $_{3-6}$ cycloalkyl, and optionally substituted 4- to 6-membered heterocyclyl;

$R^{s2}$ are each independently selected from the following group: H, optionally substituted $C_{1-4}$alkyl, optionally substituted C $_{3-6}$ cycloalkyl, optionally substituted 4 to 6-membered heterocyclyl; or two $R^{s2}$ and the carbon atom to which they are attached together form an optionally substituted C $_{3-6}$ cycloalkyl or an optionally substituted 4 to 6-membered heterocyclyl;

Unless otherwise defined, the optional substitution refers to unsubstituted or one or more (such as 1, 2, 3 or 4) hydrogen atoms in the group are replaced by an optional Rsubstituent;

R is each independently selected from the group consistingof D, Halogen, C1-6-alkyl, C1-6-haloalkyl, C1-6-hydroxyalkyl, C2-6 vinyl, C2-6 alkynyl-CN, -OR', $-NO_2$, -NR'R", -SR', -OC(O)R', -C(O)R', $-CO_2R'$, -CONR', -OC(O)NR'R", -NR"C(O)R', -NR"-C(O)NR'R", $-NR"C(O)_2R'$ , -S(O)R', $-S(O)_2R'$, $-S(O)_2NR'R"$ , $NR"S(O)_2R'$, C3-10 cycloalkyl optionally substituted with one or more R'", 4 to 10 membered heterocycloalkyl optionally substituted with one or -$_{more\ R'''}$, C3-10 cycloalkyl optionally substituted with one or more R', C3-10 cycloalkyl optionally substituted with one or more R', C3-10 cycloalkyl optionally substituted with one or more R', C3-10 cycloalkyl optionally substituted with one or more R', -C3-10 cycloalkyl optionally substituted with one or more R' , C3-10 cycloalkyl optionally substituted with one or more R', C3-10 cycloalkyl optionally substituted with one or more R' -$C_{1-4}$alkylene-C3-10 cycloalkyl optionally substituted by one or more R'", -$C_{1-4}$alkylene- 4 to 10 -membered heterocycloalkyl optionally substituted by one or more R‴, -$C_{1-4}$alkylene-$C_{6-10}$ aryl optionally substituted by one or more R' ", -$C_{1-4}$alkylene - 5 to 10 -membered heteroaryl optionally substituted by one or more R'" ;

each R' is independently selected from the group consisting of H, D, C1-6 alkyl , C1-6 haloalkyl, C3-10 cycloalkyl optionally substituted with one or more R'", $_{4-}$ to 10-membered heterocycloalkyl optionally substituted with one or more R‴, $C_{6-10}$ aryl optionally substituted with one or more R‴, 5- to 10-membered heteroaryl optionally substituted with one or more R‴, -$C_{1-4}$ alkylene-C3-10 cycloalkyl optionally substituted with one or more R'" , -$C_{1-4}$ alkylene- 4- to 10-membered heterocycloalkyl optionally substituted with one or more R‴, -$C_{1-4}$ alkylene-$C_{6-10}$ aryl optionally substituted with one or more R‴, -$C_{1-4}$ alkylene- 5- to 10 -membered heteroaryl optionally substituted with one or more R'" ;

Each R" is selected from the group consisting of H, D, $C_{1-4}$alkyl, $C_{1-4}$haloalkyl, and C $_{3-4}$ cycloalkyl;

Each R" is independently selected from the group consisting of D, halogen, hydroxy, nitro, CN, C1-6 alkyl , and C1-6 haloalkyl .

In another preferred embodiment, the compound is as shown in Formula I-1 or Formula I-2

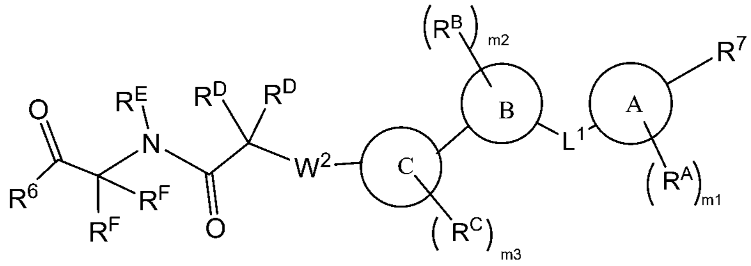

(I-1)

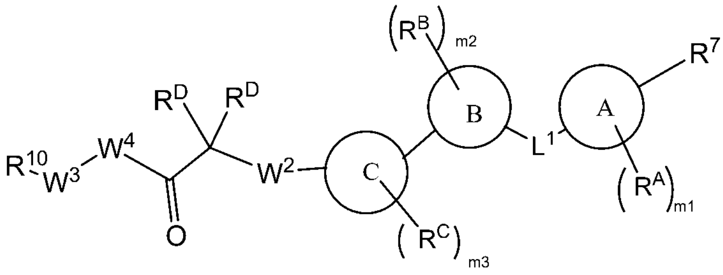

(I-2)

In another preferred embodiment, $R^7$ is an optionally substituted group selected from the group consisting of $C_{1-6}$alkyl, C3-10 cycloalkyl, 4 to 10-membered heterocycloalkyl, C3-10 cycloalkenyl, 4 to 10-membered heterocycloalkenyl, $C_{6-10}$ aryl, and 5 to 10-membered heteroaryl.

In another preferred embodiment, $R^7$ is an optionally substituted group selected from the group consisting of a $C_{6-10}$ aryl group, and a 5- to 10-membered heteroaryl group.

In another preferred embodiment, in $R^7$, the heteroaryl group includes 1, 2 or 3 nitrogen heteroatoms as ring atoms, and the remaining ring atoms in the heteroaryl group are carbon atoms.

In another preferred embodiment, $R^7$ is an optionally substituted group selected from the following group:

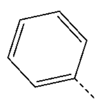, 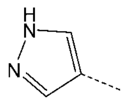, 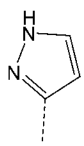, 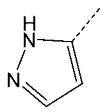, 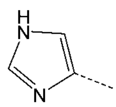, 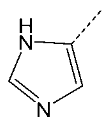, , 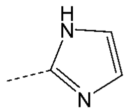, 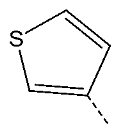, 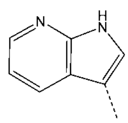,

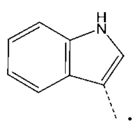.

In another preferred embodiment, $R^7$ is an optionally substituted phenyl group or a 5-membered heteroaryl group.

In another preferred embodiment, $R^7$ is an optionally substituted group selected from the following group:

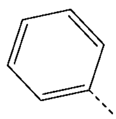, 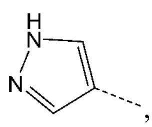, 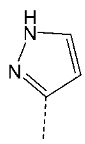, 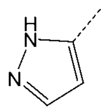, 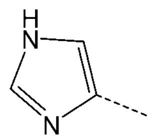, 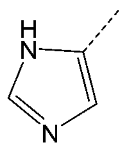 and 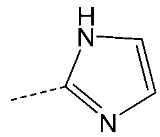.

In another preferred embodiment, in $R^7$, the optional substitution refers to unsubstituted or 1 or 2 hydrogen atoms in the group are replaced by an optional **R** substituent.

In another preferred embodiment, $R^7$ is selected from the following group:

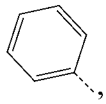, 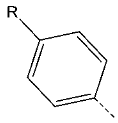, 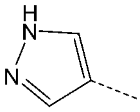, 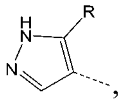, 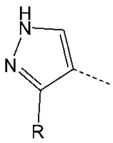, 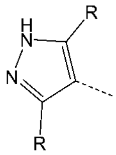, 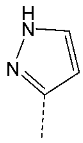, 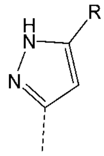, 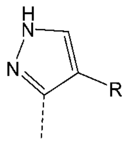,

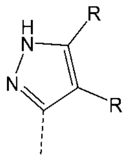,

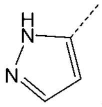, 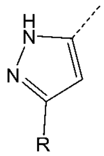, 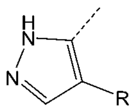, 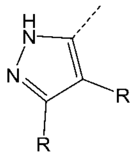, 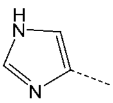, 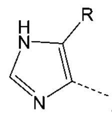, 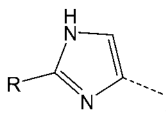, 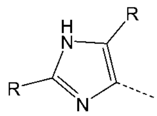,

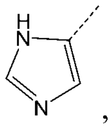, 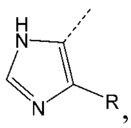, 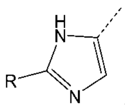, 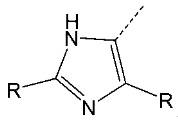, 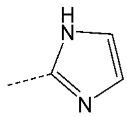, 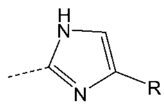, 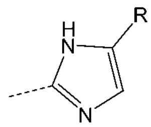

and

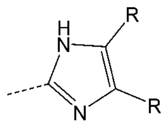.

In another preferred embodiment, in $R^7$, each **R** is independently selected from the following group: $C_{1-6}$alkyl, -NR'R"; wherein each R' is independently selected from the following group: H, $C_{1-6}$alkyl; each R" is selected from the following group: H, $C_{1-4}$alkyl.

In another preferred embodiment, ring A is a ring selected from the following group:

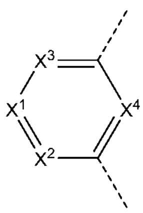, 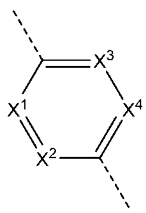;

wherein $X^1$, $X^2$, $X^3$ and $X^4$ are each independently selected from the group consisting of CH and N.

In another preferred embodiment, Ring A is:

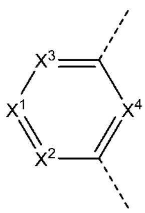;

wherein $X^1$, $X^2$, $X^3$ and $X^4$ are each independently selected from the group consisting of CH and N.

In another preferred embodiment, at most one or two of $X^1$, $X^2$, $X^3$ and $X^4$ are N.

In another preferred embodiment, $X^1$ , $X^2$, $X^3$ and $X^4$ are all CH.

In another preferred embodiment, m1=0, 1 or 2; preferably, m1=0 or 1; more preferably, m=0.

In another preferred embodiment, $R^A$ is independently $R^{s1}$ or $R^{A1}$; and $R^{A1}$ is selected from the group consisting of halogen, optionally substituted C1-6 alkyl , optionally substituted C1-6 haloalkyl , and optionally substituted C1-6 $_{alkoxy}$ (preferably, $R^{A1}$ is halogen).

In another preferred embodiment, $R^A$ is independently H, $C_{1-4}$ alkyl or $R^{A1}$; and $R^{A1}$ is selected from the group consisting of halogen, optionally substituted C1-6 alkyl , optionally substituted C1-6 haloalkyl , and optionally substituted $_{C1-6}$ alkoxy (preferably, $R^{A1}$ is halogen).

In another preferred embodiment, n1=0, 1 or 2. In another preferred embodiment, each $W^1$ is independently $-C(R^{s2})_2-$ (preferably, $-CH_2-$).

In another preferred embodiment, n1=0.

In another preferred embodiment, $L^1$ is -X-: Preferably, $L^1$ is -C(O)- or $-SO_2-$. In another preferred embodiment, $L^1$ is $-SO_2-$.

In another preferred embodiment, ring B is an optionally substituted ring selected from the group consisting of $C_{3-12}$ cycloalkyl and 4- to 12-membered heterocycloalkyl.

In another preferred embodiment, ring B is

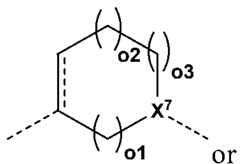 or 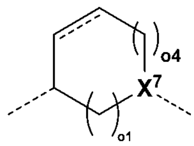 ;

wherein,

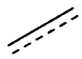

is a single bond or a double bond, $X^7$ is N or CH, o1 is 1 or 2, o2 is 0, 1, 2 or 3, o3 is 0 or 1, and o4 is 0, 1, 2 or 3.

In another preferred embodiment, $X^7$ is N.

In another preferred embodiment, o1 is 1.

In another preferred embodiment, when $X^7$ is N, N in ring B is connected to $L^1$ .

In another preferred embodiment, ring B is

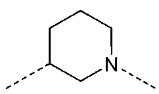

preferably, N in ring B is connected to $L^1$.

In another preferred embodiment, Ring B is

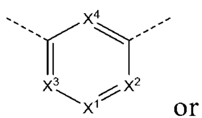 or 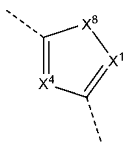 ,

wherein $X^1$ , $X^2$, $X^3$ and $X^4$ are each independently selected from the following group: CH, N, and $X^8$ is S, O or NH.

In another preferred embodiment, RB are both $R^{s1}$ or $R^{s2}$ .

In another preferred embodiment, m2=0.

In another preferred embodiment,

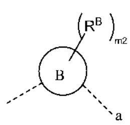

it is

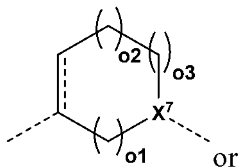

or

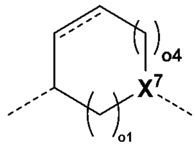

.

In another preferred embodiment,

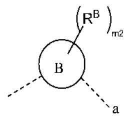

it is

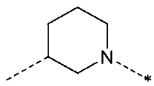

;

wherein, * indicates connection with $L^{1.}$

**In** another preferred embodiment, ring C is

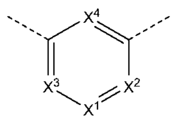

,

wherein $X^1$, $X^2$, $X^3$ and $X^4$ are each independently selected from the following group: CH, N.

In another preferred embodiment, in ring C, at most one or two of $X^1$ , $X^2$, $X^3$ and $X^4$ are $^{N.}$

In another preferred embodiment, ring C is

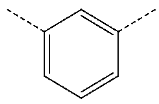

.

In another preferred embodiment, m3=0. In another preferred embodiment, m3=1, 2, 3 or 4.

In another preferred embodiment, $R^{C1}$ is selected from the group consisting of halogen (preferably F, Cl), $C_{1-6}$haloalkyl (preferably trifluoromethyl), and $C_{1-6}$alkoxy (preferably methoxy).

In another preferred embodiment, ring C is

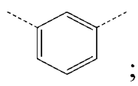

;

m3 = 0, 1 or 2; RC is H or $R^{C1\,;}$ and $R^{C1}$ is selected from the following group: halogen (preferably F, Cl), C1-6 haloalkyl (preferably trifluoromethyl), and C1-6 alkoxy (preferably methoxy); preferably, $R^{C1}$ is halogen.

In another preferred embodiment, $W_2$ is -O- or -N($R^{s1}$)-; preferably, $W_2$ is -O- or -NH-.

In another preferred embodiment, W2 is -O-.

In another preferred embodiment, $R^D$ are each independently selected from the following group: H, optionally substituted $C_{1-4}$alkyl; or, two $R^D$s and the carbon atom to which they are attached together form an optionally substituted C3-10 cycloalkyl.

In another preferred embodiment, $R^D$ are each independently selected from the following group: H, optionally substituted $C_{1-4}$ alkyl .

In another preferred embodiment, $R^D$ are independently selected from the following group: H, methyl, ethyl.

In another preferred embodiment, Rand $^D$ are both methyl.

In another preferred embodiment,

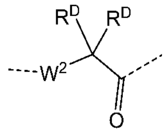

it is $-OC(R^D)_{2-}$ C(O)-. In another preferred embodiment,

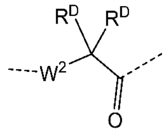

it is $-OC(CH3)_{2-}$ C(O)-.

In another preferred embodiment, RE is selected from the group consisting of H, methyl, and ethyl.

In another preferred embodiment, RE is H.

In another preferred embodiment, RF are independently selected from the following group: H, methyl, ethyl.

In another preferred embodiment, Rand $^F$ are both H.

In another preferred embodiment,

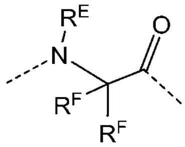

it is $-NH-CH_2-C(O)-$.

In another preferred embodiment, $R^6$ is an optionally substituted group selected from the group consisting of: $-OR^2$ or $-NR^4R^5$. In another preferred embodiment, $R^6$ is $-NR^4 R^5$.

In another preferred embodiment, $R^2$ is selected from the following group: H, optionally substituted $C_{1-6}$alkyl.

In another preferred embodiment, $-NR^4R^5$ is a 4- to 10-membered heterocycloalkyl group substituted by one or more (eg, 1, 2 or 3) $R^{3.}$

In another preferred embodiment, $-NR^4R^5$ is a 5- to 6-membered heterocycloalkyl group substituted by one or more (eg, 1, 2 or 3) $R^{3.}$

In another preferred embodiment, $-NR^4R^5$ is a 5- to 6-membered heterocycloalkyl group substituted by one $R^{3.}$

In another preferred embodiment, $-NR^4R^5$ is

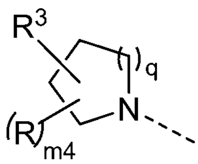,

wherein q is 0, 1, 2 or 3.

In another preferred embodiment, the 5- to 6-membered heterocycloalkyl group contains only one N heteroatom as a ring atom.

In another preferred embodiment, $-NR^4 R^5$ is substituted by

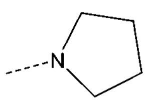

one or more $R^3$ or

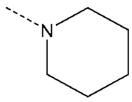.

In another preferred embodiment, $-NR^4\ R^5$ is selected from the following group:

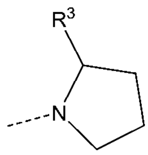, 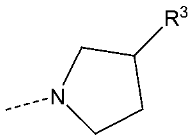, 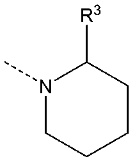, 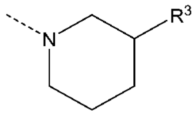, 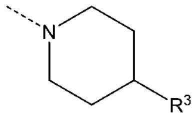.

In another preferred embodiment, $-NR^4R^5$ is

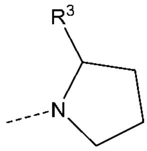.

In another preferred embodiment, $-NR^4\ R^5$ is selected from the following group:

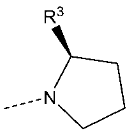, 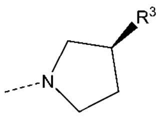, 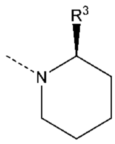, 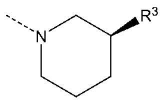, , 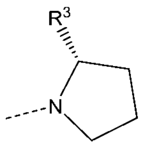, 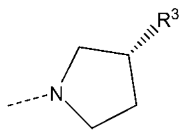, 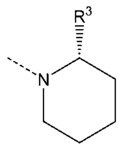,

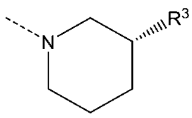.

In another preferred embodiment, $-NR^4R^5$ is

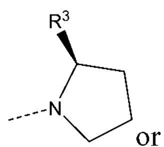 or 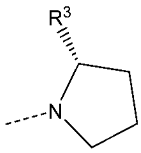.

In another preferred embodiment, $R^{31}$ is selected from the group consisting of H, $C_{1-4}$alkyl. In another preferred embodiment, $R^{31}$ is selected from the group consisting of H, $C_{1-4}$alkyl .

In another preferred embodiment, $R^{32}$ is selected from the group consisting of H, $C_{1-4}$alkyl .

In another preferred embodiment, $R^{33}$ is selected from the following group: H, optionally substituted $C_{1-4}$alkyl, $-C_{1-4}$alkylene-$R^{34}$;

In another preferred embodiment, $R^3$ is selected from the group consisting of -OH, $-CH_2OH$, $-NH_2$, $-N(CH_3)_2$, $-CH_2-NH_2$, $-CH_2-N(CH\ _3)_2$, $-CH_2-CH_2-NH_2$, and $-CH_2-CH_2-N(CH_3)_2$.

In another preferred embodiment, $R^3$ is $-CH_2-NH-CH_2-R^{34}$.

In another preferred embodiment, $R^{34}$ is selected from the following group: $C_{6-10}$ aryl, 5- to 10-membered heteroaryl.

In another preferred embodiment, $R^{34}$ is selected from the following group: phenyl, 5- or 6-membered heteroaryl.

In another preferred embodiment, in $R^{34}$, the $C_{6-10}$ aryl group is substituted by at least one $-NH_2$ group.

In another preferred embodiment, in $R^{34}$, the 5- to 10-membered heteroaryl group (preferably, a 5- or 6-membered heteroaryl group) is a heteroaryl group containing 1 or 2 nitrogen heteroatoms as ring atoms; preferably, the 5- to 10-membered heteroaryl group (preferably, a 5- or 6-membered heteroaryl group) includes 1 or 2 nitrogen heteroatoms as ring atoms, and the remaining ring atoms in the heteroaryl group are carbon atoms.

In another preferred embodiment, $R^{34}$ is selected from the following group:

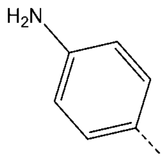, 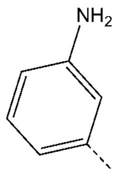, 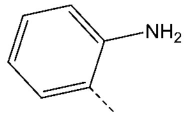, 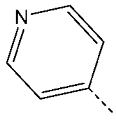, 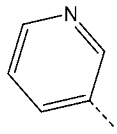, 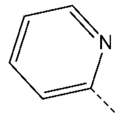,

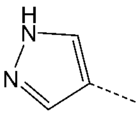, , 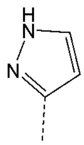, 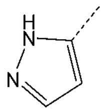, 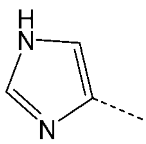, 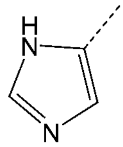, 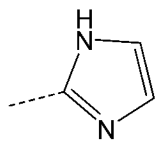.

In another preferred embodiment, $R^6$ is $-NR^4 R^5$ .

In another preferred embodiment, $R^3$ is $-C_{1-4}$alkylene-$N(R^{31})R^{32}$ ; preferably, -methylene-$N(R^{31})R^{32}$ .

In another preferred embodiment, $W^4$ is $-N(R^{s1})-$; $W^5$ is selected from the following group: -C(O)-, -S(O)-, and $-S(O)_2-$.

In another preferred embodiment, $R^{10}$ is unsubstituted or substituted by one or more RH and is selected from the group consisting of $C_{1-6}$alkyl, C3-10 cycloalkyl, 4 to 10-membered heterocycloalkyl, C3-10 cycloalkenyl, 4 to 10-membered heterocycloalkenyl, $C_{6-10}$ aryl, and 5 to 10-membered heteroaryl.

In another preferred embodiment, $R^{10}$ is unsubstituted or substituted by one or more RH and is selected from the group consisting of $C_{6-10}$ aryl, and 5- to 10-membered heteroaryl.

In another preferred embodiment, $R^{10}$ is phenyl substituted by one or more RH

In another preferred embodiment, $R^{10}$ is a phenyl group substituted by one 4-RH ( RH at position 4).

In another preferred embodiment, the compound is selected from Table AI

Table AI

| Molecu lar number | Structural formula | Molecu lar number | Structural formula |
|---|---|---|---|
| 115-01 0 | 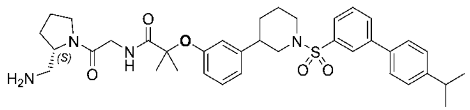 | 115-01 1 | 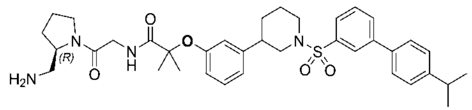 |
| 115-01 2 | 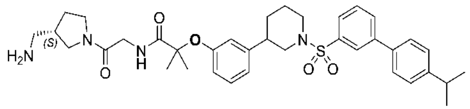 | 115-01 3 | 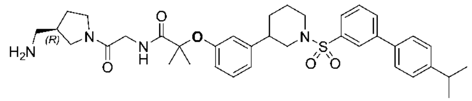 |
| 115-01 4 | 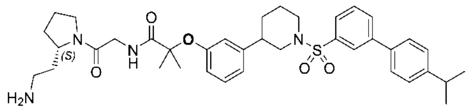 | 115-01 5 | 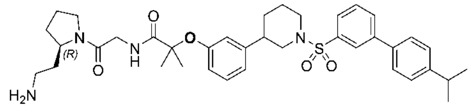 |
| 115-01 6 | 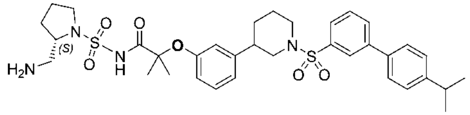 | 115-01 7 | 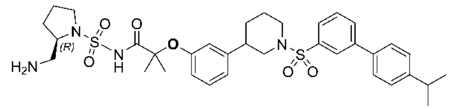 |
| 115-01 8 | 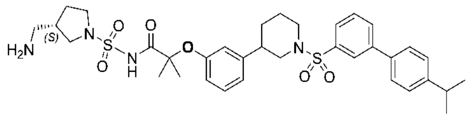 | 115-01 9 | 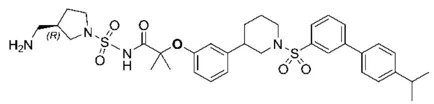 |
| 115-02 0 | 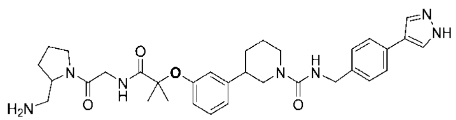 | 115-02 1 | 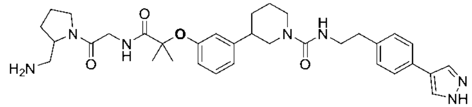 |
| 115-02 2 | 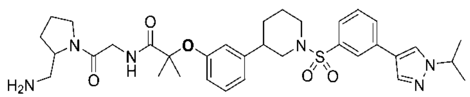 | 115-02 3 | 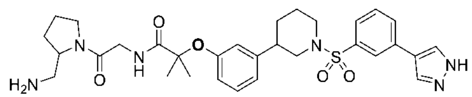 |
| 115-02 4 | 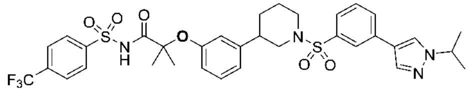 | 115-02 5 | 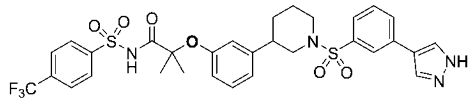 |

(continued)

| Molecu lar number | Structural formula | Molecu lar number | Structural formula |
|---|---|---|---|
| 115-02 6 | 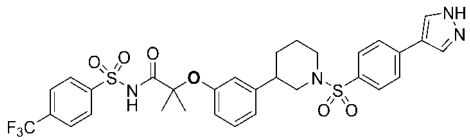 | 115-02 7 | 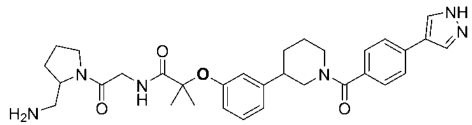 |
| 115-02 8 | 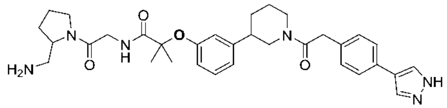 | 115-02 9 | 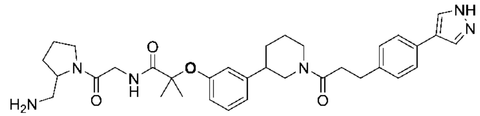 |
| 115-03 0 | 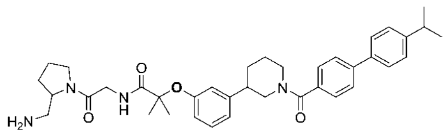 | 115-03 1 | 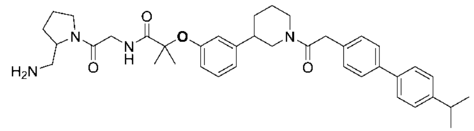 |
| 115-03 2 | 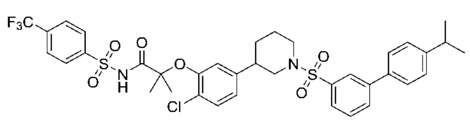 | 115-03 3 | 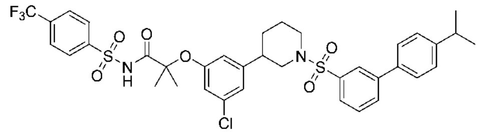 |
| 115-03 4 | 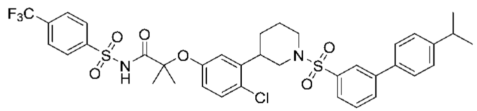 | 115-03 5 | 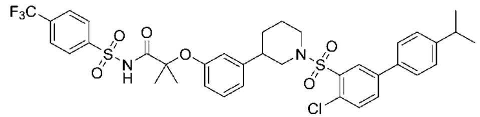 |
| 115-03 6 | 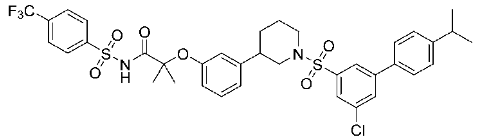 | 115-03 7 | 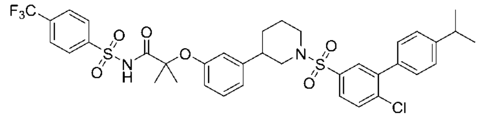 |
| 115-03 8 | 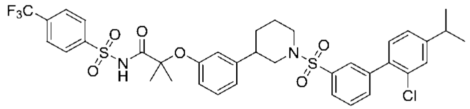 | 115-03 9 | 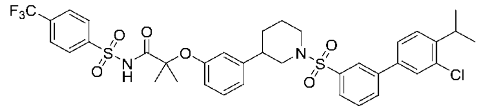 |

In another preferred embodiment, the compound is selected from Table BI

Table BI

| Molecu lar number | Structural formula | Molecu lar number | Structural formula |
|---|---|---|---|
| 115-04 0 | 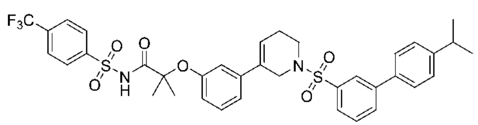 | 115-04 1 | 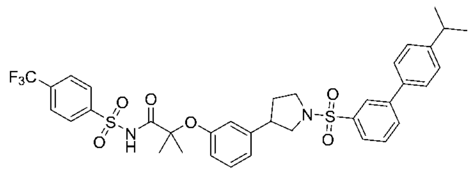 |
| 115-04 2 | 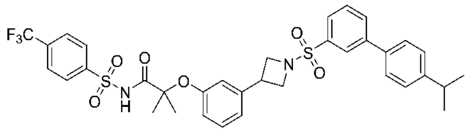 | 115-04 3 | 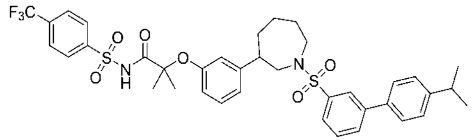 |
| 115-04 4 | 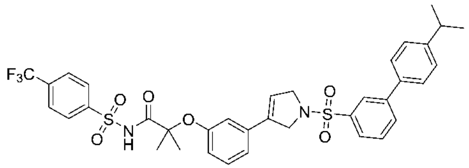 | 115-04 5 | 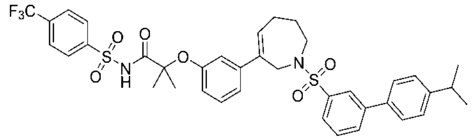 |
| 115-04 6 | 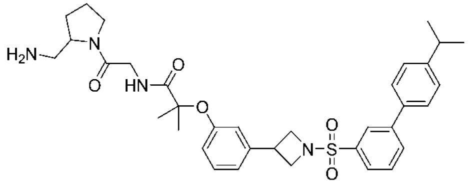 | 115-04 7 | 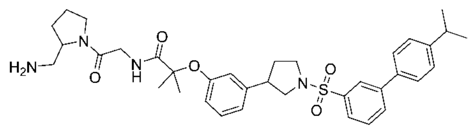 |
| 115-04 8 | 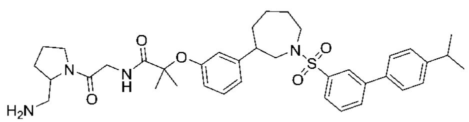 | 115-04 9 | 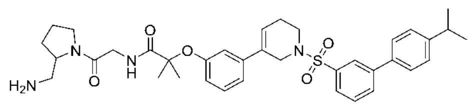 |
| 115-05 0 | 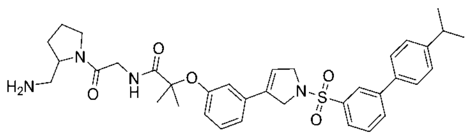 | 115-05 1 | 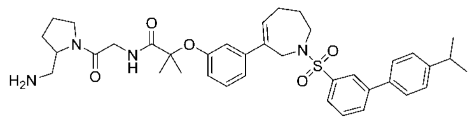 |

In another preferred embodiment, the compound is selected from Table CI

Table CI

| Molecular number | Structural formula | Molecular number | Structural formula |
|---|---|---|---|
| 115-05 2 | 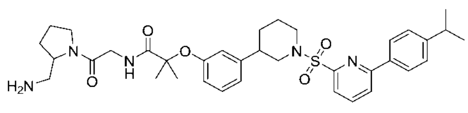 | 115-05 3 | 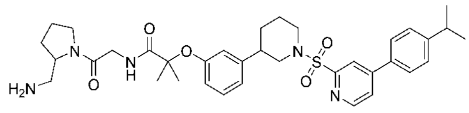 |
| 115-05 4 | 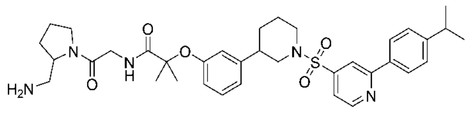 | 115-05 5 | 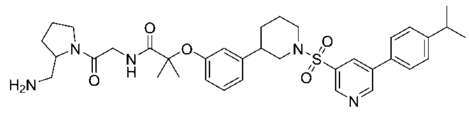 |
| 115-05 6 | 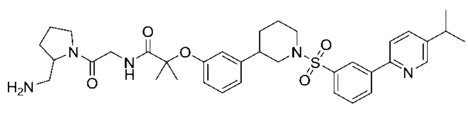 | 115-05 7 | 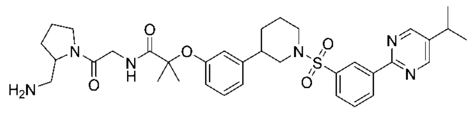 |
| 115-05 8 | 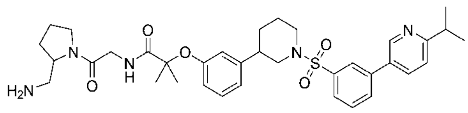 | 115-05 9 | 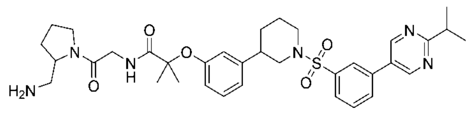 |
| 115-06 0 | 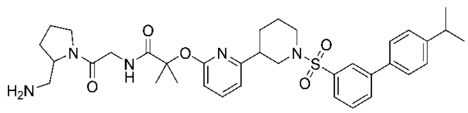 | 115-06 1 | 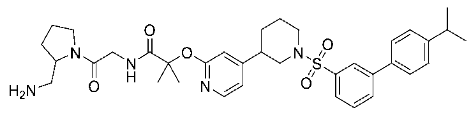 |
| 115-06 2 | 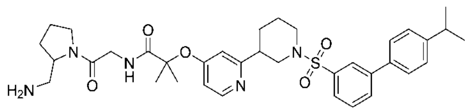 | 115-06 3 | 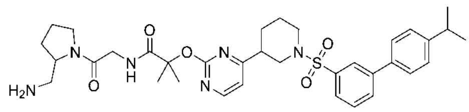 |
| 115-06 4 | 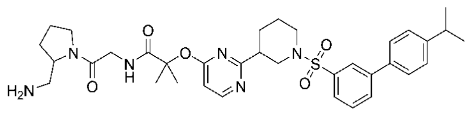 | 115-06 5 | 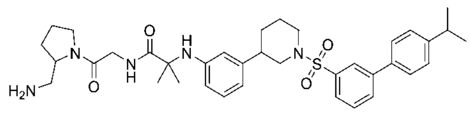 |
| 115-06 6 | 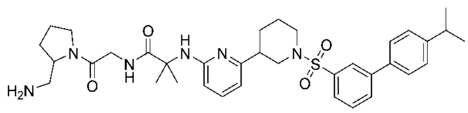 | 115-06 7 | 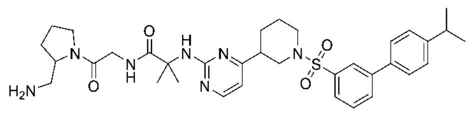 |
| 115-06 8 | 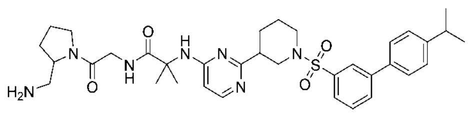 | 115-06 9 | 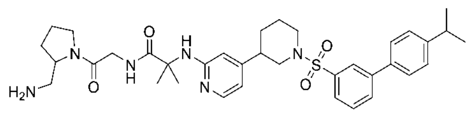 |

In another preferred embodiment, Ring A, Ring B, Ring C, $L^1$, $W^2$, $R^A$, RB, RC, $R^D$, $R^7$, $R^9$, subscript m1, subscript m2, and subscript m3 are each independently the corresponding groups in the Example compounds or the specific compounds in Tables AI, BI, and CI.

In another preferred embodiment, Ring A, Ring B, Ring C, L1, W2, $R^A$, RB, RC, $R^D$, RE, RF, R7, R6, subscript m1, subscript m2, and subscript m3 are each independently the corresponding groups in the Example compounds or the specific compounds in Tables AI, BI, and CI.

In another preferred embodiment, Ring A, Ring B, Ring C, L1, W2, $R^A$, RB, RC, $R^D$, R7, R10, W5, W4, subscript m1, subscript m2, and subscript m3 are each independently the corresponding groups in the Example compounds or the specific compounds in Tables AI, BI, and CI.

In another preferred embodiment, Ring A, Ring B, Ring C, $L^1$, $W^2$, $R^A$, RB, RC, $R^D$, $R^7$, RE, RF, $R^6$, subscript m1, subscript m2 and subscript m3 may also be defined as in the second aspect.

In the second aspect of the present invention, a compound or a pharmaceutically acceptable salt thereof, or an isomer, solvate, crystal form or prodrug thereof is provided, wherein the compound is as shown in Formula II

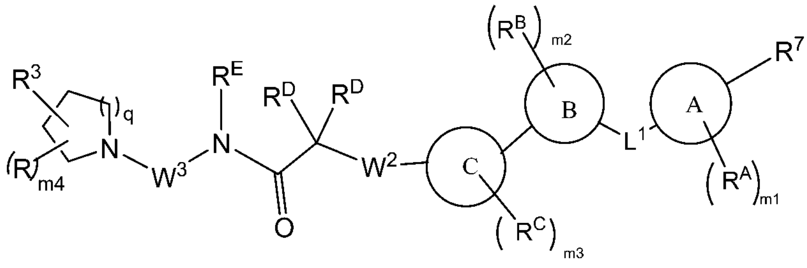

(II)

wherein

q is 0, 1, 2, or 3;

$R^3$ is selected from the group consisting of H, $-OR^{31}$, $-C_{1-4}alkylene-OR^{31}$, $-N(R^{32})R^{33}$, $-C_{1-4}alkylene-N(R^{31})R^{32}$;

m4 is 0, 1, 2, 3, 4, 5, 6 or 7;

$R^{31}$ is selected from the group consisting of H, optionally substituted $C_{1-4}$alkyl, $R^{34}$, $-C_{1-4}alkylene-R^{34}$;

$R^{32}$ is selected from the group consisting of H, optionally substituted $C_{1-4}$alkyl;

$R^{33}$ is selected from the group consisting of H, optionally substituted $C_{1-4}$alkyl, $R^{34}$, $-C_{1-4}alkylene-R^{34}$;

$R^{34}$ is selected from the group consisting of C3-10 cycloalkyl, 4- to 8-membered heterocycloalkyl, $C_{6-10}$ aryl, 5- to 10-membered heteroaryl, C3-10 cycloalkenyl, and 4- to 10-membered heterocycloalkenyl; wherein the cycloalkyl, heterocycloalkyl, aryl, heteroaryl, cycloalkenyl, and heterocycloalkenyl groups are optionally substituted with one or more groups selected from the group consisting of $-NH_2$, R;

W3 is selected from the group consisting of -C(O)-, -S(O)-, $-S(O)_2-$, $-C(RF)_{2-}$ C(O)-, $-C(RF)_{2-}$ $^{s}$ (O)-, $-C(RF)_{2-}$ $S(O)_{2-}$ (preferably, in W3 , $-C(^{RF})_{2-}$ is $_{linked\ to}$ N(RE));

RF are each independently selected from the group consisting of H, optionally substituted $C_{1-4}$ alkyl , optionally substituted C3-10 cycloalkyl, optionally substituted 4 to 10 membered heterocycloalkyl; or two RFs and the carbon atom to which they are attached together form an optionally substituted C3-6 cycloalkyl or an optionally substituted 4 to 6 membered heterocyclyl;

$R^E$ is selected from the group consisting of H, optionally substituted $C_{1-4}$alkyl, optionally substituted C3-10 cycloalkyl, and optionally substituted 4- to 10-membered heterocycloalkyl;

$R^D$ are each independently selected from the group consisting of H, optionally substituted $C_{1-4}$alkyl; or, two $R^D$s and the carbon atom to which they are attached together form a group selected from the group consisting of optionally substituted C3-10 cycloalkyl, optionally substituted 4 to 10 membered heterocycloalkyl;

W2 is selected from the group consisting $^{of}$ -O-, -S-, and $-N(R^{s1})-$;

Ring C is optionally substituted with a ring selected from the group consisting of $C_{6-10}$ aryl, and 5- to 10-membered heteroaryl;

m3=0, 1, 2, 3, or 4;

Each RC is independently $R^{C1}$ or $R^{s1}$ ;

Each $R^{C1}$ is independently selected from the group consisting of halogen, optionally substituted $C_{1-6}$alkyl, optionally substituted $C_{1-6}$haloalkyl, hydroxy, and optionally substituted $C_{1-6}$alkoxy, optionally substituted $C_{1-6}$haloalkoxy;

Ring B is an optionally substituted ring selected from the group consisting of $C_{3-12}$ cycloalkyl, 4-to 12-membered heterocycloalkyl, C3-10 cycloalkenyl, and 4- to 10-membered heterocycloalkyl;

Each RB is independently $R^{B1}$, $R^{s1}$ or $R^{s2}$ ;

each $R^{B1}$ is independently selected from the group consisting of halogen, hydroxy, cyano, optionally substituted $C_{1-6}$alkyl, optionally substituted $C_{1-6}$alkoxy, optionally substituted $C_{1-6}$alkylthio, optionally substituted C3-10 cycloalkyl, optionally substituted 4- to 10-membered heterocycloalkyl, optionally substituted C3-10 cycloalkenyl, optionally substituted 4- to 10-membered heterocycloalkenyl, optionally substituted $C_{6-10}$ aryl, and optionally substituted 5- to 10-membered heteroaryl;

m2=0, 1, 2, 3, or 4;

$L^1$ is $-X-(W^1)_{n1}-$;

X is selected from the group consisting of -C(O)-, -S(O)-, and $-S(O)_2-$;

Each $W^1$ is independently selected from the group consisting of none, -O-, -S-, -C(O)-, -S(O)-, $-S(O)_2-$, $-N(R^{s1})-$, $-C(R^{s2})_2-$;

Subscript n1 = 0, 1, 2, or 3;

Ring A is an optionally substituted ring selected from the group consisting of $C_{6-10}$ aryl; a 5- to 10-membered

heteroaryl; a $C_{6-10}$ aryl substituted by a C3-10 cycloalkyl, a 4- to 10-membered heterocycloalkyl, a C3-10 cycloalkenyl, a 4- to 10-membered heterocycloalkenyl, a $C_{6-10}$ aryl or a 5-to 10-membered heteroaryl ; a 5- to 10-membered heteroaryl substituted by a C3-10 cycloalkyl, a 4- to 10-membered heterocycloalkyl, a C3-10 cycloalkenyl, a 4- to 10-membered heterocycloalkenyl, a $C_{6-10}$ aryl or a 5- to 10-membered heteroaryl; a $C_{6-10}$ aryl fused to a C3-10 cycloalkyl, a 4- to 10-membered heterocycloalkyl , a C3-10 cycloalkenyl, a 4- to 10-membered heterocycloalkenyl, a $C_{6-10}$ aryl or a 5- to 10-membered heteroaryl ; 3-10 -membered cycloalkyl, 4- to 10-membered heterocycloalkyl, C3-10 cycloalkenyl, 4- to 10-membered heterocycloalkenyl, $C_{6-10}$ aryl, or 5- to 10-membered heteroaryl-fused 5- to 10-membered heteroaryl;

m1=0, 1, 2, 3, or 4;

Each $R^A$ is independently $R^{A1}$ , $R^{s1}$ or $R^{s2}$;

each $R^{A1}$ is independently selected from the group consisting of halogen, optionally substituted $C_{1-6}$alkyl, optionally substituted $C_{1-6}$haloalkyl, optionally substituted $C_{1-6}$alkoxy, optionally substituted $C_{1-6}$alkylthio, optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl; or, when two $R^{A1}$ are located on adjacent ring atoms, the two $R^{A1}$ and the ring atoms adjacent thereto together form a ring selected from the group consisting of optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl;

$R^7$ is an optionally substituted group selected from the group consisting of none, $C_{1-6}$alkyl, C3-10 cycloalkyl, 4 to 10-membered heterocycloalkyl, C3-10 cycloalkenyl, 4 to 10-membered heterocycloalkenyl, $C_{6-10}$ aryl, and 5 to 10-membered heteroaryl;

$R^{s1}$ are each independently selected from the group consisting of H, optionally substituted $C_{1-4}$alkyl, optionally substituted $C_{3-6}$ cycloalkyl, and optionally substituted 4- to 6-membered heterocyclyl;

$R^{s2}$ are each independently selected from the following group: H, optionally substituted $C_{1-4}$alkyl, optionally substituted $C_{3-6}$ cycloalkyl, optionally substituted 4 to 6-membered heterocyclyl; or two $R^{s2}$ and the carbon atom to which they are attached together form an optionally substituted $C_{3-6}$ cycloalkyl or an optionally substituted 4 to 6-membered heterocyclyl;

Unless otherwise defined, the optional substitution refers to unsubstituted or one or more (such as 1, 2, 3 or 4) hydrogen atoms in the group are replaced by an optional Rsubstituent;

R is each independently selected from the group consistingof D, Halogen, C1-6-alkyl, C1-6-haloalkyl, C1-6-hydroxyalkyl, C2-6 vinyl, C2-6 alkynyl-CN, -OR', $-NO_2$, -NR'R", -SR', -OC(O)R', -C(O)R', $-CO_2R'$, -CONR', -OC(O)NR'R", -NR"C(O)R', -NR"-C(O)NR'R", $-NR"C(O)_2R'$ , -S(O)R', $-S(O)_2R'$, $-S(O)_2NR'R"$ , $NR"S(O)_2R'$, C3-10 cycloalkyl optionally substituted with one or more R'", 4 to 10 membered heterocycloalkyl optionally substituted with one or -more R‴ , C3-10 cycloalkyl optionally substituted with one or more R', C3-10 cycloalkyl optionally substituted with one or more R', C3-10 cycloalkyl optionally substituted with one or more R', C3-10 cycloalkyl optionally substituted with one or more R', -C3-10 cycloalkyl optionally substituted with one or more R' , C3-10 cycloalkyl optionally substituted with one or more R', C3-10 cycloalkyl optionally substituted with one or more R' $-C_{1-4}$alkylene-C3-10 cycloalkyl optionally substituted by one or more R'", $-C_{1-4}$alkylene- 4 to 10 -membered heterocycloalkyl optionally substituted by one or more R‴, $-C_{1-4}$alkylene-$C_{6-10}$ aryl optionally substituted by one or more R' ", $-C_{1-4}$alkylene - 5 to 10 -membered heteroaryl optionally substituted by one or more R‴ ;

each R' is independently selected from the group consisting of H, D, C1-6 alkyl , C1-6 haloalkyl, C3-10 cycloalkyl optionally substituted with one or more R'", 4- to 10-membered heterocycloalkyl optionally substituted with one or more R‴, $C_{6-10}$ aryl optionally substituted with one or more R‴, 5- to 10-membered heteroaryl optionally substituted with one or more R‴, $-C_{1-4}$ alkylene-C3-10 cycloalkyl optionally substituted with one or more R'" , $-C_{1-4}$ alkylene- 4- to 10-membered heterocycloalkyl optionally substituted with one or more R‴, $-C_{1-4}$ alkylene-$C_{6-10}$ aryl optionally substituted with one or more R‴, $-C_{1-4}$ alkylene- 5- to 10 -membered heteroaryl optionally substituted with one or more R‴ ;

Each R" is selected from the group consisting of H, D, $C_{1-4}$alkyl, $C_{1-4}$haloalkyl, and $C_{3-4}$ cycloalkyl;

Each R" is independently selected from the group consisting of D, halogen, hydroxy, nitro, CN, C1-6 alkyl , and C1-6 haloalkyl .

In another preferred embodiment, $W^3$ is $-C(RF)_2-$ C(O)-, and -C(RF)2- is connected to N(RE).

In another preferred embodiment, ring B is an optionally substituted ring selected from the group consisting of $C_{3-12}$ cycloalkyl and 4- to 12-membered heterocycloalkyl.

In another preferred embodiment, ring B is

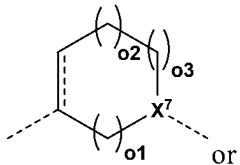 or 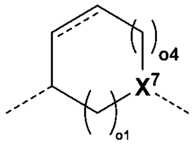 ;

wherein,

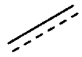

is a single bond or a double bond, $X^7$ is N or CH, o1 is 1 or 2, o2 is 0, 1, 2 or 3, o3 is 0 or 1, and o4 is 0, 1, 2 or 3.

In another preferred embodiment, $X^7$ is N.

In another preferred embodiment, o1 is 1.

In another preferred embodiment, when $X^7$ is N, N in ring B is connected to $L^1$.

In another preferred embodiment, ring B is

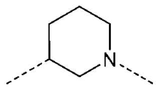 ;

preferably, N in ring B is connected to $L^1$.

In another preferred embodiment, RB are both $R^{s1}$ or $R^{s2}$.

In another preferred embodiment, m2=0.

In another preferred embodiment,

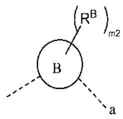

it is

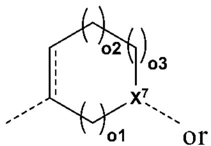 or 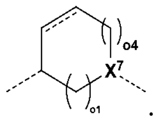 .

In another preferred embodiment,

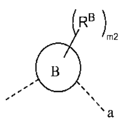

it is

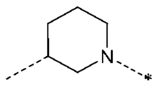 ;

wherein, * indicates connection with $L^1$.

In another preferred embodiment, n1=0.

In another preferred embodiment, n1=0, 1 or 2. In another preferred embodiment, each $W^1$ is independently $-C(R^{s2})_2-$ (preferably, $-CH_2-$).

In another preferred embodiment, $L^1$ is -X-; preferably, $L^1$ is -CO- or $-SO_2-$. In another preferred embodiment, $L^1$

is $-SO_2-$.

In another preferred embodiment, ring C is

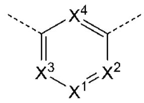

,

wherein $X^1$, $X^2$, $X^3$ and $X^4$ are each independently selected from the following group: CH, N.

In another preferred embodiment, in ring C, at most one or two of $X^1$, $X^2$, $X^3$ and $X^4$ are N.

In another preferred embodiment, ring C is

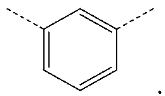

.

In another preferred embodiment, m3=0. In another preferred embodiment, m3=1, 2, 3 or 4.

In another preferred embodiment, $R^{C1}$ is selected from the group consisting of halogen (preferably F, Cl), $C_{1-6}$haloalkyl (preferably trifluoromethyl), and $C_{1-6}$alkoxy (preferably methoxy).

In another preferred embodiment, ring C is

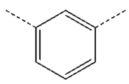

;

m3 = 0, 1 or 2; RC is H or $R^{C1}$; and $R^{C1}$ is selected from the following group: halogen (preferably F, Cl), C1-6 haloalkyl (preferably trifluoromethyl), and C1-6 alkoxy (preferably methoxy); preferably, $R^{C1}$ is halogen.

In another preferred embodiment, the compound is as shown in formula II-1

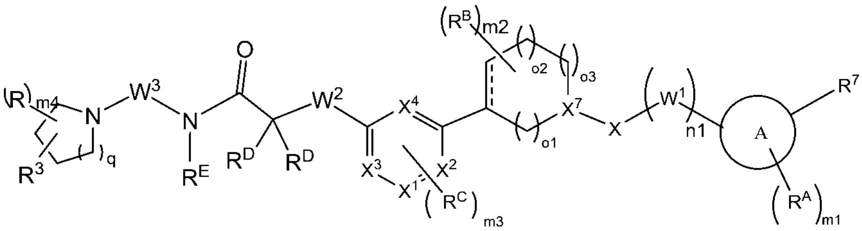

(II-1)

in,

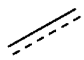

is a single bond or a double bond; $X^7$ is N or CH; o1 is 1 or 2, o2 is 0, 1, 2 or 3, and o3 is 0 or 1; $X^1$, $X^2$, $X^3$ and $X^4$ are each independently selected from the group consisting of CH, N.

In another preferred embodiment, the compound is as shown in formula II-2

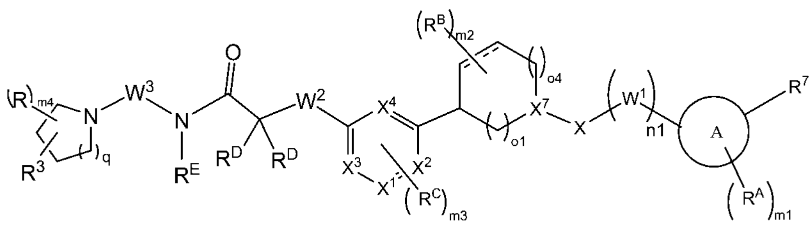

(II-2);

in,

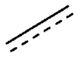

is a single bond or a double bond; $X^7$ is N or CH; o1 is 1 or 2, and o4 is 0, 1, 2 or 3; $X^1$, $X^2$, $X^3$ and $X^4$ are each independently selected from the group consisting of CH, N.

In another preferred embodiment, the compound is as shown in formula II-3 or II-4

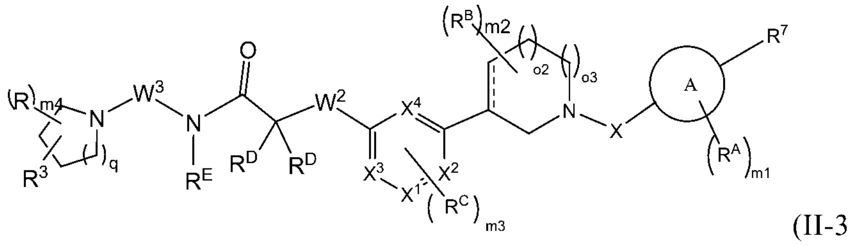

(II-3)

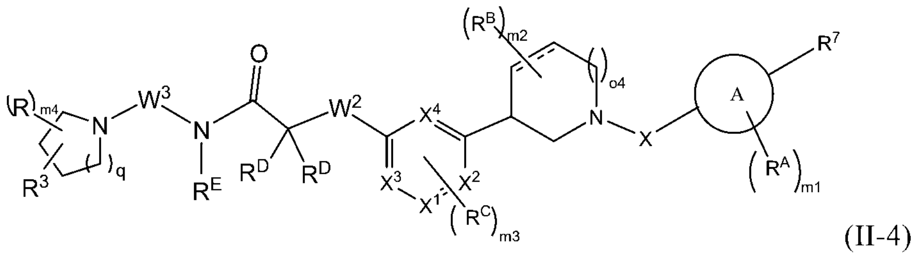

(II-4).

In another preferred embodiment, q is 1 or 2.

In another preferred embodiment,

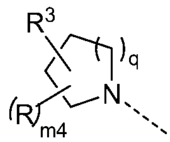

selected from the following group:

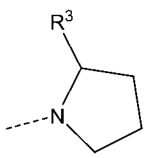, 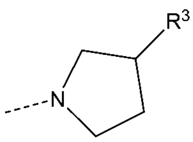, 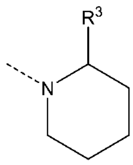, 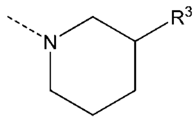, 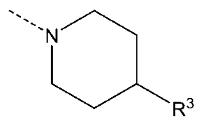.

In another preferred embodiment,

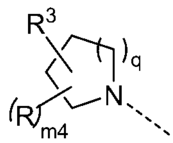

selected from the following group:

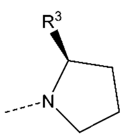, 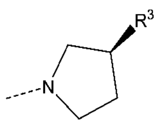, 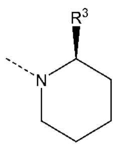, 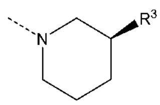, , 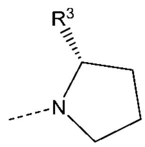, 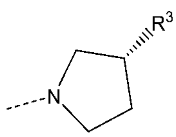, 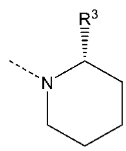,

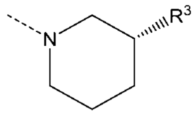.

In another preferred embodiment, the compound is as shown in formula A

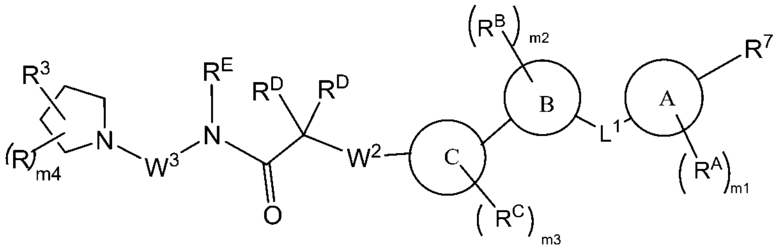

(A).

In another preferred embodiment,

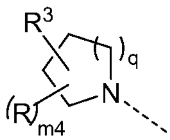 it is 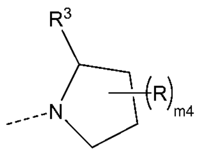 or 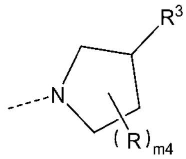.

In another preferred embodiment, m4=0.

In another preferred embodiment,

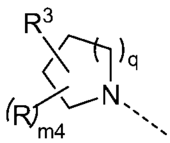

it is

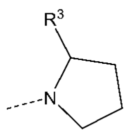.

In another preferred embodiment,

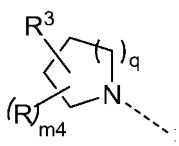

it is selected from the following group:

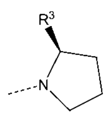,

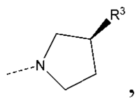, 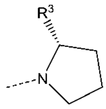, 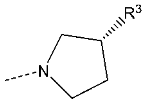.

In another preferred embodiment,

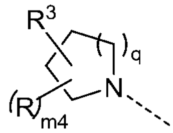

it is

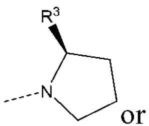 or 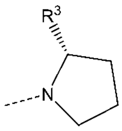.

In another preferred embodiment, $R^{31}$ is selected from the group consisting of H, $C_{1-4}$alkyl. In another preferred embodiment, $R^{31}$ is selected from the group consisting of H, $C_{1-4}$alkyl .

In another preferred embodiment, $R^{32}$ and $R^{33}$ are each independently selected from the following group: H, $C_{1-4}$alkyl.

In another preferred embodiment, $R^{32}$ and $R^{33}$ are both H.

In another preferred embodiment, $R^3$ is $-N(R^{32})R^{33}$ or $-C_{1-4}$alkylene-$N(R^{31})R^{32}$.

In another preferred embodiment, $R^3$ is $-C_{1-4}$alkylene-$N(R^{31})R^{32}$.

In another preferred embodiment, $R^3$ is selected from the group consisting of $-NH_2$, $-CH_2-NH_2$, $-CH_2-N(CH_3)_2$, $-CH_2-CH_2-NH_2$, and $-CH_2-CH_2-N(CH_3)_2$.

In another preferred embodiment, $R^7$ is an optionally substituted group selected from the group consisting of $C_{1-6}$alkyl, C3-10 cycloalkyl, 4 to 10-membered heterocycloalkyl, C3-10 cycloalkenyl, 4 to 10-membered heterocycloalkenyl, $C_{6-10}$ aryl, and 5 to 10-membered heteroaryl.

In another preferred embodiment, $R^7$ is an optionally substituted group selected from the group consisting of a $C_{6-10}$ aryl group, and a 5- to 10-membered heteroaryl group.

In another preferred embodiment, in $R^7$, the heteroaryl group includes 1, 2 or 3 nitrogen heteroatoms as ring atoms, and the remaining ring atoms in the heteroaryl group are carbon atoms.

In another preferred embodiment, $R^7$ is an optionally substituted group selected from the following group:

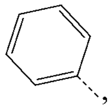, 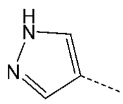, 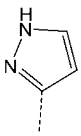, 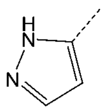, 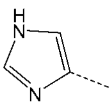, 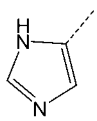, , 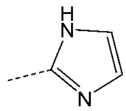, 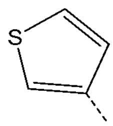, 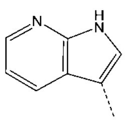,

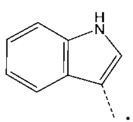.

In another preferred embodiment, $R^7$ is an optionally substituted phenyl group or a 5-membered heteroaryl group.

In another preferred embodiment, $R^7$ is an optionally substituted group selected from the following group:

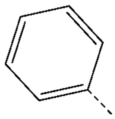, 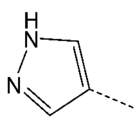, 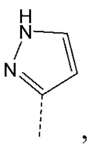, 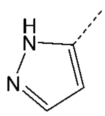, 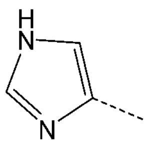, 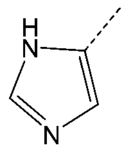 and 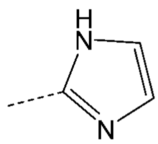.

In another preferred embodiment, in $R^7$, the optional substitution refers to unsubstituted or 1 or 2 hydrogen atoms in the group are replaced by an optional Rsubstituent.

In another preferred embodiment, $R^7$ is selected from the following group:

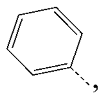, 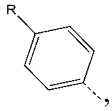, 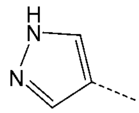, 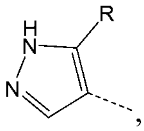, 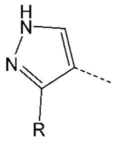, 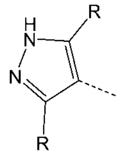, 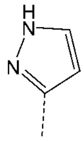, 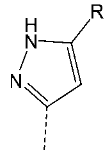, 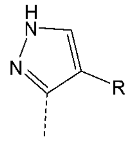,

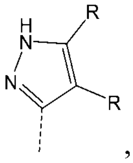,

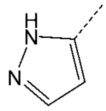, 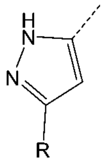, 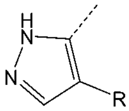, 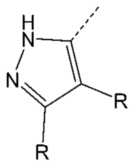, 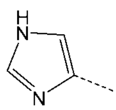, 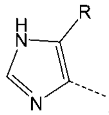, 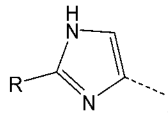, 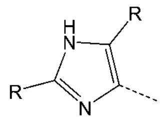,

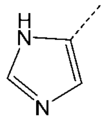, 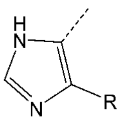, 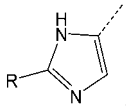, 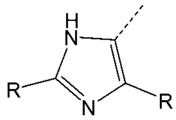, 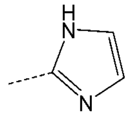, 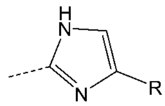, 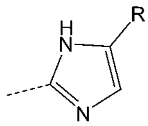

and

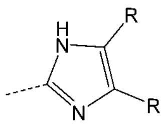.

In another preferred embodiment, in $R^7$, each R is independently selected from the following group: $C_{1-6}$alkyl, -NR'R"; wherein each R' is independently selected from the following group: H, $C_{1-6}$alkyl; each R" is selected from the following group: H, $C_{1-4}$alkyl.

In another preferred embodiment, ring A is a ring selected from the following group:

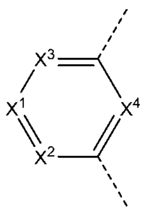, 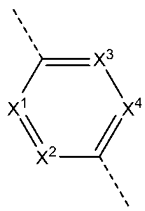;

wherein $X^1$, $X^2$, $X^3$ and $X^4$ are each independently selected from the group consisting of CH and N.

In another preferred embodiment, Ring A is:

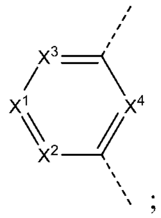

wherein $X^1$ , $X^2$, $X^3$ and $X^4$ are each independently selected from the group consisting of CH and N.

In another preferred embodiment, at most one or two of $X^1$ , $X^2$ , $X^3$ and $X^4$ are N.

In another preferred embodiment, $X^1$ , $X^2$, $X^3$ and $X^4$ are all CH.

In another preferred embodiment, m1=0, 1 or 2; preferably, m1=0 or 1; more preferably, m=0.

In another preferred embodiment, $R^A$ is independently $R^{s1}$ or $R^{A1}$; and $R^{A1}$ is selected from the group consisting of halogen, optionally substituted C1-6 alkyl , optionally substituted C1-6 haloalkyl , and optionally substituted C1-6 alkoxy (preferably, $R^{A1}$ is halogen).

In another preferred embodiment, $R^A$ is independently H, $C_{1-4}$ alkyl or $R^{A1}$; and $R^{A1}$ is selected from the group consisting of halogen, optionally substituted C1-6 alkyl , optionally substituted C1-6 haloalkyl , and optionally substituted C1-6 alkoxy (preferably, $R^{A1}$ is halogen).

In another preferred embodiment, W $^2$ is -O- or -N($R^s$)-; preferably, W $^2$ is -O- or -NH-.

In another preferred embodiment, W2 is -O-.

In another preferred embodiment, $R^D$ are each independently selected from the following group: H, optionally substituted $C_{1-4}$alkyl; or, two $R^D$s and the carbon atom to which they are attached together form an optionally substituted C3-10 cycloalkyl.

In another preferred embodiment, $R^D$ are each independently selected from the following group: H, optionally substituted $C_{1-4}$ alkyl .

In another preferred embodiment, $R^D$ are independently selected from the following group: H, methyl, ethyl.

In another preferred embodiment, Rand $^D$ are both methyl.

In another preferred embodiment,

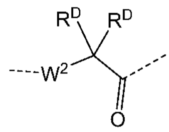

it is -OC($R^D$)$_{2-}$ C(O)-. In another preferred embodiment,

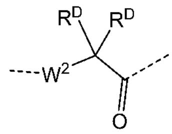

it is -OC(CH3)$_{2-}$ C(O)-.

In another preferred embodiment, RE is selected from the group consisting of H, methyl, and ethyl.

In another preferred embodiment, RE is H.

In another preferred embodiment, RF are independently selected from the following group: H, methyl, ethyl.

In another preferred embodiment, Rand $^F$ are both H.

In another preferred embodiment,

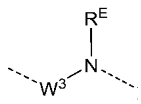

it is -C(O)-$CH_2$-NH- or -S(O)$_2$-NH-.

In another preferred embodiment, in Formula I, Ring A, Ring B, Ring C, L $^1$ , W $^2$ , W $^3$, $R^A$, RB, RC, $R^D$ , RE, $R^3$, $R^7$, R, subscript m1, subscript m2, subscript m3 and subscript m4 are each independently a corresponding group in the Example compounds or the specific compounds in Tables A-II, B-II and C-II.

In another preferred embodiment, in Formulas I-1 and I-2, Ring A, X, X1, X2, X3, X4, X7, subscript o1, subscript o2,

subscript o3, subscript o4, W1 , W2 , W3 , $R^A$, RB, RC, $R^D$, RE, R3, R7, R, subscript n1, subscript m1, subscript m2, subscript m3 and subscript m4 are each independently the corresponding groups in the Example compounds or the specific compounds in Tables A-II, B-II and C-II.

In another preferred embodiment, in Formulas I-3 and I-4, Ring A, X, X1 , X2, X3 , X4 , subscript o1, subscript o2, subscript o3, subscript o4, W2 , W3 , $R^A$, RB , RC , $R^D$, RE, $R^3$, R7 , R, subscript m1, subscript m2, subscript m3 and subscript m4 are each independently the corresponding groups in the Example compounds or the specific compounds in Tables A-II, B-II and C-II.

In another preferred embodiment, Ring A, Ring B, Ring C, L 1 , W 2 , $R^A$, RB, RC, RD, RE, $R^3$, R7, R, subscript m1 , subscript m2 , and subscript m3 may also be defined as in the first aspect.

In another preferred embodiment, the compound is selected from Table A-II

Table A-II

| Molecu lar number | Structural formula | Molecu lar number | Structural formula |
|---|---|---|---|
| 115-01 0 | 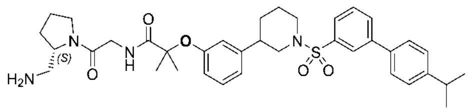 | 115-01 1 | 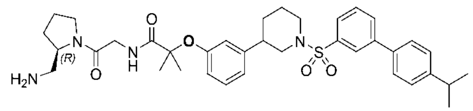 |
| 115-01 2 | 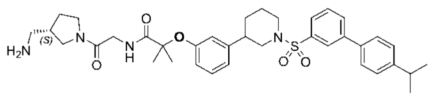 | 115-01 3 | 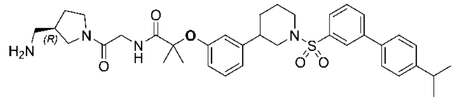 |
| 115-01 4 | 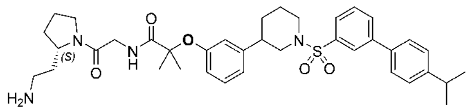 | 115-01 5 | 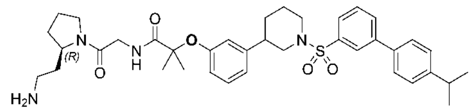 |
| 115-01 6 | 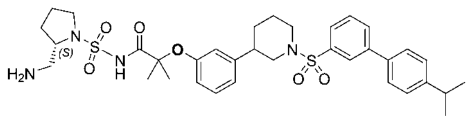 | 115-01 7 | 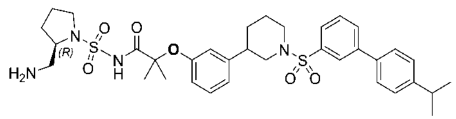 |
| 115-01 8 | 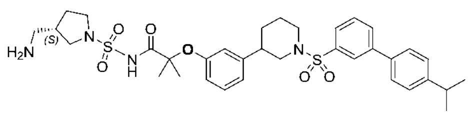 | 115-01 9 | 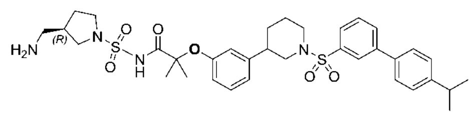 |
| 115-02 0 | 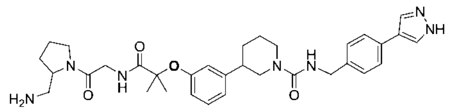 | 115-02 1 | 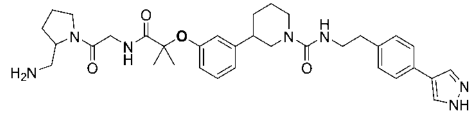 |
| 115-02 2 | 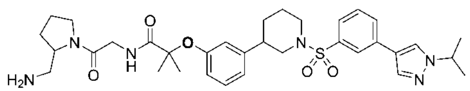 | 115-02 3 | 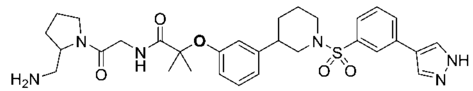 |
| | | 115-02 7 | 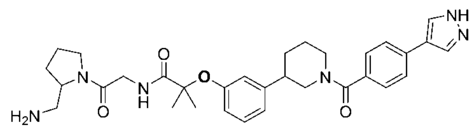 |

(continued)

| Molecu lar number | Structural formula | Molecu lar number | Structural formula |
|---|---|---|---|
| 115-02 8 | 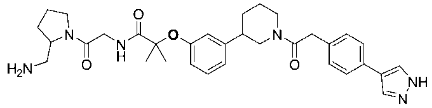 | 115-02 9 | 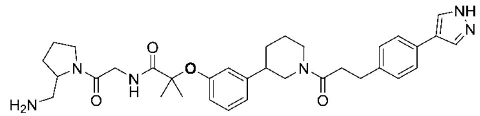 |
| 115-03 0 | 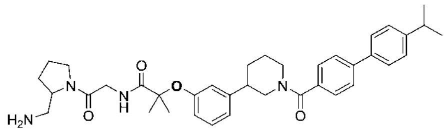 | 115-03 1 | 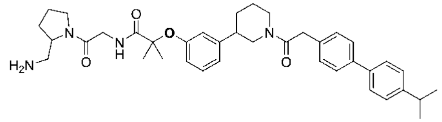 |

In another preferred embodiment, the compound is selected from Table B-II

Table B-II

| Molecular number | Structural formula | Molecular number | Structural formula |
|---|---|---|---|
| 115-04 6 | 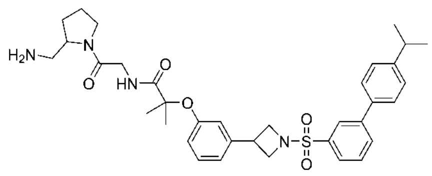 | 115-04 7 | 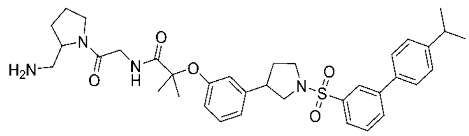 |
| 115-04 8 | 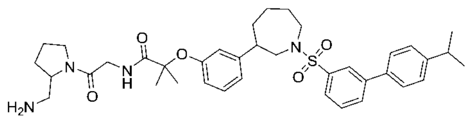 | 115-04 9 | 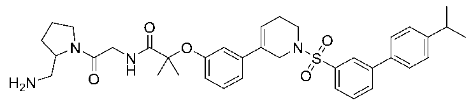 |
| 115-05 0 | 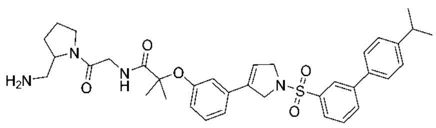 | 115-05 1 | 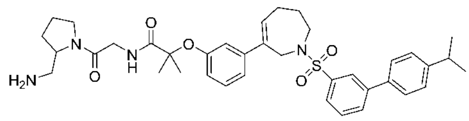 |

In another preferred embodiment, the compound is selected from Table C-II

Table C-II

| Molecular number | Structural formula | Molecular number | Structural formula |
|---|---|---|---|
| 115-05 2 | 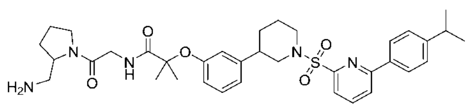 | 115-05 3 | 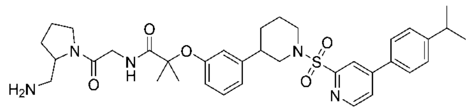 |
| 115-05 4 | 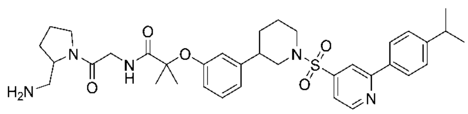 | 115-05 5 | 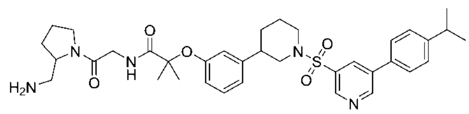 |
| 115-05 6 | 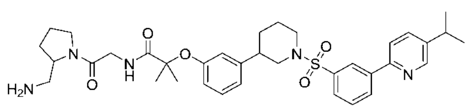 | 115-05 7 | 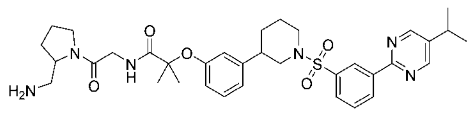 |
| 115-05 8 | 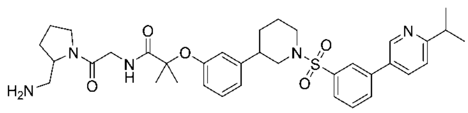 | 115-05 9 | 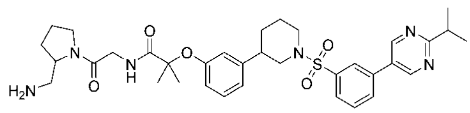 |
| 115-06 0 | 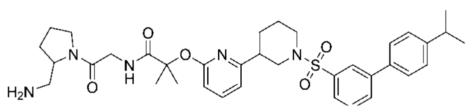 | 115-06 1 | 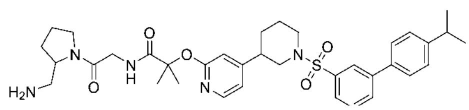 |
| 115-06 2 | 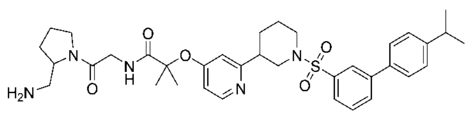 | 115-06 3 | 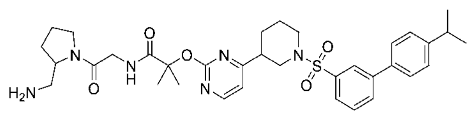 |

(continued)

| Molecular number | Structural formula | Molecular number | Structural formula |
|---|---|---|---|
| 115-06 4 | 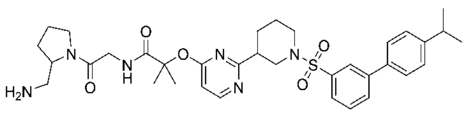 | 115-06 5 | 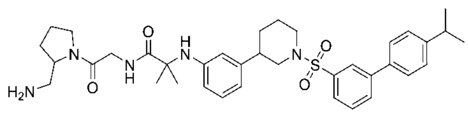 |
| 115-06 6 | 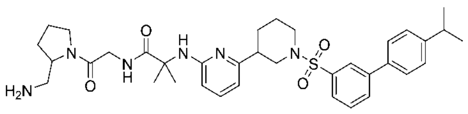 | 115-06 7 | 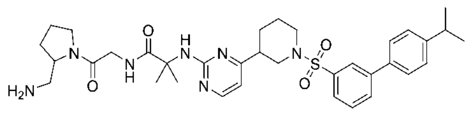 |
| 115-06 8 | 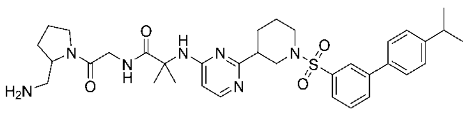 | 115-06 9 | 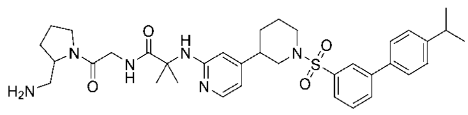 |

In a third aspect of the present invention, there is provided a pharmaceutical composition comprising:

(i) a compound according to the first or second aspect or a pharmaceutically acceptable salt thereof, or an isomer, solvate, crystal form or prodrug thereof; and
(ii) a pharmaceutically acceptable carrier or excipient.

In the fourth aspect of the present invention, provided is a use of the compound as described in the first or second aspect or a pharmaceutically acceptable salt thereof, or an isomer, solvate, crystal form or prodrug thereof in the preparation of a medicament for treating or preventing diseases associated with the BCL9/β-catenin interaction.

In another preferred embodiment, the diseases related to the interaction between BCL9 and β-catenin include cancer and tumor.

In the fifth aspect of the present invention, a method for treating or preventing a disease associated with the BCL9/β-catenin interaction is provided, comprising the steps of administering to a subject in need thereof a therapeutically effective amount of a compound as described in the first or second aspect, or a pharmaceutically acceptable salt thereof, or an isomer, solvate, crystalline form or prodrug thereof, or a pharmaceutical composition as described in the third aspect.

In another preferred embodiment, the diseases related to the interaction between BCL9 and β-catenin include cancer and tumor.

In the sixth aspect of the present invention, a method for treating, treating or preventing cancer is provided, comprising the steps of administering to a subject in need thereof a therapeutically effective amount of a compound as described in the first or second aspect or a pharmaceutically acceptable salt thereof, or an isomer, solvate, crystalline form or prodrug thereof, or a pharmaceutical composition as described in the third aspect.

In the seventh aspect of the present invention, there is provided a use of a compound as described in the first or second aspect or a pharmaceutically acceptable salt thereof, or an isomer, solvate, crystal form or prodrug thereof in the preparation of a medicament for treating or preventing fibrosis or its related diseases.

In another preferred embodiment, fibrosis or its related diseases include: pulmonary fibrosis, liver fibrosis, non-alcoholic steatohepatitis, bone fibrosis, or a combination thereof.

In the eighth aspect of the present invention, a method for treating or preventing fibrosis-related diseases is provided, comprising the steps of administering to a subject in need thereof a therapeutically effective amount of a compound as described in the first or second aspect or a pharmaceutically acceptable salt thereof, or an isomer, solvate, crystalline form or prodrug thereof, or a pharmaceutical composition as described in the third aspect.

In another preferred embodiment, fibrosis or its related diseases include: pulmonary fibrosis, liver fibrosis, non-alcoholic steatohepatitis, bone fibrosis, or a combination thereof.

In a ninth aspect of the present invention, there is provided an inhibitor of BCL9 binding to β-catenin in a subject; and/or modulating Wnt/β-catenin signaling in a subject; and/or reducing regulatory T cell survival in a subject; and/or reducing VEGF expression in a tumor in a subject; and/or increasing CD4+ T cells and CD8+ T cells infiltrating into a tumor in a subject; and/or increasing T helper 17 (Th17) cells infiltrating into a tumor in a subject; and/or reducing dendritic cells in a tumor in a subject; and/or when administered to a subject, increasing the half-life (T 112) of a tumor in a subject. A method for inhibiting tumor growth in a subject; and/or inhibiting the proliferation of cancer stem cells in a subject; and/or inhibiting tumor metastasis in a subject, comprising the steps of administering to a subject a compound as described in the first or

second aspect or a pharmaceutically acceptable salt thereof, or an isomer, solvate, crystalline form or prodrug thereof, or a pharmaceutical composition as described in the third aspect, or contacting a subject with a compound as described in the first or second aspect or a pharmaceutically acceptable salt thereof, or an isomer, solvate, crystalline form or prodrug thereof.

In another preferred embodiment, the subject is a mammal, preferably a human.

In another preferred embodiment, the subject is cell.

In another preferred embodiment, the method is non-therapeutic in vitro.

It should be understood that within the scope of the present invention, the above technical features of the present invention and the technical features specifically described in the following (eg, embodiments) can be combined with each other, thereby forming a new or preferred technical solution. Due to space limitations, it will not be repeated herein.

## DESCRIPTION OF THE DRAWINGS

none

## EMBODIMENTS FORCARRYING OUT THE INVENTION

After extensive and in-depth research, the inventors unexpectedly discovered a class of small molecule compounds with novel structures that exhibit excellent activity in inhibiting the interaction between BCL9 and β-catenin. Furthermore, the inventors discovered that these compounds have excellent therapeutic and preventive effects in fibrosis and related diseases. Based on this, the inventors completed the present invention.

## TERMS

Unless otherwise specified, herein, each term or abbreviation has the common meaning understood by those skilled in the art.

Unless otherwise specified, in this document, when a single bond in a compound structure 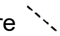 is represented by a dotted line ( ), it represents the connection point to the rest of the molecule.

As used herein, the term "containing", "comprising" or "including" means that the various components can be used together in the mixture or composition of the present invention. Therefore, the terms "mainly consisting of ..." and "consisting of ..." are within the term "comprising".

Unless otherwise stated, the term "alkyl", by itself or as part of another substituent, means a straight or branched chain hydrocarbon radical, having the number of carbon atoms designated (ie $C_{1-6}$means 1 - 6 carbons). Examples of alkyl groups include methyl, ethyl, *n* -propyl, isopropyl, *n* -butyl, *t* -butyl, *iso* -butyl, *sec* -butyl, *n* -pentyl, *n* -hexyl, *n* -heptyl, *n* -octyl, and the like.

The term "alkenyl" refers to an unsaturated alkyl group having one or more double bonds. Similarly, the term "alkynyl" refers to an unsaturated alkyl group having one or more triple bonds. Typically, alkenyl groups have 1 to 6 carbon atoms (i.e., $C_{1-6}$alkenyl) and alkynyl groups have 1 to 6 carbon atoms (i.e., $C_{1-6}$alkynyl). Examples of such unsaturated alkyl groups include ethenyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and higher homologs and isomers.

The terms "alkoxy", "alkylamino" and "alkylthio" (or thioalkoxy) are used in their conventional meanings, refer to those alkyl groups attached to the rest of the molecule via an oxygen atom, amino, or a sulfur atom, respectively. Additionally, for dialkylamino groups, the alkyl portions can be the same or different and can also be combined to form a 3-7 membered ring with the nitrogen atom to which each is attached. Accordingly, a group represented as $-NR^a R^b$ is meant to include piperidinyl, pyrrolidinyl, morpholinyl, azetidinyl and the like.

As used herein, the term "alkylene," by itself or as part of another substituent, refers to a divalent radical derived from an alkane, eg，-CH2-，-CH2CH2-·

As used herein, the term "aminoalkyl" refers to an alkyl group as defined above having the specified number of carbon atoms in which one or two hydrogen atoms are replaced by amino groups, for example, $-(CH_2)_2NH_2$.

As used herein, the term "cycloalkyl" refers to a saturated hydrocarbon ring having a specified number of ring atoms (e.g., C3-10 cycloalkyl , preferably C3-6 cycloalkyl ). "Cycloalkyl" can be a monocyclic ring (e.g., cyclopropyl, cyclobutyl, cyclohexyl, etc.), or a bicyclic and polycyclic hydrocarbon ring (including cyclic, spirocyclic, bridged, etc.), such as bicyclo[2.2.1]heptane, bicyclo[2.2.2]octane, etc. The term "heterocycloalkyl" refers to a cycloalkyl group containing one to five (preferably 1, 2, 3, or 4) heteroatoms selected from N, O, and S, wherein the nitrogen and sulfur atoms are optionally oxidized and the nitrogen atom is optionally quaternized. Heterocycloalkyl groups can be monocyclic, bicyclic, or polycyclic ring systems (including cyclic, spirocyclic, bridged, etc.). In general, heterocyclyl groups typically include 4-10 ring atoms (i.e., 4 to 10-membered heterocyclyl groups), preferably, include 4-7 (e.g., 4, 5, 6) ring atoms (i.e., 4 to 7-membered heterocyclyl groups, or 4 to 6-membered heterocyclyl groups) and contain 1, 2, 3, or 4 (preferably 1 or 2) heteroatoms. Non-limiting examples of heterocyclyl groups include pyrrolidine, imidazolidine, pyrazolidine, butyrolactam, valerolactam, imidazolidinone, hydantoin, dioxolane, phthalimide, piperidine, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinuclidine, and the like. Heterocyclyl groups can be attached to the remainder of the molecule via a ring carbon or heteroatom (e.g., ring nitrogen).

As used herein, the term "cycloalkenyl", used alone or as part of a group, refers to a cyclic hydrocarbon having the specified number of ring atoms (e.g., C3-10 cycloalkenyl, or C3-6 $_{cycloalkenyl)}$and having one or two double bonds (preferably, only one double bond) between the ring vertices. "Cycloalkenyl" can be a monocyclic ring, and can also refer to bicyclic and polycyclic hydrocarbon rings (including cyclic, spirocyclic, bridged, etc.). Examples of cycloalkenyls include, for example, cyclopropene, cyclobutene, cyclopentene, cyclopentadiene, etc. Similarly, the term "heterocycloalkenyl" refers to a cycloalkenyl containing 1 to 5 (preferably 1, 2, 3 or 4) heteroatoms selected from N, O and S, wherein the nitrogen and sulfur atoms are optionally oxidized and the nitrogen atom is optionally quaternized. Heterocycloalkenyls can be monocyclic, bicyclic or polycyclic ring systems (including cyclic, spirocyclic, bridged, etc.). In general, a heterocycloalkenyl group typically includes 4 to 10 ring atoms (i.e., a 4- to 10-membered heterocycloalkyl group), preferably, includes 4 to 7 (e.g., 4, 5, 6) ring atoms (i.e., a 4- to 7-membered heterocyclyl group, or a 4- to 6-membered heterocyclyl group) and contains 1, 2, 3 or 4 (preferably 1 or 2) heterocyclic atoms.

For terms such as cycloalkylalkyl(alkylene) and heterocycloalkylalkyl(alkylene), it means that the cycloalkyl or heterocycloalkyl group is attached to the remainder of the molecule through an alkyl or alkylene linker. For example, cyclobutylmethyl - is a cyclobutyl ring that is attached to a methylene linker to the remainder of the molecule.

The term "aryl" means, unless otherwise stated, a polyunsaturated, typically aromatic, hydrocarbon group which can be a single ring or multiple rings (up to three rings) which are fused together or linked covalently. Generally, aryl has 6 to 10 ring atoms. Nitrogen atom(s) are optionally quaternized. Generally, the heteroaryl has 5 to 10 ring atoms, ie 5-10 membered heteroaryl, preferably, has 5 to 6 ring atoms, ie 5-6 membered heteroaryl, and contains 1, 2, 3, or 4 heteroatoms. A heteroaryl group can be attached to the remainder of the molecule through a heteroatom. Non-limiting examples of aryl groups include phenyl, naphthyl, and biphenyl, while non-limiting examples of heteroaryl groups include pyridyl, pyridazinyl, pyrazinyl, pyrimidinyl, triazinyl, quinolinyl, quinoxalinyl, quinazolinyl, cinnolinyl, phthalazinyl, benzotriazinyl, purinyl, benzimidazolyl, benzopyrazolyl, benzotriazolyl, benzisoxazolyl, isobenzofuranyl ... ] furyl), isoindolyl, indolizinyl, benzotriazinyl, thienopyridinyl, thienopyrimidinyl, pyrazolopyrimidinyl, imidazopyridine, benzothiazolyl, benzofuranyl, benzothiophenyl, indolyl, quinolyl, isoquinolyl, isothiazolyl, pyrazolyl, indazolyl, pteridinyl, imidazolyl, triazolyl, tetrazolyl, oxazolyl, isoxazolyl, thiadiazolyl, pyrrolyl, thiazolyl, furanyl, thienyl, and the like. Substituents for the above-stated aryl and heteroaryl ring systems are selected from the group of acceptable substituents described below.

Thus, the term "aralkyl" is meant to include those groups in which an aryl group is attached to an alkyl group that is attached to the rest of the molecule (e.g., benzyl, phenethyl, pyridylmethyl, etc.).

In some embodiments, the above terms (such as "alkyl," "aryl," and "heteroaryl") will include both substituted and unsubstituted forms of the designated radical. The preferred substituents for each type of group are provided below. For brevity, the terms aryl and heteroaryl will refer to substituted or unsubstituted versions as provided below, while the term "alkyl" and related aliphatic radicals is meant to refer to unsubstituted version, unless indicated to be substituted.

Substituents for alkyl groups (including those groups commonly referred to as alkylene, alkenyl, alkynyl and cycloalkyl) can be various groups selected from the group consisting of -halogen, -OR', -NR'R", -SR', -SiR'R"R"', -OC(O) R' , -C(O)R', -CO2R', -CONR'R", -OC(O)NR'R", -NR"C(O)R', -NR'-C(O)NR"R"', -NR"C(O)$_{2R}$', -S(O)R', -S(O)$_{2R}$', -S(O)$_{2NR'R}$", -NR'S(O)$_{2R}$", -CN and -NO2 , in an amount ranging from zero to (2m'+1), where m' is the total number of carbon atoms in such group. R', R" and R"' each independently refer to hydrogen, unsubstituted C1-8 alkyl , unsubstituted heteroalkyl, unsubstituted When R' and R" are attached to the same nitrogen atom, they can be combined with the nitrogen atom to form a 3-, $_{4-}$ , $_{5-}$ , $_{6-}$ , or 7-membered ring. For example, -NR'R" is meant to include 1-pyrrolidinyl and $_4$ -morpholinyl. The term "acyl" as used by itself or as part of another group refers to groups wherein two substitutents on the carbon that is closest to the point of attachment for the radical is replaced with the substitutent =0 (e.g., $C(O)CH_3$, $-C(O)CH_2CH_2$ OR' and the like).

Similarly, substituents for aryl and heteroaryl groups are numerous and are typically selected from: -halogen, -OR', -OC(O)R', -NR'R", -SR', -R', -CN, -NO2 , -CO2R', -CONR'R" , -C(O)R' , -OC(O)NR'R", -NR"C(O)R', -NR"C(O)$_2$R', $_{-NR'}$ -C(O)NR"R"', -S(O)R', -S(O)$_2$R', s ( -O)$_{2NR'R}$", -NR'S(O)$_{2R}$", -N3 , perfluoro(C1 $_-$ C4)alkoxy, and perfluoro(C1 $_-$ C4)$_{alkyl, in a number from zero to the}$ total number of open valences on the aromatic ring system; wherein R', R" and R"' are independently selected from hydrogen, $_{C1-8}$alkyl , C $_{3-6}$ cycloalkyl, C $_{2-8}$ alkenyl, C $_{2-8}$ alkynyl, unsubstituted aryl and heteroaryl, (unsubstituted aryl)-$C_{1-4}$alkyl and unsubstituted aryloxy-$C_{1-4}$alkyl. Other suitable substituents include each of the above aryl substituents attached to a ring atom by an alkylene tether of from 1-4 carbon atoms.

As used herein, the term "heteroatom" is meant to include oxygen (O), nitrogen (N), sulfur (S) and silicon (Si).

As used herein, the term "halogen" refers to F, Cl, Br and I. More preferably, the halogen atom is selected from F, Cl, and Br.

For the compounds provided herein, a bond that is drawn from a substituent (typically an Rgroup) to the center of an aromatic ring (eg, benzene, pyridine, and the like) will be understood to refer to a bond providing a connection at any of the available vertices of the aromatic ring. In some embodiments, this description also includes connections fused to the rings of the aromatic ring. For example, a bond drawn to the center of the benzene portion of an indole, will indicate a bond to any available vertex of the six- or five-membered ring portions of the indole.

Preferably, herein, unless otherwise specified, when a single bond in a certain compound structure is represented by "", 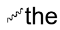 the compound includes a compound in which the single bond is a single configuration of S configuration or Rconfiguration, or a mixture of S configuration and Rconfiguration (such as a racemate).

## Active Ingredients

As used herein, the term "compound of the present invention" refers to the compound as described in the first aspect of the present invention. The term also includes various crystalline forms, pharmaceutically acceptable salts, hydrates or solvates of the compound as described in the first aspect of the present invention.

As used herein, the term "pharmaceutically acceptable" component(s) refers to a substance suitable for use in human and/or animals without excessive adverse side reactions (such as toxicity, stimulation and allergic reaction), ie a substance with reasonable benefit/risk ratio.

As used herein, the term "therapeutically effective dose" refers to any amount of a drug that, when used alone or in combination with another therapeutic agent, promotes disease regression, as evidenced by a decrease in the severity of disease symptoms, an increase in the frequency and duration of disease-free periods, or prevents impairment or disability resulting from the disease. A "therapeutically effective dose" of a drug of the present invention also includes a "prophylactically effective dose," which is any amount of a drug that, when administered alone or in combination with another therapeutic agent to a subject at risk of developing a disease or experiencing recurrence of a disease, inhibits the onset or recurrence of the disease.

The term "pharmaceutically acceptable salts" is meant to include salts of the active compounds which are prepared with relatively nontoxic acids or bases, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Examples of salts derived from pharmaceutically acceptable inorganic bases include aluminum, ammonium, calcium, copper, iron, ferrous, lithium, magnesium, manganese, manganous, potassium, sodium, zinc, and the like. Salts derived from pharmaceutically-acceptable organic bases include salts of primary, secondary and tertiary amines, including substituted amines, cyclic amines, naturally-occuring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. When compounds of the present disclosure contain relatively basic functionalities, acid addition salts can be obtained by contacting the neutral form of such compounds with a sufficient amount of the desired acid, either neat or in a suitable inert solvent. monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, malonic, benzoic, succinic, suberic, fumaric, mandelic, phthalic, benzenesulfonic, *p* -tolylsulfonic, citric, tartaric, methane sulfonic, and the like. Also included are salts of amino acids such as arginate and the like, and salts of organic acids like glucuronic or galactunoric acids and the like (see, for example, Berge, SM, et al, "Pharmaceutical Salts", Journal of Pharmaceutical Science, 1977, 66, 1-19). Certain specific compounds of the present disclosure contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms thereof in certain physical properties, such as solubility in polar solvents, but in addition to the above, those salts are equivalent to the parent form of the compound for the purposes of the present invention.

In addition to salt forms, the invention also provides compounds in prodrug form. Prodrugs of the compounds described herein are those compounds that readily undergo chemical changes under physiological conditions to provide the compounds of the present disclosure. Additionally, prodrugs can be converted to the compounds of the present disclosure by chemical or biochemical methods in an ex vivo environment. For example, when placed in a transdermal patch reservoir containing suitable enzymes or chemical reagents, the prodrug can be slowly converted to the compound of the invention.

Certain compounds of the present disclosure may exist in unsolvated forms as well as solvated forms (i.e., solvates), including hydrated forms (i.e., hydrates). The solvated forms are generally equivalent to the non-solvated forms and should be included in the scope of the present invention. Certain compounds of the present disclosure may exist in

multiple crystallie or amorphous forms. Generally, as for then application considered in the present invention, all physical forms are equivalent and should be included in the scope of the present invention.

Certain compounds of the present disclosure possess asymmetric carbon atoms (optical centers) or double bonds; the racemates, diastereomers, geometric isomers, regioisomers and individual isomers (eg, separate enantiomers) are all intended to be encompassed within the scope of the present disclosure. When compounds are provided herein with an identified stereochemistry (indicated as Ror S, or with dashed or wedge bond designations), those compounds will be understood by one of skill in the art to be substantially free of other isomers (eg, at least 80%, 90%, 95%, 98%, 99%, and up to 100% free of the other isomers).

The compounds of the present disclosure may also contain unnatural proportions of isotope atomic isotope at one or more of isotopic atoms that constitute such compounds. The unnatural proportions of certain isotope can be defined as the amount from the naturally found amount of the atom discussed to 100% $^{of}$ that $^{atom}$ . carbon-14 ( $^{14}$ C), or non-radioactive isotopes, such as deuterium ( $^{2}$ H) or carbon-13 ( $^{13}$ C). Such isotopic variants may provide additional uses in addition to those described in this application. For instance, isotopic variants of the compounds of the disclosure may find additional utility, including but not limited to, as diagnostic and/or imaging reagents, or as cytotoxic/radiotoxic therapeutic agents. Additionally, isotopic variants of the compounds of the disclosure can have altered pharmacokinetic and pharmacodynamic characteristics which can contribute to enhanced safety, tolerability or efficacy during treatment. All isotopic variations of the compounds of the present disclosure, whether radioactive or not, should be encompassed within the scope of the present disclosure.

**pharmaceutical compositions** and **administration methods**

Since the compounds of the present invention have excellent inhibitory activity against the BCL9/β-catenin interaction (BCL9/β-catenin PPI), the compounds of the present invention and their various crystalline forms, pharmaceutically acceptable inorganic or organic salts, hydrates or solvates, and pharmaceutical compositions containing the compounds of the present invention as the main active ingredient can be used to treat, prevent, and alleviate diseases associated with the BCL9/β-catenin interaction. According to the prior art, the compounds of the present invention can be used to treat the following diseases: cancer, tumors, etc., for example, familial adenomatous polyposis (FAP), eye cancer, rectal cancer, colon cancer, colorectal cancer, cervical cancer, prostate cancer, breast cancer, bladder cancer, oral cancer, benign and malignant tumors, stomach cancer, liver cancer, pancreatic cancer, lung cancer, uterine corpus, ovarian cancer, prostate cancer, testicular cancer, kidney cancer, brain/CNS cancer, laryngeal cancer, multiple myeloma, skin melanoma, acute lymphocytic leukemia, acute myeloid leukemia, Ewing's sarcoma, Kaposi's sarcoma, basal cell carcinoma and squamous cell carcinoma, small cell lung cancer, choriocarcinoma, rhabdomyosarcoma, angiosarcoma, hemangioendothelioma, Wilms' tumor, neuroblastoma, oral/pharyngeal cancer, esophageal cancer, laryngeal cancer, lymphoma, neurofibromatosis, tuberous sclerosis, hemangioma, gastric cancer, ovarian cancer, hepatocellular carcinoma, lymphatic vessels, etc.

Furthermore, the compounds of the present invention exhibit excellent therapeutic activity for fibrosis. Therefore, the compounds of the present invention, their various crystalline forms, pharmaceutically acceptable inorganic or organic salts, hydrates, or solvates, and pharmaceutical compositions containing the compounds of the present invention as the primary active ingredient can be used to treat, prevent, and alleviate fibrosis and fibrosis-related diseases. Fibrosis can occur in a variety of organs, with the primary pathological changes being an increase in fibrous connective tissue and a decrease in parenchymal cells within organ tissues. Continued progression can lead to structural destruction and decreased function, even to organ failure, posing a serious threat to human health and life.

Exemplary diseases of fibrosis and its related diseases are listed below:

| **major organs** | **Typical diseases** and **syndromes** |
|---|---|
| **1 Lungs** | **Diseases with known causes:** Occupational diseases caused by inorganic dust (silicosis, asbestosis, coal lung, etc.); organic dust and allergic pneumonia (farmer's lung, air conditioning humidifier lung, pigeon breeder's lung, bagasse pneumoconiosis, etc.); diseases related to drugs/treatments (antibiotics, nonsteroidal anti-inflammatory preparations, cardiovascular drugs, anti-tumor drugs, oral hypoglycemic drugs, oxygen, morphine, etc.); infectious diseases (tuberculosis, viral pneumonia, pneumocystis jiroveci infection, etc.); secondary lung diseases (left heart failure, congenital heart disease, adult respiratory distress syndrome, chronic heart failure-related, transplant rejection-related lung diseases). diseases, etc.); **diseases of** |

(continued)

| major organs | Typical diseases and syndromes |
|---|---|
| | **unknown etiology** : primary lung diseases (idiopathic interstitial pneumonia, bronchiolitis obliterans with organizing pneumonia, pulmonary lymphangioleiomyomas, etc.); lung diseases associated with collagen vascular disease (systemic lupus erythematosus, rheumatoid arthritis, progressive systemic sclerosis, polymyositis, dermatomyositis, mixed connective tissue disease, etc.); alveolar filling diseases (diffuse alveolar hemorrhage syndrome, pulmonary alveolar proteinosis, eosinophilic pneumonia, pulmonary vasculitis, lymphocytic interstitial pneumonia, necrotizing nodular granuloma, familial pulmonary fibrosis, etc.) |
| **2 Cardiovascular system** | Ischemic heart disease (replacement and interstitial fibrosis after myocardial infarction); hypertensive heart disease; inflammatory cardiomyopathy (viral myocarditis); metabolic cardiomyopathy (hemochromatosis cardiomyopathy, amyloid cardiomyopathy, glycogen accumulation cardiomyopathy, diabetic cardiomyopathy, etc.); Keshan disease; dilated cardiomyopathy; hypertrophic cardiomyopathy, restrictive cardiomyopathy; arrhythmogenic right ventricular cardiomyopathy, etc. |
| **3. Liver** | Viral cirrhosis (hepatitis B, C, and D); schistosomal cirrhosis; alcoholic cirrhosis; biliary cirrhosis (primary biliary cirrhosis, secondary gallstones, periportal inflammation); metabolic cirrhosis (Wilson's disease, hemochromatosis); toxic cirrhosis (organophosphorus poisoning, carbon tetrachloride poisoning, hepatotoxic drugs such as isoniazid, tetracycline, chlorpromazine poisoning); nutritional cirrhosis; cardiac cirrhosis (chronic congestive heart failure) |
| **4. Pancreas** | Acute pancreatitis; pancreatic duct obstruction; chronic alcoholism; sphincter of Oddi dysfunction; pancreatic ischemia, etc. |
| **5. Kidneys** | Vascular (hypertension); immune (glomerulonephritis, systemic lupus erythematosus, scleroderma, renal transplant rejection); infectious (pyelonephritis, kidney stones); metabolic (hyperlipidemia, diabetes, hyperuricemia, hypercalciuria), etc. |
| **6. Spleen** | Splenic fibroproliferative disease |
| **7 Eyes** | Diabetic retinal fibroplasia after eye trauma and surgery |
| **8 Nervous System** | Post-spinal cord trauma, stroke scarring, Alzheimer's disease |
| **9 bone marrow** | Idiopathic and drug-induced myelofibrosis, polycythemia vera, chronic myeloid leukemia, Hodgkin's disease |

The pharmaceutical composition of the invention comprises the compound of the present invention or the pharmaceutically acceptable salts thereof in a safe and effective dosage range, and pharmaceutically acceptable excipients or carriers. In which, "safe and effective amount" is meant that the amount of the compound is sufficient to significantly improve the condition without causing serious side effects. invention/dose. Preferably, the "one dose" is one capsule or one pill.

Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agents (such as sodium lauryl sulfate), colorants, flavorings, stabilizers, antioxidants, preservatives, pyrogen-free water, etc.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets, and pills, the dosage form may also include a buffer.

Solid dosage forms such as tablets, sugar pills, capsules, pills, and granules can be prepared using coating and shell materials, such as casings and other well-known materials in the art. They can contain an opaque agent. The release of the active compounds or compounds in the compositions can be released in a delayed mode in a given portion of the digestive tract. one or more of the aforementioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition can also include additives such as wetting agents, emulsifiers and suspending agents, sweeteners, corrigents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with a physiologically acceptable carriers and any preservatives, buffers, or propellants that may be necessary.

The compounds of the present invention can be administered separately or in combination with other pharmaceutically acceptable compounds.

In some embodiments, the pharmaceutical composition comprising the compounds of the present invention may further comprise at least one additional agent. In some embodiments, the at least one additional agent is selected from one or more of a checkpoint inhibitor, an EGFRinhibitor, a VEGF inhibitor, a VEGFRinhibitor, and an anticancer drug.

In some embodiments, the pharmaceutical compositions described herein may include checkpoint inhibitors. In one embodiment, the checkpoint inhibitor is an anti-PD-1 antibody, an anti-PD-L1 antibody, or an anti-CTLA4 antibody. In one embodiment, the checkpoint inhibitor targets and stimulates checkpoint molecules, for example, CD27, CD40, OX40, GITR, or CD138. In another embodiment, the checkpoint inhibitor targets and stimulates checkpoint molecules, for example, A2AR, B7-H3, B7-H4, B and T lymphocyte attenuation factor (BTLA), indoleamine 2,3-dioxygenase

(IDO), killer cell immunoglobulin-like receptor (KIR), lymphocyte activation gene-3 (LAG3), T cell immunoglobulin and mucin domain protein 3 (TIM-3), VISTA (C10orf54), or T cell activation V domain Ig inhibitor.

In some embodiments, the pharmaceutical compositions described herein include an EGFRinhibitor. In one embodiment, the EGFRinhibitor is erlotinib, gefitinib, lapatinib, panitumumab, vandetanib, or cetuximab.

In some embodiments, the pharmaceutical compositions described herein may include a VEGF or VEGFRinhibitor. In one embodiment, the VEGF or VEGFRinhibitor is pazopanib, Avastin, sorafenib, sunitinib, axitinib, ponatinib, carboplatin, vandetanib, cabozantinib, ramucirumab, lenvatinib, or aflibercept.

In some embodiments, the pharmaceutical compositions described herein include an anticancer drug. The anticancer drug can be selected from the group consisting of cyclophosphamide, methotrexate, 5-fluorouracil (5-FU), doxorubicin, mustine, vincristine, procarbazine, penicillin, dacarbazine, bleomycin, etoposide, cisplatin, epirubicin, capecitabine, folinic acid, actinomycin, all-trans retinoic acid, azacitidine, azathioprine, bortezomib, carboplatin, chlorambucil, cytarabine, Daunorubicin, Europaclitaxel, doxifluridine, fluorouracil, gemcitabine, hydroxyurea, idarubicin, imatinib, elenodicon, mechlorethamine, mercaptopurine, mitoxantrone, paclitaxel, pemetrexed, teniposide, thioguanine, toprofen, valrubicin, vinblastine, vindesine, vinpoline, and oxaliplatin.

When the pharmaceutical composition is used, a safe and effective amount of the compound of the present invention is administered to a mammal (such as a human) in need of treatment, where the dosage at the time of administration is the pharmaceutically effective dosage, for people having a body weight of 60kg, the daily dose is usually 1~2000mg, preferably 20~500mg. Of course, the specific dosage should also consider factors such as the route of administration and the patient's health status, which are within the skill range of a skilled physician.

BCL-9, β-catenin, and Wnt signaling

Aberrant activation of Wnt signaling is implicated in a variety of cancers, as tumors can become dependent on Wnt signaling for growth and survival. Up to 90% of all sporadic colorectal cancer cases are associated with constitutive activation of Wnt signaling.

β-catenin is a protein that can participate in protein-protein interactions that stimulate Wnt signal transduction, resulting in changes in transcriptional activation that may cause tumor growth and development. β-catenin is usually phosphorylated and targeted for degradation by the Axin complex. If there is stimulation of the Wnt signal transduction pathway, unphosphorylated β-catenin accumulates and binds to lymphoid enhancer factor/T cell factor (LEF/TCF) and translocates to the nucleus to stimulate transcription of Wnt target genes. Wnt target genes include c-myc and CD44, which are upregulated genes in tumor models. BCL9 is a protein required for efficient β-catenin-mediated transcription in

mammalian cells.

"Canonical" Wnt/β-catenin signaling is a pathway activated by binding of Wnt ligands to the Frizzled family of cell-surface receptors, which then regulate the expression and intracellular localization of β-catenin. In the absence of Wnt ligands, β-catenin is phosphorylated and ubiquitinated within a destruction complex composed of adenomatous polyposis coli (APC), glycogen synthase kinase-3 (GSK-3), casein kinase-1 (CK1), and Axin, and targeted for degradation in a proteasome-dependent manner. In the presence of Wnt ligands, β-catenin ubiquitination within the complex is inhibited, leading to saturation of phosphorylated β-catenin, which is then stabilized and translocated to the nucleus. There, phosphorylated β-catenin engages nuclear T-cell factor (TCF) transcription factors, such as lymphoid enhancer factor/3 (LEF/TCF), to induce the expression of genes that promote cell proliferation, migration, and survival, including c-Myc and Cyclin D.

Several molecules comprising BCL9 and its homologue B-cell lymphoma 9-like (B9L) have been shown to be coactivators of Wnt/β-catenin transcription. The formation of a complex consisting of TCF, β-catenin and BCL9 (or B9L) enhances the Wnt transcriptional activity that β-catenin depends on. In normal cells, when the Wnt ligand is uncoupled from its receptor, this transcriptional pathway is shut down. However, various loss-of-function mutations in APC and Axin, as well as activating mutations in β-catenin itself, enable β-catenin to escape the destruction complex and accumulate in the nucleus. This type of inappropriate persistence of β-catenin promotes a wide range of common human epithelial cancers, including hepatocellular carcinoma, breast cancer, colorectal cancer and hematological malignancies, such as the occurrence of multiple myeloma. In addition, active β-catenin signal transduction leads to T cell rejection, particularly CD8+ T cell rejection, which leads to therapy resistance and shortens patient survival time. Therefore, blocking Wnt signaling by targeting β-cat may provide a powerful avenue for the treatment of CRC, thereby potentially preventing tumor initiation and metastasis.

Similar to other transcription factors, the development and translation of selective, nontoxic β-catenin inhibitors to the clinic has proven to be a considerable challenge, as β-catenin interacts with most of its protein partners through a common binding surface. Consequently, Wnt pathway inhibitors targeting this common binding surface have exhibited significant adverse effects in animal models and clinical trials. Only a few drugs targeting β-catenin are in clinical trials, including PRI-724 (Eisai Pharmaceuticals; Phase II), LGK974 (Novartis; Phase I), and OMP-54F28 and OMP-18R5 (OncoMed/Bayer; Phase I). Furthermore, disrupting the LEF/TCF interaction through small molecule and peptide inhibitors of β-catenin can produce severe side effects, including severe myeloid dysplasia, anemia, and systemic wasting in treated mice-a potential consequence of disrupting homeostatic Wnt signaling in normal hematopoietic and intestinal stem cells. These therapeutic limitations may arise from disruption of both β-catenin-TCF and β-catenin-E-cadherin interactions, which could compromise epithelial tissue integrity. In addition, biopharmaceuticals targeting Frizzled receptors (OMP-54F28 and OMP-18R5) have shown significant bone marrow toxicity during clinical trials. Wnt ligands are required for Wnt/β-cat activation, but APC and β-catenin mutations in cancer cells can induce downstream transcription without Wnt ligand activation, so blocking Wnt secretion cannot inhibit endogenous oncogenic Wnt activity caused by APC and β-catenin mutations inducing downstream gene transcription. As indicated by certain biomarkers, LGK974 targets only a small patient population. PRI-724, a small molecule inhibitor, is undergoing Phase II trials using daily infusions, but intravenous (IV) dosing more than once a week has shown undesirable and untenable properties for clinical development.

Traditionally, the Wnt signaling pathway comprises three different types of signal transduction: the canonical Wnt signaling pathway, in which Wnt regulates various transcriptional target genes in a β-catenin-dependent manner; the non-canonical Wnt signaling pathway, which is primarily involved in planar cell polarity, in which Wnt can function independently of β-catenin; and the non-canonical Wnt/calcium pathway, which regulates intracellular calcium levels. In this application, "canonical Wnt signaling" is interchangeably referred to as "canonical Wnt/β-catenin signaling" or "Wnt signaling." As described herein, canonical Wnt/β-catenin signaling can refer to pathway components that control the amount of β-catenin in a patient or sample by transducing the stability of β-catenin. In some embodiments, canonical Wnt/β-catenin signaling includes pathway components that transcriptionally transduce one or more genes such as c-myc, ccnd1, cd44, LGR5, VEGFA, AXIN2, and LEF1. In some embodiments, canonical Wnt/β-catenin signaling includes pathway components that are transregulated by the interaction between β-catenin and BCL9. In some embodiments, canonical Wnt/β-catenin signaling includes one or more genes that are transcriptionally controlled by the interaction between β-catenin and BCL9. The one or more genes controlled by the interaction between β-catenin and BCL9 may include c-myc, ccnd1, cd44, LGR5, VEGFA, AXIN2, and LEF1. In some embodiments, canonical Wnt/β-catenin signaling includes one or more proteins whose transcriptional expression is transregulated by the interaction between β-catenin and BCL9. These components may include, for example, c-Myc, Cyclin D1, CD44, LGR5, VEGFA, AXIN2, and LEF1.

## How to use

In some embodiments, administering a compound of the present invention to a subject inhibits Wnt signaling in the subject. In some embodiments, administering a compound of the present invention inhibits the binding of BCL9 to β-catenin. In some embodiments, administering a compound of the present invention inhibits canonical Wnt/β-catenin signaling. In some embodiments, administering a compound of the present invention treats a disease in a subject.

In some embodiments, the compounds of the present invention are capable of inhibiting the binding of BCL9 to β-catenin in vitro and/or in vivo. In some embodiments, the compounds of the present invention have one or more improved effects. The one or more effects may be selected from one or more of the following: (1) inhibiting the binding of BCL9 to β-catenin; (2) inhibiting canonical Wnt signaling; (3) reducing regulatory T cell survival; (4) reducing VEGF expression in tumors; (5) increasing CD4+ T cells and CD8+ T cells infiltrating into tumors; (6) increasing T helper 17 (Th17) cells entering tumors; (7) reducing dendritic cells in tumors; (8) having a half-life (T1/2) greater than at least 2 hours when administered to a subject; (9) inducing a tumor microenvironment that is conducive to an immune response; and (10) inhibiting tumor growth, tumor stem cell proliferation and/or tumor metastasis.

In some embodiments, compounds of the invention exhibit advantageous biological functions in some or each of the categories listed above, for example, potency in various biochemical and cellular biological assays, including cell-based Wnt and/or β-catenin transcription assays.

BCL9 binds to β-catenin

Pygopus (Pygo) and Legless (Lgs) were discovered in Drosophila as new components of Wnt signaling that are essential for armadillo-mediated transcription during normal development. Pygo and BCL9/Legless transduce Wnt signals by promoting the transcriptional activity of β-catenin/Armadillo in normal and malignant cells. The ability of a compound to inhibit the binding of BCL9 to β-catenin can be assessed in various assays inhibited in the art. In some embodiments, a homogeneous time-resolved fluorescence (HTRF) binding assay can be used to assess the ability of a compound of the present invention to inhibit the binding of BCL9 to β-catenin. In this assay, a compound/small molecule is bound to a label that can recognize another label attached to a target protein (i.e., β-catenin) that is attached to another label. When the compound/small molecule binds to the target protein and the two labels are therefore adjacent, a signal is generated and can be quantitatively read to calculate the binding affinity of the compound/small molecule. In some embodiments, the binding affinity of a compound/small molecule in this assay is compared to the binding affinity of a control to detect improved binding affinity compared to the binding affinity of the control.

In some embodiments, the ability of the compounds of the present invention to inhibit the binding of BCL9 to β-catenin can be assessed in an amplified luminescent proximity homogeneous assay (ALPHA). In this assay, the compound is conjugated to a donor bead and its target protein (i.e., β-catenin) is attached to an acceptor bead. When the two beads are close due to the binding of the compound to the target protein, a signal is generated and the binding affinity of the compound can be quantitatively calculated. In some embodiments, the binding affinity of the compound in this assay is compared to the binding affinity of a vehicle or a control to detect an improved binding affinity compared to the binding affinity of the vehicle or control.

In various embodiments, the ability of the compounds of the present invention to inhibit the binding of BCL9 to β-catenin can be assessed in a Wnt transcription assay. In some embodiments, the Wnt transcription assay is a cell-based assay. In some embodiments, the cell-based Wnt transcription assay is a β-lactamase (bla) reporter assay. Various cell lines derived from healthy subjects or subjects with disease, transformed cell lines, or primary cells can be used for this assay. Cell lines known to rely on classical Wnt/β-catenin signal transduction for their survival can also be used. In some embodiments, CellSensor ™ LEF/TCF-bla HCT-116 cells and Cignal Wnt reporter are used for this reporter assay. These cells contain a β-lactamase (BLA) reporter gene under the control of a β-lactamase/LEF/TCF response element stably integrated into HCT-116 cells. Because cells constitutively express β-lactamase, adding a compound that inhibits the binding of BCL9 to β-catenin in this assay reduces the production of β-lactamase. Therefore, the potency of compounds to inhibit Wnt transcription can be quantified in this assay.

In some embodiments, the ability of the compounds of the present invention to inhibit the binding of BLC9 to β-catenin can be assessed in a cell viability assay. In some embodiments, the cell viability assay is a CellTiterGlo luminescence assay, in which cell viability is quantitatively measured. Various cell lines, transformed cell lines, or primary cells derived from healthy subjects or subjects with disease can be used in this assay.

**Canonical Wnt/β-catenin signaling**

In certain embodiments, the ability of the compounds of the present invention to inhibit canonical Wnt/β-catenin signaling can be assessed in various in vitro and/or in vivo assays. In some embodiments, the effects of the compounds of the present invention on canonical Wnt/β-catenin signaling are assessed in a cell-based Wnt transcription assay, such as a β-lactamase (bla) reporter assay. The β-lactamase (bla) reporter assay measures the strength of canonical Wnt/β-catenin signaling by its ability to control β-catenin/LEF/TCF response elements and can therefore be used to assess whether a test agent can attenuate or increase the strength of control of canonical Wnt/β-catenin signaling on its transcriptional target.

The ability of the compounds of the present invention to inhibit canonical Wnt/β-catenin signal transduction can

also be assessed by measuring gene expression and/or protein expression of target genes that are transcriptionally controlled by canonical Wnt/β-catenin signal transduction. The expression of target genes can be assessed in transcriptional cells contacted with the compounds of the present invention or in subjects administered with these compounds. Target genes include, for example, CMYC, CCND1, CD44, LGR5, VEGFA, AXIN2, and LEF1. The expression levels of one or more target genes associated with canonical Wnt/β-catenin signal transduction can be analyzed using methods known in the art, such as cell staining, flow cytometry, immunoblotting, and/or real-time quantitative PCR(rt-qPCR) analysis.

**Regulatory T cell survival**

It is known that various markers are expressed on regulatory T cells, such as CD4, FOXP3 and CD25. The ability of the compounds of the present invention to reduce the survival of regulatory T cells can be assessed by counting the total number of regulatory T cells present in blood and/or specific tissues (such as tumors). For example, a sample obtained from an object contacted with the compounds of the present invention can be stained with an antibody that detects a marker associated with regulatory T cells. The sample can also be processed and labeled with an antibody that detects such a marker and analyzed by flow cytometry. The gene and/or protein expression of such a marker can be measured in a sample and analyzed by, for example, immunoblotting and/or rt-qPCR.

**VEGF expression in tumors**

Various assays can be used to measure VEGF gene expression and/or protein expression in a tumor sample. For example, after contacting a subject with a compound, tumor cells can be collected and stained with an anti-VEGF antibody to detect VEGF protein. Cells can also be analyzed, for example, by RT-qPCRto determine VEGF gene expression. Other assays that indicate changes in VEGF expression can be used. For example, tumor samples from subjects contacted with a compound of the invention can be analyzed to detect various angiogenic markers controlled by VEGF. In some embodiments, a compound of the invention reduces VEGF expression more effectively than a vehicle or control.

**CD4+ and/or CD8+ T cells infiltrate into the tumor**

The infiltration of CD4+T cells and/or CD8+T cells into a tumor can be assessed by counting the total number of CD4+T cells and/or CD8+T cells present in a tumor or a sample (e.g., a biopsy) from a tumor. Various markers, such as CD4 and CD45, are known to be expressed on CD4+T cells (also referred to as helper T cells). Various markers, such as CD8 and CD45, are known to be expressed on CD8+T cells (also referred to as cytotoxic T cells). The ability of a compound to increase the infiltration of CD4+ and/or CD8+T cells into a tumor can be assessed in vivo by administering a compound to a subject with a tumor. Tumor samples can be collected from a subject and stained with antibodies that detect markers associated with CD4+/CD8+T cells. The sample can also be processed and labeled with, for example, an antibody that detects such markers, and analyzed, for example, by flow cytometry. The gene and/or protein expression of such markers can also be determined in a sample and analyzed, for example, by immunoblotting and/or rt-qPCR.

**T helper 17 cells infiltrate the tumor**

In some embodiments, when administered to a subject bearing a tumor, the compounds of the present invention are capable of increasing the infiltration of T helper 17 cells into the tumor. The infiltration of T helper 17 cells into the tumor can be assessed by counting the total number of T helper 17 cells present in the tumor. Various markers are known to be expressed on T helper 17 cells, for example, IL-17. The ability of a compound to increase the infiltration of T helper 17 cells into a tumor can be assessed in vivo by administering the compound to a subject with a tumor. Tumor samples can be collected from the subject and stained with, for example, antibodies that detect markers associated with T helper 17 cells. The sample can also be processed and labeled with antibodies that detect such markers and analyzed by flow cytometry. The gene and/or protein expression of such markers can also be measured in the sample and analyzed by, for example, immunoblotting and/or rt-qPCR. The sample can be analyzed to detect the amount of IL-17 present in the sample.

**Dendritic cells in tumors**

In some embodiments, when administered to a subject carrying a tumor, the compounds of the present invention are capable of transducing dendritic cells present in the tumor. The number of dendritic cells present in the tumor can be assessed, for example, by staining the tumor with antibodies that recognize one or more markers associated with dendritic cells. Various markers are known to be expressed on dendritic cells, such as CD11c. The ability of a compound to reduce dendritic cells in a tumor can be assessed in vivo by administering the compound to the subject. Tumor samples can be

collected from the subject and stained with antibodies that detect markers associated with dendritic cells. The sample can also be processed and labeled, for example, with antibodies that detect such markers, and analyzed, for example, by flow cytometry. The gene and/or protein expression of such markers can be analyzed, for example, by immunoblotting and/or RT-qPCR.

**biomarkers**

The present disclosure also encompasses methods of measuring at least one biomarker for monitoring the therapeutic efficacy of a compound or pharmaceutical composition of the invention or for selecting a subject for treatment with such a compound or pharmaceutical composition. In some embodiments, the biomarker is one or more of BCL9, CD44, Axin2, cMyc, LGR5, VEGFA, Sox2, Oct4, Nanog, and/or active β-catenin. As used herein, active β-catenin refers to the non-phosphorylated form of β-catenin.

Various known methods can be used to measure the gene expression level and/or protein level of such biomarkers. For example, a sample from an object treated with a compound or pharmaceutical composition, such as a biopsy of a tumor, blood, plasma, serum, urine, amniotic fluid, synovial fluid, endothelial cells, leukocytes, monocytes, other cells, organs, tissues, bone marrow, lymph nodes or spleen can be obtained. In some embodiments, the sample is a tumor biopsy in the object. The sample obtained from the object can be stained with one or more antibodies or other detection reagents that detect such biomarkers. The sample can also or alternatively be processed to detect the presence of nucleic acids (such as mRNA) encoding biomarkers by, for example, rt-qPCRmethods.

In some embodiments, reduced gene expression levels and/or protein levels of BCL9, CD44, Axin2, cMyc, LGR5, VEGFA, Sox2, Oct4, Nanog and/or active β-catenin indicate the therapeutic efficacy of the compounds or pharmaceutical compositions described herein. The expression levels of such biomarkers can be measured, for example, after 1 day, 2 days, 3 days, 4 days, 5 days, one week or two weeks of administration of the compound or pharmaceutical composition, or any time period therebetween. In some embodiments, a method is disclosed that includes measuring the level of one or more biomarkers after one or more rounds of use of the compounds or pharmaceutical compositions of the present invention. In some embodiments, the method further includes continuing to administer the compound or pharmaceutical composition if the biomarker level decreases. In some embodiments, the method further includes administering an increased dose of the compound or pharmaceutical composition of the present invention if the biomarker level does not decrease, or increasing the frequency of subsequent administration. In some embodiments, if the biomarker level does not decrease after the initial administration, treatment is stopped. In various embodiments, marker levels are also measured prior to the first use of a compound or pharmaceutical composition of the invention and compared to levels after one or more rounds of administration, wherein treatment efficacy and continued treatment steps are determined based on the change in one biomarker level from one or more levels prior to administration.

In some embodiments, increased gene expression levels and/or protein levels of BCL9, CD44, Axin2, cMyc, LGR5, VEGFA, Sox2, Oct4, Nanog, and/or active β-catenin indicate that the subject will benefit from treatment with a compound or pharmaceutical composition of the invention, compared to a subject without increased gene expression levels and/or protein levels. In some embodiments, a method of treatment is disclosed comprising selecting a patient with increased biomarker levels and administering a compound or pharmaceutical composition of the invention.

In certain embodiments, subjects with elevated gene and/or protein expression levels of BCL9, CD44, Axin2, cMyc, LGR5, VEGFA, Sox2, Oct4, Nanog, and/or active beta-catenin are selected for treatment with a compound or pharmaceutical composition of the present invention. In some embodiments, after obtaining a tumor sample from a subject and identifying elevated gene and/or protein expression of BCL9, CD44, Axin2, cMyc, LGR5, VEGFA, Sox2, Oct4, Nanog, and/or active beta-catenin, the subject with the tumor is selected for treatment. In some embodiments, after obtaining a tumor sample from a subject and identifying elevated gene and/or protein expression of BCL9, the subject with the tumor is selected for treatment. In some embodiments, after obtaining a tumor sample from a subject and identifying elevated gene and/or protein expression of CD44, the subject with the tumor is selected for treatment. In some embodiments, after obtaining a tumor sample from a subject and identifying elevated gene and/or protein expression of active beta-catenin, the subject with the tumor is selected for treatment.

**Half-life in receptors**

In some embodiments, the compounds of this invention have one or more improved pharmacokinetic parameters compared to a vehicle or a control. Such pharmacokinetic parameters can include, for example, maximum observed concentration (Cmax), time to maximum concentration (Tmax), terminal half-life (T1/2), systemic clearance (CL), volume of distribution (Vz), area under the curve from the time of administration to the last measurable concentration (AUC0-t), area under the curve extrapolated from the time of administration to infinity (AUC0-inf) and bioavailability.

Methods for evaluating the pharmacokinetics of pharmaceutical agents are known in the art. For example, blood samples from subjects administered with a compound described herein can be obtained at 5 minutes, 1, 2, 4, 6, 8, 12, and

24 hours after administration. The concentration of the compound in the blood sample can be analyzed by various analytical tools, such as LC/MS. Pharmacokinetic parameters are calculated based on the compound concentration at each time point. As used herein, the term "maximum observed concentration (Cmax)" refers to the maximum serum concentration reached by the compound after administration. Related to the concept of Cmax , the time to maximum concentration (Tmax)is the time for the compound to reach maximum serum concentration. The terms "terminal half-life ($T1_{/2}$ )" and "half-life ($T1_{/2}$ )" are used interchangeably and refer to the time it takes for a compound to lose half its serum concentration. Systemic clearance (CL) represents the amount of blood that is completely cleared of the compound per unit time. The term "distribution volume (Vz)" refers to the theoretically calculated volume that would need to contain the total amount of compound administered to the subject at the same concentration observed in the blood. The term "bioavailability" refers to the extent and rate at which a drug is absorbed into a biological system, or becomes available at physiologically active sites. Bioavailability can be a function of several of the previously described properties, including stability, solubility, immunogenicity, and pharmacokinetics, and can be assessed using methods known to those skilled in the art.

The pharmacokinetic parameters of the compound can be assessed in mammals (including, for example, mice, rats, or humans). Various routes of administration, such as intravenous, intraperitoneal, subcutaneous, and intramuscular routes of administration can also be used to assess parameters. In some embodiments, the pharmacokinetic parameters of the compounds of the invention are assessed in mice. In some embodiments, the pharmacokinetic parameters of the compounds described herein are assessed in mice to which the compound is administered subcutaneously. In some embodiments, the pharmacokinetic parameters of the compounds of the invention are assessed in humans. In some embodiments, the pharmacokinetic parameters of the compounds of the invention are assessed in humans after subcutaneous administration.

**Tumor microenvironment conducive to immune response**

In various embodiments, the compounds of the invention induce a tumor microenvironment that is more favorable for an immune response. In various embodiments, the compounds of the invention induce a tumor microenvironment that is more favorable for an immune response than vehicle or a control.

Various parameters can be used to assess the tumor microenvironment. For example, an increased ratio of cytotoxic T cells to regulatory T cells in and/or around tumor tissue can indicate that the tumor microenvironment is conducive to an immune response. A decrease in the number of dendritic cells and/or regulatory T cells in and/or around tumor tissue can also indicate that the tumor microenvironment is conducive to an immune response. Other parameters include an increase in circulating T cells in peripheral blood and an increase in the ratio of T helper 17 cells to regulatory T cells in and/or around tumor tissue. These parameters can indicate that the tumor microenvironment is conducive to an immune response.

In some embodiments, the compounds of the invention can increase the ratio of the amount of cytotoxic T cells to the amount of regulatory T cells in the tumor microenvironment. In some embodiments, the change in ratio caused by the compound is greater than the change in ratio caused by the vehicle or control.

**Tumor growth, cancer stem cell proliferation, and/or tumor metastasis**

Because Wnt signaling is a regulator of tumor growth, the therapeutic efficacy of compounds that affect BCL9 binding to β-catenin can be evaluated in animal models.

For example, BALB/c nude mice can be used to assess the in vivo effectiveness of the present invention in a human cancer model, because xenografts of human cancer cells will grow into tumors in these mice. For example, subcutaneous inoculation of Colo320DM tumor cells, a commercially available cell line derived from human colon cancer tissue, can be used to form tumors in BALB/c nude mice. Other in vivo models can also be utilized to assess the in vivo effectiveness of compounds disclosed herein. For example, human DLD-1 colon cancer cells can be implanted in nude mice to assess tumor growth. The CT26 isogenic mouse model of colon cancer can also be used, because the model allows assessment of tumor growth under the background of a complete immune system. Other types of cancer cells, for example, B16 melanoma, 4T1 breast cancer, human kidney cancer and Lewis lung cancer cells can also be used in these known animal models, to assess the in vivo effectiveness of compounds disclosed herein.

By administering a compound of the invention to one or more animal models, the effect of the compound in reducing tumor growth in vivo can be assessed. Based on animal data treated with stabilized BCL9 peptides, the ability of the peptide to inhibit Wnt signaling can be assessed, for example, by staining tissue samples with markers of Wnt signaling. These downstream markers of Wnt signaling include, for example, Axin2 and CD44.

Orthotopic mouse models can be used to evaluate the effects of the compounds described herein on tumor metastasis. For example, orthotropic animal models can be injected with cells carrying a luciferase construct and then administered with their designated treatment. The presence of injected cells can be detected by administering a luciferin

substrate to each treated animal. The intensity of the bioluminescent signal can be quantitatively measured and used as an indicator of cell growth.

In some embodiments, the effects of the compounds of the present invention on cancer stem cell proliferation can be assessed by measuring various cancer stem cell biomarkers. For example, the expression levels of CD44 and/or LGR5 can indicate the amount of cancer stem cells present in a sample. Tumor samples can be collected from a subject and stained with antibodies that detect markers associated with cancer stem cells. The samples can also be processed and labeled, for example, with antibodies that detect such markers, and analyzed, for example, by flow cytometry. Gene and/or protein expression of such markers can be detected and analyzed, for example, by immunoblotting and/or RT-qPCR.

## Diseases with abnormal Wnt/β-catenin signaling

Aberrant Wnt/β-catenin signaling is associated with the malignant transformation of normal cells into cancer cells. Activation of Wnt signaling and β-catenin nuclear localization is associated with tumor phenotypes in various models.

The present disclosure encompasses compositions for use and methods of using the compounds disclosed herein to inhibit the binding of BCL9 to β-catenin in a subject by administering the compounds or pharmaceutical compositions comprising the compounds to the subject. The present disclosure also encompasses inhibiting canonical Wnt/β-catenin signaling in a subject by administering the compounds or pharmaceutical compositions disclosed herein. The present disclosure further encompasses methods of treating a disease in a subject by administering the compounds or pharmaceutical compositions of the present invention to the subject. The disease may be a cancer or other neoplastic disease associated with aberrant canonical Wnt/β-catenin signaling.

In some embodiments, the disease, disorder, or condition can be a disease that can benefit from inhibition of canonical Wnt/β-catenin signaling. In some embodiments, such a disease, disorder, or condition is cancer. In some embodiments, the cancer is a cancer in which BCL9 and/or β-catenin are highly expressed. In some embodiments, the cancer is a cancer in which BCL9 and β-catenin are co-localized in the nucleus of cancer cells. In some embodiments, the cancer is selected from the group consisting of familial adenomatous polyposis (FAP), eye cancer, rectal cancer, colon cancer, colorectal cancer, cervical cancer, prostate cancer, breast cancer, bladder cancer, oral cancer, benign and malignant tumors, stomach cancer, liver cancer, pancreatic cancer, lung cancer, uterine corpus, ovarian cancer, prostate cancer, testicular cancer, kidney cancer, brain/CNS cancer, laryngeal cancer, multiple myeloma, cutaneous melanoma, acute lymphoblastic leukemia, acute myeloid leukemia, Ewing's sarcoma, Kaposi's sarcoma, basal cell carcinoma and squamous cell carcinoma, small cell lung cancer, choriocarcinoma, rhabdomyosarcoma, angiosarcoma, hemangioendothelioma, Wilms' tumor, neuroblastoma, oral/pharyngeal cancer, esophageal cancer, laryngeal cancer, lymphoma, neurofibromatosis, tuberous sclerosis, hemangioma, gastric cancer, ovarian cancer, hepatocellular carcinoma, and lymphangiogenesis. In some embodiments, the cancer is colorectal cancer. In some embodiments, the cancer is gastric cancer. In some embodiments, the cancer is ovarian cancer. In some embodiments, the cancer is hepatocellular carcinoma. In some embodiments, the cancer is breast cancer. In some embodiments, the cancer is prostate cancer. In some embodiments, the cancer is skin melanoma. In some embodiments, the cancer is lung cancer.

In some embodiments, any of the compounds or variants disclosed herein, or pharmaceutical compositions comprising such compounds, can be used to treat diseases, such as the cancers listed above.

Treatment and measured therapeutic parameters can be assessed after administration of the compound or pharmaceutical composition alone or in combination with one or more additional therapeutic agents (e.g., as a single bolus or separate sequential administrations). The additional agent can be any additional therapeutic agent mentioned herein or known to those skilled in the art. Depending on the selected regimen, the compound or pharmaceutical composition comprising the compound and/or the additional agent can be administered once or multiple times.

The present invention also encompasses compounds or pharmaceutical compositions disclosed herein for use in treating a disease in a subject. In some embodiments, the disease can benefit from inhibition of canonical Wnt/β-catenin signaling. In some embodiments, the disease is cancer.

The present disclosure further encompasses the use of a compound or pharmaceutical composition disclosed herein in the manufacture of a medicament for treating a disease in a subject. In some embodiments, the disease can benefit from inhibition of canonical Wnt/β-catenin signaling. In some embodiments, the disease is cancer.

In another embodiment, the disease treated is a disease other than cancer. In certain embodiments, the disease is bone density deficiency, ocular vascular defects, familial exudative vitreoretinopathy, early coronary artery disease, Alzheimer's disease, autosomal dominant oligodontia, retinal angiogenesis, osteogenesis imperfecta, Tetra-Amelia syndrome, Mullerian-duct regression and virilization, SERKAL syndrome, type II diabetes, Fuhrmann syndrome, onychodermal dysplasia, obesity, cleft limbs, tail duplication, tooth agenesis, skeletal dysplasia, focal dermal dysplasia, autosomal recessive scleroderma, neural tube defects or sclerosteosis, and Van Buchem disease.

## Combination therapy

In certain embodiments, the compounds disclosed herein or pharmaceutical compositions are administered together with at least one additional agent. That is, the compounds disclosed herein and additional agents can be administered to the patient continuously or simultaneously in a separate dosage form as described herein. In some embodiments, the at least one additional agent is selected from checkpoint inhibitors, EGFRinhibitors, VEGF inhibitors, VEGFRinhibitors, anticancer drugs (e.g., any therapeutic agent in additional therapeutic agents described herein) stapled peptides and additional agents can be administered in a therapeutically effective amount.

In certain embodiments, a subject administered a compound or pharmaceutical composition disclosed herein is also treated with radiation therapy and/or chemotherapy prior to, after, or concurrently with administration of the compound or pharmaceutical composition.

## Kits

The present invention also includes pharmaceutical kits useful, for example, for treating the disorders, diseases, and conditions described herein, comprising one or more containers containing a pharmaceutical composition comprising a therapeutically effective amount of a compound of the present invention. If desired, such kits may also include various conventional pharmaceutical kit components, for example, one or more containers with one or more pharmaceutically acceptable carriers, additional containers, etc. The kit may also include instructions, either as an insert or as a label, indicating the amounts of the components to be administered, instructions for administration, and/or instructions for mixing the components.

Also disclosed herein are kits for performing the methods described herein. In various embodiments, kits for making the compounds of the present invention are provided. In some embodiments, the kits include compounds capable of undergoing a reaction for forming one or more hydrocarbon linkers. In some embodiments, the kits include a metal catalyst for performing metal-mediated ring-closing metathesis.

In some embodiments, the kit includes an agent for detecting gene and/or protein expression of BCL9, CD44, Axin2, cMyc, LGR5, VEGFA, Sox2, Oct4, Nanog, and/or active β-catenin.

The present invention was further described hereafter in combination with specific embodiments. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are percentages by weight and parts by weight.

### Preparation Example

### General Synthesis Method

The compounds of the present invention may be prepared, isolated or obtained by any method that is obvious to a person skilled in the art. The compounds of the present invention can also be prepared according to the exemplary preparation schemes provided below (such as the methods in the examples). Reaction conditions, steps and reactants not provided in the exemplary preparation schemes are obvious and known to a person skilled in the art. As used herein, the symbols and conventions used in these processes, schemes and examples, whether or not a particular abbreviation is specifically defined, have meanings that are well known to a person skilled in the art.

Specifically, but not limited to, the following abbreviations may be used in the examples and throughout the specification: rt (room temperature); g (gram); mg (milligram); mL (milliliter); μL (microliter); mm (millimole) μ; M (micromole); MHz (hertz); MHz (megahertz); mmol (millimole); hr (hour); min (minute); MS) (mass spectrometry); ESI (electrospray ionization); TLC (thin layer chromatography); HPLC (high performance liquid chromatography); BOC (tert-butyloxycarbonyl ); *tBu* (tert-butyl); HATU (2-(7-azabenzotriazole)-N,N,N',N'-tetramethyluronium hexafluorophosphate); TFA (trifluoroacetic acid); Pd2 $_($ dba) $_3$ (tris(dibenzylideneacetone)dipalladium); DIPEA (N,N-diisopropylethylamine).

### Preparation Example 1: Synthesis of Compounds 115-010, 115-011, 115-012, 115-013, 115-014, 115-015, 115-022, and 115-023

### (1) Synthesis of compound 115-010

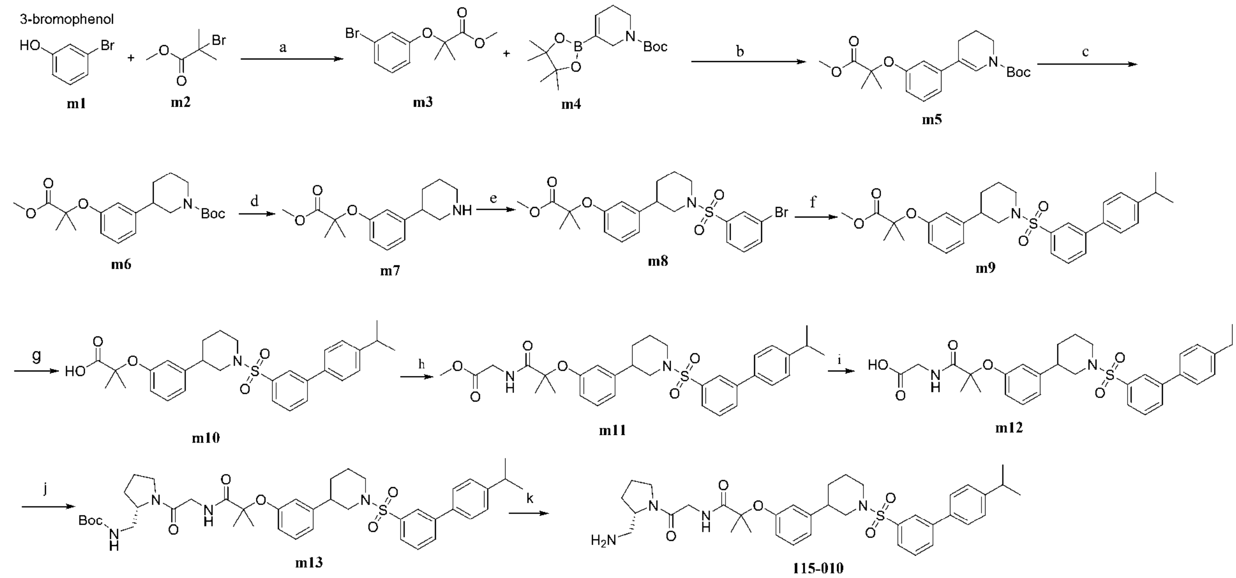

m1 m2 m3 m4 m5 m6 m7 m8 m9 m10 m11 m12 m13 115-010

**Reagents** and **conditions:** (a) $K_2CO_3$, NaH, MeCN, reflux, overnight; (b) $Pd(dppf)Cl_2$, $CH_3COOK$, KF, THF/$H_2O$(5:1), 75 °C , 16h; (c) $H_2$, 10% Pt/C, THF, 2 psi, 50 °C , 48 h; (d) TFA/DCM(1:3), RT, 6h; (e) 5-bromo-2-chlorobenzenesulfonyl chloride, TEA, dry DCM, RT, 24 h; (f) 4-isopropylphenylboronic acid pinacol ester, $Pd(PPh_3)_4$, THF/2 M $K_2CO_3$ (5:1), 75 °C , 24 h; (g) LiOH ·$H_2O$, THF/MeOH/$H_2O$ (3:1:1), RT, overnight. (h) Glycine methyl ester hydrochloride, HATU, DIPEA, THF, RT, 6 h. (i) LiOH ·$H_2O$, THF/MeOH/$H_2O$ (3:1:1), rt, overnight. (j) tert-Butyl (R)-(pyrrolidin-2-ylmethyl)carbamate, HATU, DIPEA, THF, rt, 6 h. (k) TFA/DCM (1:3), RT, 6 h.

**Specific steps:**

Synthesis of intermediate m3: Raw materials m1 (2.00 g, 1 eq), m2 (6.28 g, 3 eq), $K_2CO_3$ ( 6.30 g, 4 eq), and catalyst NaH (0.04 g, 0.1 eq) were added to MeCN (60 mL). The mixture was heated to 85°C and refluxed with stirring overnight. The reaction was monitored by TLC until completion, and the filtrate was concentrated by evaporation under reduced pressure. The resulting residue was extracted with water and ethyl acetate, and the organic layer was washed with saturated aqueous NaCl solution, dried over anhydrous $Na_2SO_4$ , and evaporated to dryness to obtain the crude product. The crude product was purified by silica gel column chromatography (petroleum ether/ethyl acetate = 30:1) to obtain intermediate m3 as a colorless oil (2.83 g, yield 89.56%).

Synthesis of intermediate m5: Raw materials m3 (0.94 g, 1 eq), m4 (0.78 g, 1.1 eq), potassium acetate (1.35 g, 4 eq), potassium fluoride (0.60 g, 3 eq), and catalyst $Pd(dppf)Cl_2$ (0.24 g, 0.1 eq) were added to THF/$H_2O$ ( 48 mL). The mixture was heated to 75 ° C and stirred under nitrogen for 16 h. The reaction was monitored by TLC until completion. The filtrate was concentrated by evaporation under reduced pressure and evaporated to dryness to obtain the crude product. The crude product was purified by silica gel column chromatography (petroleum ether/ethyl acetate = 6:1) to obtain intermediate m5 as a light yellow oil (0.88 g, 90.7% yield).

Synthesis of intermediate m6: 20% Pt/C (6 g) and m5 (0.88 g, 1 eq) were added to a THF (15 mL) solution and heated to 50 ° C with stirring under 2 psi of hydrogen (hydrogen balloon) for 48 hours. After the reaction reached 80% completion as monitored by HPLC, insoluble material was filtered off, and the filtrate was concentrated by evaporation under reduced pressure. Intermediate m6 (crude) was obtained as a pale yellow oil (1.04 g), with an approximate 75% yield (0.78 g).

Synthesis of intermediate m7: m6 (crude) was added to a 15 mL DCM solution, followed by the dropwise addition of 5 mL of TFA. The reaction was stirred at room temperature for 6 h. TLC was monitored until completion of the reaction. The filtrate was concentrated by evaporation under reduced pressure to dryness to obtain 1.47 g of crude product m7 as a pale yellow oil with an approximate 40% yield.

Synthesis of Intermediate m8: Intermediate m7 (0.57 g, 1 eq), 5-bromo-2-chlorobenzenesulfonyl chloride (0.65 g, 1.1 eq), TEA (0.62 g, 3 eq), and anhydrous DCM solution (15 ml) were added to a 50 ml round-bottom flask and stirred at room temperature under nitrogen. After TLC monitoring until completion of the reaction, the filtrate was concentrated by evaporation under reduced pressure, diluted with water, and extracted with ethyl acetate. The organic layer was dried over anhydrous $Na_2SO_4$ , and the filtrate was concentrated by evaporation under reduced pressure to obtain the crude product. The crude product was purified by silica gel column chromatography (petroleum ether/ethyl acetate = 5:1) to obtain intermediate m8 as a colorless oil (0.77 g, yield 70.64%).

Synthesis of intermediate m9: m8 (0.30 g, 1 eq), 4-isopropylphenylboronic acid pinacol ester (0.15 g, 1.1 eq), and $Pd(PPh_3)_4$ (216.09 mg, 0.19 mmol) were added to a solution of THF/2M $K_2CO_3$ (5:1) (15:3 mL) at 75 ° C . The reaction was stirred under nitrogen for 12 h. TLC monitoring was performed until the reaction was complete. The filtrate was concentrated by evaporation under reduced pressure, and the residue was extracted with water and ethyl acetate. The organic layer was washed with saturated NaCl solution and dried over anhydrous $Na_2SO_4$. The filtrate was concentrated by evaporation under reduced pressure to obtain the crude product. The crude product was purified by silica gel column chromatography (petroleum ether/ethyl acetate = 10:1) to obtain intermediate m9 as a colorless oil, 0.23 g, in a yield of 74.19%.

Synthesis of intermediate m10: m9 (0.23 g, 1 eq) and $LiOH \cdot H_2O$ ( 69 mg, 4 eq) were added to a solution of THF/MeOH/$H_2O$ ( 3:1:1) (12:4:4 mL) and stirred overnight at room temperature. After completion of the reaction, the mixture was concentrated under reduced pressure. The resulting residue was dissolved in ethyl acetate, adjusted to pH 2 with 1M HCl, and extracted with ethyl acetate. The organic layer was dried over anhydrous $Na_2SO_4$ and the reaction mixture was concentrated by evaporation under reduced pressure to afford crude product m10 as a white solid (0.26 g), which was used directly in the next step.

Synthesis of intermediate m11: To a 100 ml dry reaction flask, intermediate m10 (0.26 g, 0.57 mmol) was added and dissolved in 40 ml of THF. Glycine methyl ester hydrochloride (0.12 g, 0.93 mmol), DIPEA (0.40 g, 3.09 mmol), and HATU (0.32 g, 0.85 mmol) were then added and stirred at room temperature for 6 h. The reaction was monitored by TLC (petroleum ether:ethyl acetate = 1:1). 30 ml of water was added to the reaction solution, and the mixture was extracted with DCM (30 ml x 3). The organic phase was collected, washed with 40 ml of saturated brine, and dried over anhydrous $Na_2SO_4$. The residue was purified by silica gel column chromatography (eluent: petroleum ether:ethyl acetate = 1:1) to afford product m11 (0.37 g, 81% yield) as a pale yellow viscous solid.

Synthesis of intermediate m12: Compound m11 (0.37 g, 0.63 mmol) was added to a 50 mL dry reaction flask and dissolved in 20 mL of THF. Lithium hydroxide (0.12 g, 2.52 mmol) was dissolved in 5 mL of water, followed by 5 mL of methanol, which was then added to the reaction mixture and stirred at room temperature for 4 h. The reaction was monitored by TLC (petroleum ether:ethyl acetate = 1: 1). The reaction mixture was concentrated, and the residue was added with 20 mL of water. The pH was adjusted to approximately 4-5 with 1 M HCl, and then extracted with ethyl acetate (30 mL x 3). The organic phases were combined, washed with 40 mL of saturated brine, and dried three times over anhydrous $Na_2SO_4$. The organic phase was concentrated to afford compound m12 (0.33 g, 99% yield) as a pale yellow solid.

Synthesis of Intermediate m13: To a 100-ml dry reaction flask, intermediate m12 (0.32 g, 0.57 mmol) was added and dissolved in 15 ml of THF. Tert-butyl (R)-(pyrrolidin-2-ylmethyl)carbamate (0.14 g, 0.69 mmol), DIPEA (0.30 g, 2.30 mmol), and HATU (0.24 g, 0.63 mmol) were then added and stirred at room temperature for 6 h. The reaction was monitored by TLC (ethyl acetate). The reaction solution was concentrated, and 20 ml of water was added to the residue. The mixture was extracted with DCM (30 ml x 3). The organic phases were combined, washed with 40 ml of saturated brine, and dried over anhydrous $Na_2SO_4$. The residue was purified by silica gel column chromatography (ethyl acetate as the eluent) to afford product m13 (0.32 g, 74% yield) as a pale yellow solid.

Synthesis of 115-010: m13 (100 mg) was added to a DCM (15 mL) solution, followed by the dropwise addition of 5 mL of TFA. The reaction mixture was stirred at room temperature for 6 h. TLC was monitored until completion of the reaction. The filtrate was concentrated by evaporation under reduced pressure and evaporated to dryness to obtain the crude product, which was then extracted with water and DCM. The organic layer was washed with a saturated aqueous NaCl solution and dried over anhydrous Na2SO4 solid. The reaction mixture was concentrated by evaporation under reduced pressure to obtain the crude product. The crude product was purified by reverse phase preparative purification (water/acetonitrile/trifluoroacetic acid) to obtain the product 115-010. The product was frozen at -80 ° C for 6 h and then freeze-dried to obtain the final product, 115-010, as a white solid (46 mg, yield 54.14%); HPLC analysis >95%.

**(2)** Compounds 115-011, 115-012, 115-013, 115-014, 115-015, 115-022, and 115-023 were prepared by a method similar to that of compound 115-010.

**Preparation Example 2: Synthesis of Compounds 115-016, 115-017, 115-018,** and **115-019**

**(1) Synthesis of compound 115-016**

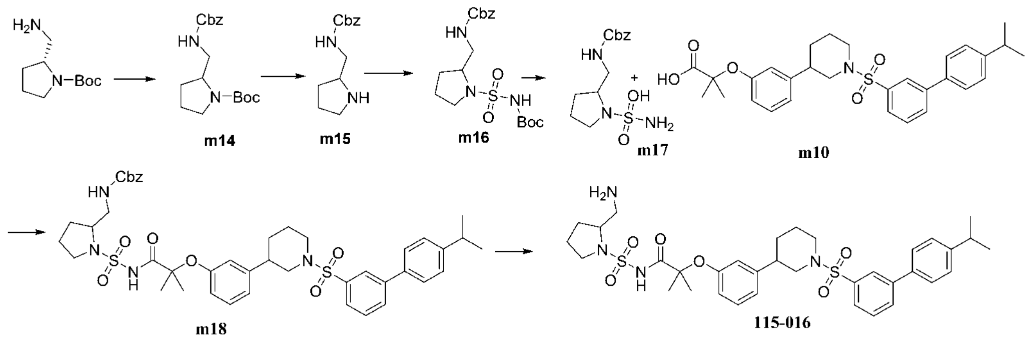

m14 m15 m16 m17 m10

m18 115-016

**Reagents** and **conditions:** (a) Benzyl chloroformate, DIPEA, DCM, RT, 3h; (b) TFA/DCM (1:3), RT, 6h; (c) N-Boc yellow yl chloride, TEA, THF, RT, 3h; (d) M10, EDCI, DMAP, DCM, RT, overnight; (e) Hydrogen, 5% palladium hydroxide on carbon, THF, 50 ° C , 16h.

**Specific steps:**

Synthesis of Intermediate m14: To a dry reaction flask, tert-butyl (R)-2-(aminomethyl)pyrrolidine-1-carboxylate (0.5g, 1eq), DIPEA (1.29g, 4eq), and DCM were added. The mixture was stirred at room temperature for 10 minutes, followed by the dropwise addition of benzyl chloroformate (0.47g, 1.1eq). The reaction was stirred at room temperature for 3 hours. TLC was monitored until completion of the reaction. Water was added, and the mixture was extracted with DCM. The organic layer was washed with saturated aqueous NaCl solution and dried over anhydrous Na2SO4. The reaction solution was concentrated by evaporation under reduced pressure to obtain the crude product. Intermediate m14 (0.71g) was eluted on a silica gel column with a 10:1 ratio of petroleum ether to ethyl acetate, yielding 85.5%.

Synthesis of intermediate m15: To a dry reaction flask, m14 (0.71 g, 1 eq), 15 ml of DCM, and 5 ml of TFA were added. The mixture was stirred at room temperature for 6 h. After TLC monitoring, the reaction was completed. Water was then added and the mixture was extracted with DCM. The organic layer was washed with saturated aqueous NaCl and dried over solid anhydrous NaSO. The reaction mixture was concentrated by evaporation under reduced pressure to afford crude product m15 (0.67 g) in a 100% yield.

Synthesis of Intermediate m16: To a dry reaction flask, m15 (0.67 g, 1 eq) and TEA (0.84 g, 4 eq) were added and dissolved in THF. The mixture was stirred at room temperature for 30 minutes, followed by the dropwise addition of N-Boc yellow yl chloride (1.2 eq). The reaction was stirred at room temperature for 6 hours. After TLC monitoring, the reaction solution was concentrated by evaporation under reduced pressure, water was added, and extraction was performed with DCM. The organic layer was washed with saturated aqueous NaCl and dried over anhydrous $Na_2SO_4$. $_{The}$ reaction solution was concentrated by evaporation under reduced pressure to obtain the crude product, intermediate m16 (0.31 g), which was eluted on a silica gel column with a 5:1 ratio of petroleum ether to ethyl acetate. The yield was 31%.

Synthesis of intermediate m17: To a dry reaction flask, m15 (0.31 g, 1 eq), 15 ml of DCM, and 5 ml of TFA were added. The mixture was stirred at room temperature for 6 h. After TLC monitoring, the reaction was completed. Water was then added and the mixture was extracted with DCM. The organic layer was washed with saturated aqueous NaCl and dried over solid anhydrous Na2SO4. The reaction mixture was concentrated by evaporation under reduced pressure to give crude product m15 (0.38 g) in a 100% yield.

Synthesis of intermediate m18: To a dry reaction flask, m17 (0.14 g, 1.1 eq), m10 (0.21 g, 1 eq), EDCI (2 eq), DMAP (2 eq), and DCM were added. The mixture was stirred at room temperature for 6 h. After TLC monitoring, the reaction was completed with water and extracted with DCM. The organic layer was washed with saturated aqueous NaCl and dried over anhydrous $Na_2SO_4$. $_{The}$ reaction mixture was concentrated by evaporation under reduced pressure to obtain the crude product. Intermediate m17 (0.23 g) was eluted on a silica gel column with a 3:1 ratio of petroleum ether to ethyl acetate, yielding 69%.

Synthesis of Intermediate 115-016: To a dry reaction flask, m18 (0.1 g, 1 eq), 5% palladium hydroxide on carbon (20% W, 0.02 g), THF, and hydrogen (2-3 psi) were added. The mixture was stirred at 50 ° C for 16 h. After completion of the reaction, water was added and the reaction solution was concentrated by evaporation under reduced pressure to obtain a crude product, which was extracted with DCM. The organic layer was washed with saturated aqueous NaCl and dried over anhydrous Na2SO4 solid. The reaction solution was concentrated by evaporation under reduced pressure to obtain crude product m15 (0.67 g). The crude product was purified by reverse phase preparative purification (water/acetonitrile/trifluoroacetic acid) to obtain a solution of product 115-016. The solution was frozen at -80 ° C for 6 h and then freeze-dried in a lyophilizer to obtain the final product 115-016 as a white solid (21 mg, yield 23.14%) (HPLC >95%).

(2) Compounds 115-017, 115-018, and 115-019 were prepared by a method similar to that of compound 115-016.

**Preparation Example 3: Synthesis of Compounds 115-020** and **115-021**

**(1) Synthesis of compound 115-020**

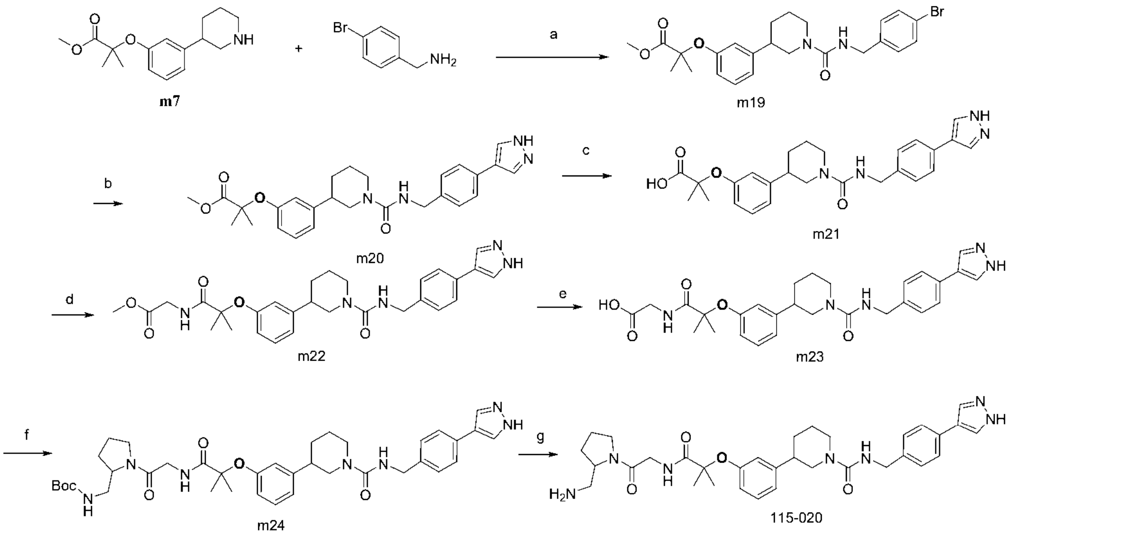

m7
m19
m20
m21
m22
m23
m24
115-020

**Reagents** and **conditions:** (a) CDI, DIPEA, DCM, RT, overnight; (b) Pd(dppf)Cl $_2$ , CH $_3$ COOK, KF, THF/H $_2$ O (5:1), 75 °C , 16h; (c) LiOH ·H $_2$ O, THF/MeOH/H $_2$ O (3:1:1), rt, overnight. (d) Glycine methyl ester hydrochloride, HATU, DIPEA, THF, RT, 6h (e) LiOH ·H $_2$ O, THF/MeOH/H $_2$ O (3:1:1), rt, overnight. (f) tert-Butyl-2-pyrrolidin-1-ylmethylcarbamate, HATU, DIPEA, THF, rt, 6h. (g) TFA/DCM (1:3), RT, 6h.

**Specific steps:**

Synthesis of Intermediate m19: 4-Bromobenzylamine (0.41 g, 1 eq) was added to a 100 ml dry reaction flask and dissolved in 20 ml of anhydrous DCM. DIPEA (1.14 g, 4 eq) was then added and stirred at room temperature for 1 hour. Intermediate m7 (0.61 g, 1 eq) dissolved in 10 ml of DCM was then added dropwise to the reaction mixture. Stirring was continued overnight at room temperature and the reaction was monitored by TLC (petroleum ether:ethyl acetate = 5:1). 30 ml of water was added to the reaction mixture, and extraction with DCM (30 ml x 3) was performed. The organic phase was collected, washed with 40 ml of saturated brine, and dried over anhydrous $Na_2SO_4$ . The residue was purified by silica gel column chromatography (eluent: petroleum ether:ethyl acetate = 5:1) to yield Intermediate m19 (0.66 g, 62% yield).

Synthesis of Intermediate M20: To a 100-ml dry reaction flask, intermediate M19 (0.66 g, 1 eq) was added and dissolved in 30 ml of dioxane. Potassium acetate (0.53 g, 4 eq), 31 (1-Boc-pyrazole-boronic acid pinacol ester, 0.47 g, 1.2 eq), and $Pd(dppf)Cl_2$ ( 0.07 g, 0.1 eq) were then added. 5 ml of water was then added. The reaction was stirred at 80 ° C under nitrogen for 36 h. The reaction was monitored by TLC (petroleum ether:ethyl acetate = 1:1). The reaction solution was then cooled to room temperature, the insoluble material was filtered off, and the filtrate was concentrated. The residue was purified by silica gel column chromatography (V (petroleum ether): V (ethyl acetate) = 1:1 as eluent) to afford intermediate M20 (0.48 g, 75% yield).

Synthesis of intermediate m21: Compound m20 (0.48 g, 1 eq) was added to a 50 ml dry reaction flask and dissolved in 20 ml of THF. Lithium hydroxide (0.17 g, 4 eq) was dissolved in 5 ml of water, followed by 5 ml of methanol, which was then added to the reaction mixture and stirred at room temperature for 4 h. The reaction was monitored by TLC (petroleum ether:ethyl acetate = 1:1). The reaction mixture was concentrated, and 20 ml of water was added to the residue . The pH was adjusted to approximately 4-5 with 1 M HCl, and then extracted with ethyl acetate (30 ml x 3). The organic phases were combined , washed with 40 ml of saturated brine, and dried three times over anhydrous $Na_2SO_4$ . The organic phase was concentrated to afford compound m21 (0.46 g, 99% yield) as a pale yellow solid.

Synthesis of Intermediate m22: To a 100 ml dry reaction flask, intermediate m21 (0.46 g, 1 eq) was added and dissolved in 40 ml of THF. Glycine methyl ester hydrochloride (0.15 g, 1.2 eq), DIPEA (0.52 g, 4 eq), and HATU (0.45 g, 12 eq) were then added and stirred at room temperature for 6 h. The reaction was monitored by TLC (petroleum ether:ethyl acetate = 1:1). 30 ml of water was added to the reaction solution, and the mixture was extracted with DCM (30 ml x 3). The organic phase was collected, washed with 40 ml of saturated brine, and dried over anhydrous $Na_2SO_4$. The residue was purified by silica gel column chromatography (eluent: petroleum ether:ethyl acetate = 1:1) to afford product m22 (0.35 g,

66% yield) as a pale yellow viscous solid.

Synthesis of intermediate m23: Compound m22 (0.35 g, 1 eq) was added to a 50 ml dry reaction flask and dissolved in 20 ml of THF. Lithium hydroxide (0.11 g, 4 eq) was dissolved in 5 ml of water, followed by 5 ml of methanol, which was then added to the reaction mixture and stirred at room temperature for 4 h. The reaction was monitored by TLC (petroleum ether:ethyl acetate = 1:1). The reaction mixture was concentrated, and the residue was added with 20 ml of water. The pH was adjusted to approximately 4-5 with 1 M HCl, and then extracted with ethyl acetate (30 ml x 3). The organic phases were combined, washed with 40 ml of saturated brine, and dried three times over anhydrous $Na_2SO_4$ . The organic phase was concentrated to afford compound m23 (0.34 g, 99% yield) as a pale yellow solid.

Synthesis of Intermediate m24: To a 100-ml dry reaction flask, intermediate m23 (0.34 g, 1 eq) was added and dissolved in 15 ml of THF. Tert-butyl-(pyrrolidin-2-ylmethyl)carbamate (0.14 g, 1.2 eq), DIPEA (0.40 g, 4 eq), and HATU (0.27 g, 1.2 eq) were then added and stirred at room temperature for 6 h. The reaction was monitored by TLC (ethyl acetate). The reaction solution was concentrated, and 20 ml of water was added to the residue. The mixture was extracted with DCM (30 ml x 3). The organic phases were combined, washed with 40 ml of saturated brine, and dried over anhydrous $Na_2SO_4$. The residue was purified by silica gel column chromatography (ethyl acetate as the eluent) to afford product m24 (0.27 g, 58% yield) as a pale yellow solid.

Synthesis of 115-020: m24 (100 mg) was added to a 15 mL DCM solution, followed by the dropwise addition of 5 mL of TFA. The reaction mixture was stirred at room temperature for 6 h. TLC was monitored until completion of the reaction. The filtrate was concentrated by evaporation under reduced pressure and evaporated to dryness to obtain the crude product, which was then extracted with water and DCM. The organic layer was washed with a saturated aqueous NaCl solution and dried over anhydrous Na2SO4 solid. The reaction mixture was concentrated by evaporation under reduced pressure to obtain the crude product. The crude product was purified by reverse phase preparative purification (water/acetonitrile/trifluoroacetic acid) to obtain the product 115-020. The product was then frozen at -80 ° C for 6 h and then freeze-dried to obtain the final product, 115-020, as a white solid (44 mg, yield 51.76%); HPLC analysis >95%.

(2) Compound 115-021 was prepared by a method similar to that of compound 115-020.

**Preparation Example 4: Synthesis of Compounds 115-027, 115-028, 115-029, 115-030,** and **115-031**

(1) Synthesis of Compound 115-027

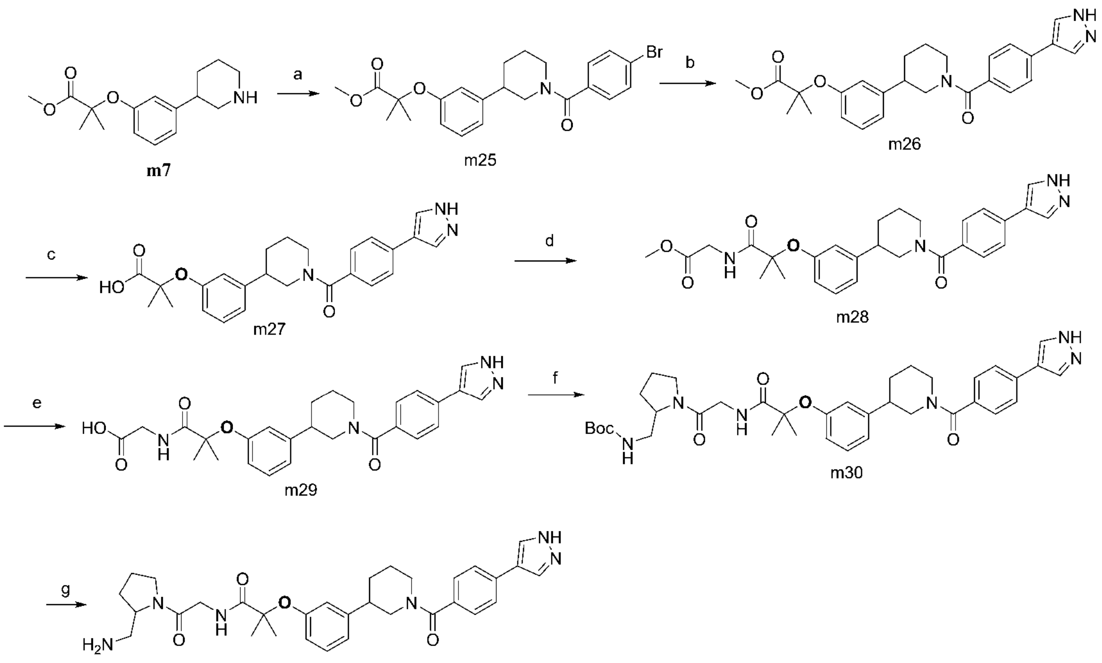

115-027

**Reagents** and **conditions:** (a) 4-Bromobenzoic acid HATU, DIPEA, THF, rt, 6h; (b) Pd(dppf)$Cl_2$ , $CH_3COOK$, KF, THF/$H_2O$ (5:1), 75 °C , 16h; (c) LiOH ·$H_2O$, THF/MeOH/$H_2O$ (3:1:1), rt, overnight. (d) Glycine methyl ester hydrochloride, HATU, DIPEA, THF, RT, 6h. (e) LiOH ·$H_2O$, THF/MeOH/$H_2O$ (3:1:1), rt, overnight. (f) tert-Butyl (2-pyrrolidin-1-ylmethyl)carbamate, HATU, DIPEA, THF, rt, 6h. (g) TFA/DCM (1:3), RT, 6h.

**Specific steps:**

Synthesis of Intermediate m25: To a 100 ml dry reaction flask, add Intermediate m7 (0.4 g, 1 eq) and dissolve in 40 ml of THF. Then, add p-bromobenzoic acid (0.35 g, 1.2 eq), DIPEA (0.75 g, 4 eq), and HATU (0.67 g, 1.2 eq) and stir at room temperature for 6 h. The reaction was monitored by TLC (petroleum ether:ethyl acetate = 10:1). 30 ml of water was added to the reaction solution, and the mixture was extracted with DCM (30 ml x 3). The organic phase was collected, washed with 40 ml of saturated brine, and dried over anhydrous $Na_2SO_4$ . The residue was purified by silica gel column chromatography (eluent: petroleum ether:ethyl acetate = 10:1) to afford product m25 (0.47 g, 71% yield) as a pale yellow viscous solid.

Synthesis of Intermediate M26: To a 100-ml dry reaction flask, intermediate M25 (0.47 g, 1 eq) was added and dissolved in 30 ml of dioxane. Potassium acetate (0.4 g, 4 eq), 1-Boc-pyrazole-boronic acid pinacol ester (0.34 g, 1.1 eq), and Pd(dppf)$Cl_2$ ( 0.07 g, 0.01 eq) were then added. 5 ml of water was then added. The reaction was stirred at 80 ° C under nitrogen for 36 h. The reaction was monitored by TLC (petroleum ether:ethyl acetate = 1:1). The reaction solution was then cooled to room temperature, the insoluble material was filtered off, and the filtrate was concentrated. The residue was purified by silica gel column chromatography (V (petroleum ether): V (ethyl acetate) = 1:1 as eluent) to afford intermediate M26 (0.28 g, 62% yield).

Synthesis of intermediate m27: Compound m26 (0.28 g, 1 eq) was added to a 50 ml dry reaction flask and dissolved in 20 ml of THF. Lithium hydroxide (0.11 g, 4 eq) was dissolved in 5 ml of water, followed by 5 ml of methanol, which was then added to the reaction mixture and stirred at room temperature for 4 h. The reaction was monitored by TLC (petroleum ether:ethyl acetate = 1:1). The reaction mixture was concentrated, and 20 ml of water was added to the residue. The pH was adjusted to approximately 4-5 with 1 M HCl, and then extracted with ethyl acetate (30 ml x 3). The organic phases were combined, washed with 40 ml of saturated brine, and dried three times over anhydrous $Na_2SO_4$ . The organic phases were concentrated to afford compound m27 (0.27 g, 99% yield) as a pale yellow solid.

Synthesis of Intermediate m28: To a 100 ml dry reaction flask, intermediate m27 (0.27 g, 1 eq) was added and dissolved in 40 ml of THF. Glycine methyl ester hydrochloride (0.1 g, 1.2 eq), DIPEA (0.32 g, 4 eq), and HATU (0.28 g, 1.2 eq) were then added and stirred at room temperature for 6 h. The reaction was monitored by TLC (petroleum ether:ethyl acetate = 1:1). 30 ml of water was added to the reaction solution, and the mixture was extracted with DCM (30 ml x 3). The organic phase was collected, washed with 40 ml of saturated brine, and dried over anhydrous $Na_2SO_4$. The residue was purified by silica gel column chromatography (eluent: petroleum ether:ethyl acetate = 1:1) to afford product m28 (0.26 g, 81.2% yield) as a pale yellow viscous solid.

Synthesis of intermediate m29: Compound m28 (0.26 g, 1 eq) was added to a 50 mL dry reaction flask and dissolved in 20 mL of THF. Lithium hydroxide (0.09 g, 4 eq) was dissolved in 5 mL of water, followed by 5 mL of methanol, which was then added to the reaction mixture and stirred at room temperature for 4 h. The reaction was monitored by TLC (petroleum ether:ethyl acetate = 1:1). The reaction mixture was concentrated, and the residue was added with 20 mL of water. The pH was adjusted to approximately 4-5 with 1 M HCl, and then extracted with ethyl acetate (30 mL x 3). The organic phases were combined, washed with 40 mL of saturated brine, and dried three times over anhydrous $Na_2SO_4$ . The organic phases were concentrated to afford compound m29 (0.25 g, 99% yield) as a pale yellow solid.

Synthesis of Intermediate M30: To a 100-ml dry reaction flask, intermediate M29 (0.25 g, 1 eq) was added and dissolved in 15 ml of THF. Tert-butyl-(pyrrolidin-2-ylmethyl)carbamate (0.13 g, 1.2 eq), DIPEA (0.32 g, 1.2 eq), and HATU (0.25 g, 1.2 eq) were then added and stirred at room temperature for 6 h. The reaction was monitored by TLC (ethyl acetate). The reaction solution was concentrated, and 20 ml of water was added to the residue. The mixture was extracted with DCM (30 ml x 3). The organic phases were combined, washed with 40 ml of saturated brine, and dried over anhydrous $Na_2SO_4$. The residue was purified by silica gel column chromatography (ethyl acetate as the eluent) to afford product M30 (0.22 g, 65% yield) as a pale yellow solid.

Synthesis of 115-027: m30 (100 mg) was added to a 15 mL DCM solution, followed by the dropwise addition of 5 mL of TFA. The reaction mixture was stirred at room temperature for 6 hours. TLC was monitored until the reaction was complete. The filtrate was concentrated by evaporation under reduced pressure and evaporated to dryness to obtain the crude product, which was then extracted with water and DCM. The organic layer was washed with a saturated aqueous NaCl solution and dried over anhydrous $Na_2SO_4$ solid. The reaction mixture was concentrated by evaporation under reduced pressure to obtain the crude product. The crude product was purified by reverse phase preparative purification (water/acetonitrile/trifluoroacetic acid) to obtain a preparation of product 115-027. The product was then frozen at -80 ° C for 6 hours and then freeze-dried to obtain the final product, 115-027, as a white solid (28 mg, yield 35.88%); HPLC analysis >95%.

(2) Compounds 115-028, 115-029, 115-030, and 115-031 were prepared by a method similar to that of compound 115-027.

**Preparation Example 5: Synthesis of Compounds 115-047, 115-028, 115-029, 115-030,** and **115-031**

(1) Synthesis of compound 115-047

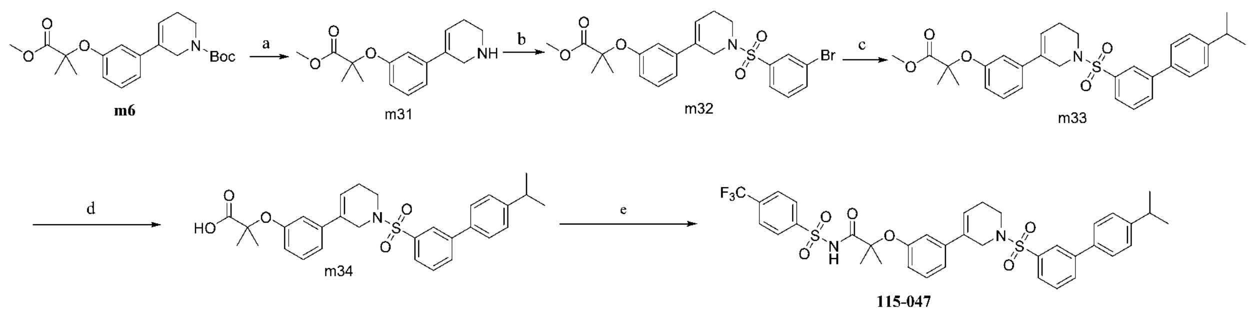


**Reagents** and **conditions:** (a) TFA/DCM (1:3), RT, 6 h; (b) 5-Bromo-2-chlorobenzenesulfonyl chloride, TEA, dry DCM, RT, 24 h; (c) 4-Isopropylphenylboronic acid pinacol ester, $Pd(PPh_{3})_4$ , THF/2 M$K_2CO_3$ (5:1), 75 °C , 24 h; (d) LiOH $\cdot H_2O$, THF/MeOH/$H_2O$ (3:1:1), RT, overnight. (e) p-Trifluoromethylbenzenesulfonamide, EDCI, DMAP, DCM, RT, overnight.

**Specific steps:**

Synthesis of intermediate m31: m6 (0.3 g, 1 eq) was added to a DCM solution (15 mL), followed by the dropwise addition of 5 mL of TFA. The reaction mixture was stirred at room temperature for 6 h. TLC was monitored until completion of the reaction. The filtrate was concentrated by evaporation under reduced pressure to dryness to obtain the crude product m7 (0.29 g, 100% yield) as a pale yellow oil.

Synthesis of Intermediate m32: Intermediate m31 (0.29 g, 1 eq), 5-bromo-2-chlorobenzenesulfonyl chloride (0.33 g, 1.1 eq), TEA (0.31 g, 3 eq), and anhydrous DCM solution (15 ml) were added to a 50 ml round-bottom flask and stirred at room temperature under nitrogen. After TLC monitoring until completion of the reaction, the filtrate was concentrated by evaporation under reduced pressure, diluted with water, and extracted with ethyl acetate. The organic layer was dried over anhydrous $Na_2SO_4$ , and the filtrate was concentrated by evaporation under reduced pressure to obtain the crude product. The crude product was purified by silica gel column chromatography (petroleum ether/ethyl acetate = 5:1) to obtain intermediate m32 as a colorless oil, 0.35 g, in a yield of 67.31%.

Synthesis of intermediate m33: m32 (0.30 g, 1 eq), 4-isopropylphenylboronic acid pinacol ester (0.35 g, 1.1 eq), and $Pd(PPh_{3})_4$ (436.09 mg, 0.19 mmol) were added to a solution of THF/2M $K_2CO_3$ (5:1) (15:3 mL) at 75 ° C . The reaction was stirred under nitrogen for 12 h. TLC monitoring was performed until the reaction was complete. The filtrate was concentrated by evaporation under reduced pressure, and the residue was extracted with water and ethyl acetate. The organic layer was washed with saturated NaCl solution and dried over anhydrous $Na_2SO_4$. The filtrate was concentrated by evaporation under reduced pressure to obtain the crude product. The crude product was purified by silica gel column chromatography (petroleum ether/ethyl acetate = 10:1) to obtain intermediate m33 as a colorless oil, 0.24 g, in a yield of 64.89%.

Synthesis of intermediate m34: m33 (0.24 g, 1 eq) and LiOH·$H_2O$ ( 69 mg, 4 eq) were added to a solution of THF/MeOH/$H_2O$ ( 3:1:1) (12:4:4 mL) and stirred overnight at room temperature. After completion of the reaction, the mixture was concentrated under reduced pressure. The resulting residue was dissolved in ethyl acetate, adjusted to pH 2 with 1M HCl, and extracted with ethyl acetate. The organic layer was dried over anhydrous $Na_2SO_4$ and the reaction mixture was concentrated by evaporation under reduced pressure to afford crude product m34 as a white solid (0.23 g), which was used directly in the next step.

Synthesis of 115-047: m33 (0.23 g, 1 eq), p-trifluoromethylbenzenesulfonamide (89.2 mg, 1.1 eq), EDCI (147 mg, 1.3 eq), and DMAP (127 mg, 2 eq) were added to a DCM solution (15 ml). The mixture was stirred at room temperature overnight. After TIC monitoring until the reaction was complete, the mixture was adjusted to pH 2 with 1 M HCl and extracted with DCM. The organic layer was washed with saturated aqueous NaCl and dried over anhydrous $Na_2SO_4$. The reaction mixture was concentrated by evaporation under reduced pressure to obtain a crude product. The crude product was purified by reverse phase preparative purification (water/acetonitrile/trifluoroacetic acid) to obtain a preparation of product 115-047. The product was frozen at -80 ° C for 6 h and then freeze-dried to obtain the final product 115-047 as a white solid (126 mg, yield 39.34%); HPLC purity >95%.

**Preparation Example 6:** Synthesis of Compounds 115-024, 115-025, and 115-026

(1) Synthesis of Compound -025

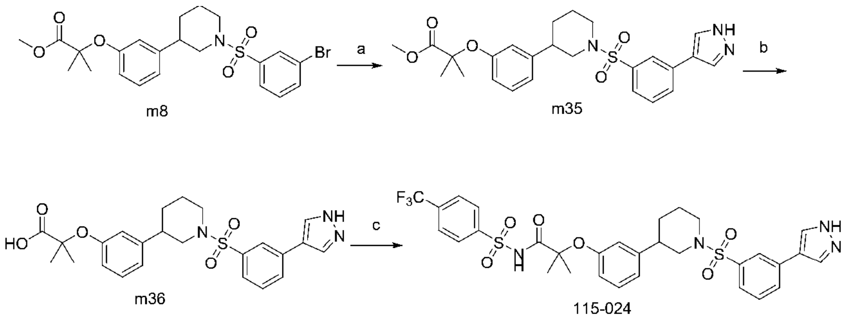


m8 m35 m36 115-024

Reagents and conditions: (a) Pd(dppf)Cl2, CH3COOK, KF, THF/H2O (5:1), 75°C, 16 h; (b) LiOH ·H2O, THF/MeOH/$H_2O$ (3:1:1), RT, overnight; (c) p-Trifluoromethylbenzenesulfonamide, EDCI, DMAP, DCM, RT, overnight.

**Specific steps:**

Synthesis of intermediate m35: Raw materials m8 (0.1 g, 1 eq), m2 (0.0652 g, 1.1 eq), potassium acetate (0.0791 g, 4 eq), potassium fluoride (0.0468 g, 4 eq), and catalyst Pd(dppf)Cl2 (0.0148 g, 0.1 eq) were added to THF/H2O (24 mL). The mixture was heated to 75°C and stirred under nitrogen for 16 h. The reaction was monitored by TLC until completion. The filtrate was concentrated by evaporation under reduced pressure and evaporated to dryness to obtain the crude product. The crude product was purified by silica gel column chromatography (petroleum ether/ethyl acetate = 6:1) to obtain intermediate m35 as a light yellow oil (0.0883 g, 90.7% yield).

Synthesis of intermediate m36: m35 (0.0883 g, 1 eq) and LiOH·$H_2O$ (0.0176 g, 4 eq) were added to a solution of THF/MeOH/$H_2O$ (3:1:1) (12:4:4 mL) and stirred at room temperature overnight. After completion of the reaction, the mixture was concentrated under reduced pressure. The resulting residue was dissolved in ethyl acetate, adjusted to pH 2 with 1M HCl, and extracted with ethyl acetate. The organic layer was dried over anhydrous $Na_2SO_4$ and the reaction mixture was concentrated by evaporation under reduced pressure to afford crude product m36 as a white solid (0.0788 g), which was used directly in the next step.

Synthesis of 115-025: m36 (0.0788 g, 1 eq), p-trifluoromethylbenzenesulfonamide (0.0407 mg, 1.1 eq), EDCI (0.0331 g, 1.3 eq), and DMAP (0.0401 g, 2 eq) were added to a DCM solution (15 ml). The mixture was stirred at room temperature overnight. After TIC monitoring until the reaction was complete, the mixture was adjusted to pH 2 with 1 M HCl and extracted with DCM. The organic layer was washed with saturated aqueous NaCl and dried over anhydrous Na2SO4. The reaction solution was concentrated by evaporation under reduced pressure to obtain a crude product. The crude product was purified by reverse phase preparative purification (water/acetonitrile/trifluoroacetic acid) to obtain a preparation of product 115-025. The product was frozen at -80°C for 6 h and then freeze-dried to obtain the final product 115-025 as a white solid, 0.0262 g, in a yield of 23.57% (HPLC >95%).

(2) Compounds 115-024 and 115-026 were prepared by a method similar to that of compound 115-025.

**Preparation Example 7:** Synthesis of Compound 115-049

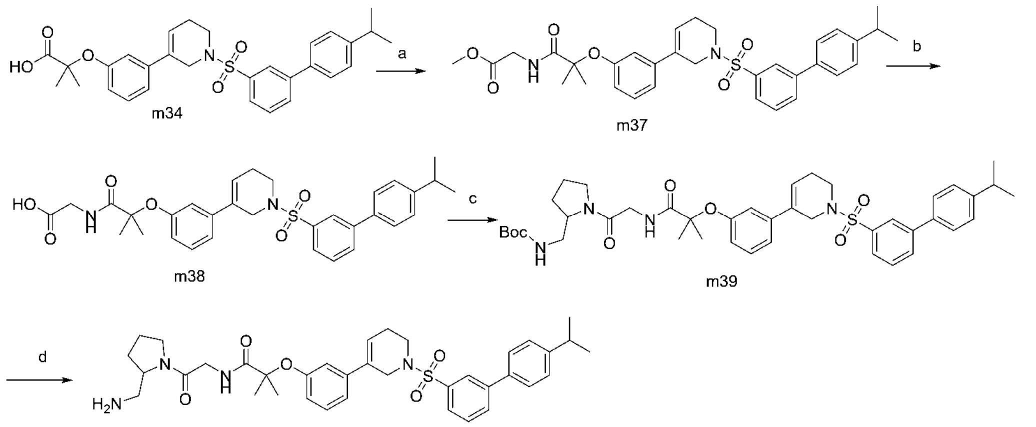

m34
m37
m38
m39
115-049

Reagents and conditions: (a) Glycine methyl ester hydrochloride, HATU, DIPEA, THF, RT, 6 h; (b) LiOH ·H2O, THF/MeOH/$H_2O$ (3:1:1), RT, overnight; (c) tert-Butyl (2-pyrrolidin-1-ylmethyl)carbamate, HATU, DIPEA, THF, rt, 6 h; (d) TFA/DCM (1:3), RT, 6 h.

**Specific steps:**

Synthesis of Intermediate m37: To a 100 ml dry reaction flask, add intermediate m34 (0.28 g, 1 eq) and dissolve in 40 ml of THF. Then, add glycine methyl ester hydrochloride (0.09 g, 1.2 eq), DIPEA (0.62 g, 8 eq), and HATU (0.23 g, 1 eq) and stir at room temperature for 6 h. The reaction was monitored by TLC (petroleum ether:ethyl acetate = 1:1). 30 ml of water was added to the reaction solution, and the mixture was extracted with DCM (30 ml x 3). The organic phase was collected, washed with 40 ml of saturated brine, and dried over anhydrous Na2SO4. The residue was purified by silica gel column chromatography (eluent: petroleum ether:ethyl acetate = 1:1) to afford product m37 (0.33 g, 98.3% yield) as a pale yellow viscous solid.

Synthesis of intermediate m38: Compound m37 (0.33 g, 1 eq) was added to a 50 mL dry reaction flask and dissolved in 20 mL of THF. Lithium hydroxide (0.124 g, 5 eq) was dissolved in 5 mL of water, followed by 5 mL of methanol, which was then added to the reaction mixture and stirred at room temperature for 4 h. The reaction was monitored by TLC (petroleum ether:ethyl acetate = 1:1). The reaction mixture was concentrated, and the residue was added with 20 mL of water. The pH was adjusted to approximately 4-5 with 1 M HCl, and then extracted with ethyl acetate (30 mL x 3). The organic phases were combined, washed with 40 mL of saturated brine, and dried three times over anhydrous Na2SO4. The organic phase was concentrated to afford compound m38 (0.29 g, 93.54% yield) as a pale yellow solid.

Synthesis of Intermediate m39: To a 100-ml dry reaction flask, intermediate m38 (0.29 g, 1 eq) was added and dissolved in 25 ml of THF. Tert-butyl-(pyrrolidin-2-ylmethyl)carbamate (0.13 g, 1.2 eq), DIPEA (0.572 g, 8 eq), and HATU (0.21 g, 1 eq) were then added and stirred at room temperature for 6 h. The reaction was monitored by TLC (ethyl acetate). The reaction solution was concentrated, and 20 ml of water was added to the residue. The mixture was extracted with DCM (30 ml x 3). The organic phases were combined, washed with 40 ml of saturated brine, and dried over anhydrous $Na_2SO_4$. The residue was purified by silica gel column chromatography (ethyl acetate as the eluent) to afford product m39 (0.23 g, 53% yield) as a pale yellow solid.

Synthesis of 115-049: m39 (100 mg) was added to a 15 mL DCM solution, followed by the dropwise addition of 5 mL of TFA. The reaction mixture was stirred at room temperature for 6 hours. TLC was monitored until the reaction was complete. The filtrate was concentrated by evaporation under reduced pressure and evaporated to dryness to obtain the crude product, which was then extracted with water and DCM. The organic layer was washed with a saturated aqueous NaCl solution and dried over anhydrous $Na_2SO_4$ solid. The reaction mixture was concentrated by evaporation under reduced pressure to obtain the crude product. The crude product was purified by reverse phase preparative purification (water/acetonitrile/trifluoroacetic acid) to obtain a preparation of product 115-049. The product was then frozen at -80°C for 6 hours and then freeze-dried to obtain the final product, 115-049, as a white solid (43 mg, yield 49.60%); HPLC analysis >95%.

**Preparation Example 8:** Synthesis of Compounds 115-052 and 115-055

(1) Synthesis of compound 115-052

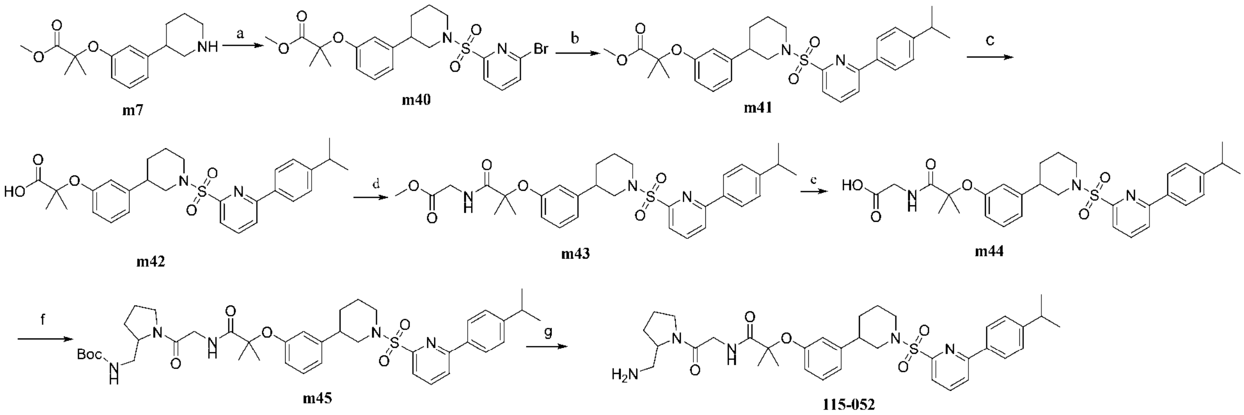

m7 m40 m41 m42 m43 m44 m45 115-052

Reagents and conditions: (a) 5-Bromo-2-pyridinium sulfonyl chloride, TEA, dry DCM, RT, 24 h; (b) 4-Isopropylphenylboronic acid pinacol ester, Pd(PPh3)4, THF/2 M K2CO3 (5:1), 75°C, 24 h; (c) LiOH ·H2O, THF/MeOH/$H_2O$ (3:1:1), RT, overnight. (d) Glycine methyl ester hydrochloride, HATU, DIPEA, THF, RT, 6 h. (e) LiOH ·H2O, THF/MeOH/-$H_2O$ (3:1:1), rt, overnight. (f) tert-Butyl (R)-(pyrrolidin-2-ylmethyl)carbamate, HATU, DIPEA, THF, rt, 6 h. (g) TFA/DCM (1:3), RT, 6 h.

**Specific steps:**

Synthesis of Intermediate m40: Intermediate m7 (0.6 g, 1 eq), 5-bromo-2-sulfonylpyridine chloride (0.7 g, 1.1 eq), TEA (0.62 g, 3 eq), and anhydrous DCM solution (15 ml) were added to a 50 ml round-bottom flask and stirred at room temperature under nitrogen. After TLC monitoring until completion of the reaction, the filtrate was concentrated by evaporation under reduced pressure, diluted with water, and extracted with ethyl acetate. The organic layer was dried over anhydrous $Na_2SO_4$ solid, and the filtrate was concentrated by evaporation under reduced pressure to obtain the crude product. The crude product was purified by silica gel column chromatography (petroleum ether/ethyl acetate = 5:1) to obtain intermediate m8 as a colorless oil, 0.77 g, in a yield of 70.64%.

Synthesis of intermediate m41: m40 (0.30 g, 1 eq), 4-isopropylphenylboronic acid pinacol ester (0.15 g, 1.1 eq), and Pd(PPh3)4 (216.09 mg, 0.19 mmol) were added to a solution of THF/2M K2CO3 (5:1) (15:3 mL) at 75°C. The reaction was stirred under nitrogen for 12 h. TLC was monitored until the reaction was complete. The filtrate was concentrated by evaporation under reduced pressure, and the residue was extracted with water and ethyl acetate. The organic layer was washed with saturated NaCl solution and dried over anhydrous Na2SO4. The filtrate was concentrated by evaporation under reduced pressure to obtain the crude product. The crude product was purified by silica gel column chromatography (petroleum ether/ethyl acetate = 10:1) to obtain intermediate m9 as a colorless oil, 0.23 g, in a yield of 74.19%.

Synthesis of intermediate m42: m41 (0.23 g, 1 eq) and LiOH·$H_2O$ (69 mg, 4 eq) were added to a solution of THF/MeOH/$H_2O$ (3:1:1) (12:4:4 mL) and stirred at room temperature overnight. After completion of the reaction, the mixture was concentrated under reduced pressure. The resulting residue was dissolved in ethyl acetate, adjusted to pH 2 with 1M HCl, and extracted with ethyl acetate. The organic layer was dried over anhydrous $Na_2SO_4$ and the reaction solution was concentrated by evaporation under reduced pressure to afford crude product m10 as a white solid (0.26 g), which was used directly in the next step.

Synthesis of Intermediate m43: To a 100 ml dry reaction flask, intermediate m42 (0.26 g, 0.57 mmol) was added and dissolved in 40 ml of THF. Glycine methyl ester hydrochloride (0.12 g, 0.93 mmol), DIPEA (0.40 g, 3.09 mmol), and HATU (0.32 g, 0.85 mmol) were then added and stirred at room temperature for 6 h. The reaction was monitored by TLC (petroleum ether:ethyl acetate = 1:1). 30 ml of water was added to the reaction solution, and the mixture was extracted with DCM (30 ml x 3). The organic phase was collected, washed with 40 ml of saturated brine, and dried over anhydrous $Na_2SO_4$. The residue was purified by silica gel column chromatography (eluent: petroleum ether:ethyl acetate = 1:1) to afford product m11 (0.37 g, 81% yield) as a pale yellow viscous solid.

Synthesis of intermediate m44: Compound m43 (0.37 g, 0.63 mmol) was added to a 50 mL dry reaction flask and dissolved in 20 mL of THF. Lithium hydroxide (0.12 g, 2.52 mmol) was dissolved in 5 mL of water, followed by 5 mL of methanol, which was then added to the reaction mixture and stirred at room temperature for 4 h. The reaction was monitored by TLC (petroleum ether:ethyl acetate = 1:1). The reaction mixture was concentrated, and 20 mL of water was added to the residue. The pH was adjusted to approximately 4-5 with 1 M HCl, and then extracted with ethyl acetate (30 mL x 3). The organic phases were combined, washed with 40 mL of saturated brine, and dried three times over anhydrous $Na_2SO_4$. The organic phase was concentrated to afford compound m12 (0.33 g, 99% yield) as a pale yellow solid.

Synthesis of Intermediate m45: To a 100-ml dry reaction flask, intermediate m44 (0.32 g, 0.57 mmol) was added and dissolved in 15 ml of THF. Tert-butyl (R)-(pyrrolidin-2-ylmethyl)carbamate (0.14 g, 0.69 mmol), DIPEA (0.30 g, 2.30

mmol), and HATU (0.24 g, 0.63 mmol) were then added and stirred at room temperature for 6 h. The reaction was monitored by TLC (ethyl acetate). The reaction solution was concentrated, and 20 ml of water was added to the residue. The mixture was extracted with DCM (30 ml x 3). The organic phases were combined, washed with 40 ml of saturated brine, and dried over anhydrous Na2SO4. The residue was purified by silica gel column chromatography (ethyl acetate as the eluent) to afford product m13 (0.32 g, 74% yield) as a pale yellow solid.

Synthesis of 115-052: m45 (100 mg) was added to a 15 mL DCM solution, followed by the dropwise addition of 5 mL of TFA. The reaction mixture was stirred at room temperature for 6 hours. TLC was monitored until the reaction was complete. The filtrate was concentrated by evaporation under reduced pressure and evaporated to dryness to obtain the crude product, which was then extracted with water and DCM. The organic layer was washed with a saturated aqueous NaCl solution and dried over anhydrous $Na_2SO_4$ solid. The reaction mixture was concentrated by evaporation under reduced pressure to obtain the crude product. The crude product was purified by reverse phase preparative purification (water/acetonitrile/trifluoroacetic acid) to obtain the product 115-052. The product was frozen at -80°C for 6 hours and then freeze-dried to obtain the final product, 115-052, as a white solid (44 mg, yield 52.11%); HPLC analysis >95%.

**(2)** Compound 115-055 was prepared by a method similar to that of compound 115-052.

**Preparation Example 9: Characterization of Compounds**

The characterization results of the compounds prepared above are shown in the following table:

| | |
|---|---|
| 115-010 | 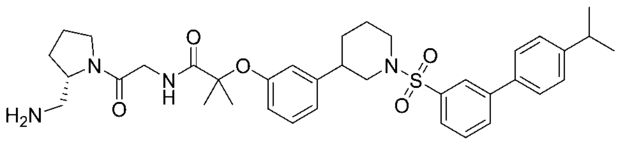<br>N-(2-((S)-2-(Aminomethyl)pyrrolidin-1-yl)-2-oxoethyl)-2-(3-(1-((4'-isopropyl-[1,1'-biphenyl]-3-yl)sulf onylpiperidin-3-yl)phenoxy)-2-methylpropanamide<br>$^1$ H NMR(400 MHz, DMSO- *d* $_{6)}\delta$ 8.28 (t, *J* = 5.3 Hz, 1H), 7.93 (dd, *J* = 6.9, 1.5 Hz, 2H), 7.83 - 7.75 (m, 1H), 7.63 - 7.56 (m, 2H), 7.60 - 7.48 (m, 1H), 7.33 (d, *J* = 8.1 Hz, 2H), 7.25 - 7.17 (m, 1H), 6.96 - 6.88 (m, 1H), 6.79 (t, *J* = 2.2 Hz, 1H), 6.74 (dt, *J* = 7.0, 1.6 Hz, 1H), 4.48 (t, *J* = 6.8 Hz, 2H), 4.22 (dd, *J* = 5.1, 1.0 Hz, 2H), 3.95 - 3.86 (m, 1H), 3.78 - 3.68 (m, 1H), 3.51 (ddd, *J* = 12.2, 4.9, 3.1 Hz, 1H), 3.40 - 3.31 (m, 2H), 3.20 (dd, *J* = 12.5, 5.1 Hz, 1H), 2.91 (ddd, *J* = 12.6, 9.1, 5.9 Hz, 2H), 2.65 (dtd, *J* = 11.3, 6.8, 4.6 Hz, 1H), 2.51 (p, *J* = 5.4 Hz, 1H), 2.46 - 2.35 (m, 1H), 2.12 - 2.00 (m, 1H), 1.97 - 1.83 (m, 2H), 1.77 (dddd, *J* = 11.2, 9.6, 7.4, 4.4 Hz, 2H), 1.67 - 1.53 (m, 8H), 1.39 (dddd, *J* = 12.2, 8.4, 6.4, 2.7 Hz, 1H), 1.22 (d, *J* = 6.5 Hz, 3H), 1.17 (d, *J* = 6.5 Hz, 3H). ESI-MS<br>Calculated for $C_{37}H_{48}N_4O_5S[M+H]^+$ :661.88, measured : 661.43 |
| 115-011 | 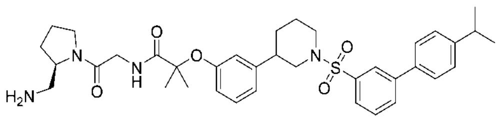<br>N-(2-((R)-2-(Aminomethyl)pyrrolidin-1-yl)-2-oxoethyl)-2-(3-(1-((4'-isopropyl-[1,1'-biphenyl]-3-yl)sul fonylpiperidin-3-yl)phenoxy)-2-methylpropanamide<br>$^1$ H NMR(400 MHz, DMSO- *d* $_{6)}\delta$ 8.28 (t, *J* = 5.3 Hz, 1H), 7.93 (dd, *J* = 6.9, 1.5 Hz, 2H), 7.83 - 7.75 (m, 1H), 7.63 - 7.56 (m, 2H), 7.60 - 7.48 (m, 1H), 7.33 (d, *J* = 8.1 Hz, 2H), 7.25 - 7.17 (m, 1H), 6.96 - 6.88 (m, 1H), 6.79 (t, *J* = 2.2 Hz, 1H), 6.74 (dt, *J* = 7.0, 1.6 Hz, 1H), 4.48 (t, *J* = 6.8 Hz, 2H), 4.22 (dd, *J* = 5.1, 1.0 Hz, 2H), 3.95 - 3.86 (m, 1H), 3.78 - 3.68 (m, 1H), 3.51 (ddd, *J* = 12.2, 4.9, 3.1 Hz, 1H), 3.40 - 3.31 (m, 2H), 3.20 (dd, *J* = 12.5, 5.1 Hz, 1H), 2.91 (ddd, *J* = 12.6, 9.1, 5.9 Hz, 2H), 2.65 (dtd, *J* = 11.3, 6.8, 4.6 Hz, 1H), 2.51 (p, *J* = 5.4 Hz, 1H), 2.46 - 2.35 (m, 1H), 2.12 - 2.00 (m, 1H), 1.97 - 1.83 (m, 2H), 1.77 (dddd, *J* = 11.2, 9.6, 7.4, 4.4 Hz, 2H), 1.67 - 1.53 (m, 8H), 1.39 (dddd, *J* = 12.2, 8.4, 6.4, 2.7 Hz, 1H), 1.22 (d, *J* = 6.5 Hz, 3H), 1.17 (d, *J* = 6.5 Hz, 3H).. ESI-MS<br>Calculated for $C_{37}H_{48}N_4O_5S[M+H]^+$ :661.88, measured : 661.47 |

(continued)

| | |
|---|---|
| 115-012 | 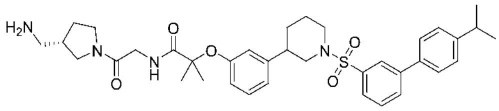<br>N-(2-((S)-3-(Aminomethyl)pyrrolidin-1-yl)-2-oxoethyl)-2-(3-(1-((4'-isopropyl-[1,1'-biphenyl]-3-yl)sulf onylpiperidin-3-yl)phenoxy)-2-methylpropanamide<br>$^{1}$ H NMR(400 MHz, DMSO- $d_{6)}$$\delta$ 8.28 (t, *J* = 5.0 Hz, 1H), 7.93 (dd, J = 7.0, 1.5 Hz, 2H), 7.83 - 7.75 (m, 1H), 7.63 - 7.56 (m, 2H), 7.56 - 7.47 (m, 1H), 7.33 (d, *J* = 8.1 Hz, 2H), 7.25 - 7.17 (m, 1H), 6.96 - 6.88 (m, 1H), 6.79 (t, *J* = 2.2 Hz, 1H), 6.74 (dt, *J* = 7.0, 1.6 Hz, 1H), 3.84 (d, *J* = 5.0 Hz, 2H), 3.73 (ddd, *J* = 12.3, 7.1, 5.3 Hz, 1H), 3.56 - 3.46 (m, 2H), 3.40 - 3.30 (m, 2H), 3.30 - 3.16 (m, 2H), 2.91 (ddd, *J* = 12.6, 9.1, 5.9 Hz, 2H), 2.73 - 2.61 (m, 1H), 2.61 - 2.50 (m, 1H), 2.27 (ddt, *J* = 12.5, 7.3, 5.1 Hz, 1H), 2.01 (dddd, *J* = 12.5, 7.5, 5.2, 3.1 Hz, 1H), 1.98 - 1.84 *ESI* - MS<br>Calculated for $C_{37}H_{48}N_{4}O_{5}S[M+H]^{+}$ :661.88, measured : 661.49 |
| 115-013 | 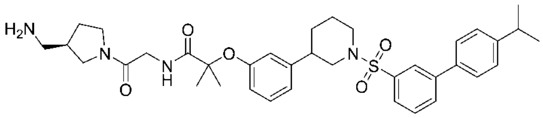<br>N-(2-((S)-3-(Aminomethyl)pyrrolidin-1-yl)-2-oxoethyl)-2-(3-(1-((4'-isopropyl-[1,1'-biphenyl]-3-yl)sulf onylpiperidin-3-yl)phenoxy)-2-methylpropanamide<br>$^{1}$ H NMR(400 MHz, DMSO- $d_{6)}$$\delta$ 8.28 (t, *J* = 5.0 Hz, 1H), 7.93 (dd, *J* = 7.0, 1.5 Hz, 2H), 7.83 - 7.75 (m, 1H), 7.63 - 7.56 (m, 2H), 7.56 - 7.47 (m, 1H), 7.33 (d, *J* = 8.1 Hz, 2H), 7.25 - 7.17 (m, 1H), 6.96 - 6.88 (m, 1H), 6.79 (t, *J* = 2.2 Hz, 1H), 6.74 (dt, *J* = 7.0, 1.6 Hz, 1H), 3.84 (d, *J* = 5.0 Hz, 2H), 3.73 (ddd, *J* = 12.3, 7.1, 5.3 Hz, 1H), 3.56 - 3.46 (m, 2H), 3.40 - 3.30 (m, 2H), 3.30 - 3.16 (m, 2H), 2.91 (ddd, *J* = 12.6, 9.1, 5.9 Hz, 2H), 2.73 - 2.61 (m, 1H), 2.61 - 2.50 (m, 1H), 2.27 (ddt, *J* = 12.5, 7.3, 5.1 Hz, 1H), 2.01 (dddd, *J* = 12.5, 7.5, 5.2, 3.1 Hz, 1H), 1.98 - 1.84 *ESI* - MS<br>Calculated for $C_{37}H_{48}N_{4}O_{5}S[M+H]^{+}$ :661.88, measured : 661.44 |
| 115-014 | 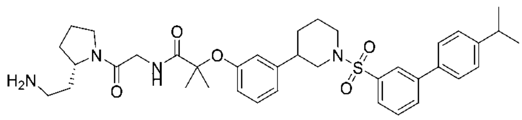<br>N-(2-((S)-2-(2-aminoethyl)pyrrolidin-1-yl)-2-oxoethyl)-2-(3-(1-((4'-isopropyl-[1,1'-biphenyl]-3-yl)sulf onylpiperidin-3-yl)phenoxy)-2-methylpropanamide)<br>$^{1}$ H NMR(400 MHz, DMSO- $d_{6)}$$\delta$ 8.28 (t, *J* = 5.3 Hz, 1H), 7.93 (dd, *J* = 6.9, 1.5 Hz, 2H), 7.83 - 7.75 (m, 1H), 7.63 - 7.56 (m, 2H), 7.60 - 7.48 (m, 1H), 7.33 (d, *J* = 8.1 Hz, 2H), 7.25 - 7.17 (m, 1H), 6.96 - 6.88 (m, 1H), 6.79 (t, *J* = 2.2 Hz, 1H), 6.74 (dt, *J* = 7.0, 1.6 Hz, 1H), 4.95 (qd, *J* = 6.5, 3.9 Hz, 1H), 4.46 (t, *J* = 6.7 Hz, 2H), 4.22 (dd, *J* = 5.1, 1.0 Hz, 2H), 3.78 - 3.68 (m, 1H), 3.51 (ddd, *J* = 12.1, 4.9, 3.1 Hz, 1H), 3.40 - 3.31 (m, 2H), 3.20 (dd, *J* = 12.5, 5.1 Hz, 1H), 3.10 - 2.85 (m, 5H), 2.56 - 2.46 (m, 1H), 2.34 - 2.22 (m, 1H), 2.12 - 1.98 (m, 2H), 1.97 - 1.83 (m, 2H), 1.76 (dddd, *J* = 11.4, 9.7, 8.4, 4.6 Hz, 2H), 1.67 - 1.53 (m, 8H), 1.39 (dddd, *J* = 12.2, 8.4, 6.4, 2.7 Hz, 1H), 1.22 (d, *J* = 6.5 Hz, 3H), 1.17 (d, *J* = 6.5 Hz, 3H). ESI-MS:<br>Calculated for $C_{37}H_{48}N_{4}O_{5}S[M+H]^{+}$ :661.88, measured : 661.44 |

(continued)

| | |
|---|---|
| 115-016 | 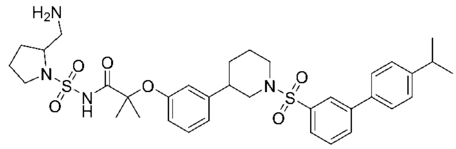 N-((2-(Aminomethyl)pyrrolidin-1-yl)sulfonyl)-2-(3-(1-((4'-isopropyl-[1,1'-biphenyl]-3-yl)sulfonylpipe ridin-3-yl)phenoxy)-2-methylpropionamide $^1$ H NMR(400 MHz, DMSO- *d* 6)δ 7.93 (dd, *J* = 6.9, 1.5 Hz, 2H), 7.83 - 7.75 (m, 1H), 7.63 - 7.56 (m, 2H), 7.60 - 7.47 (m, 1H), 7.33 (d, *J* = 8.1 Hz, 2H), 7.25 - 7.17 (m, 1H), 7.00 (s, 1H), 6.96 - 6.88 (m, 1H), 6.79 (t, *J* = 2.2 Hz, 1H), 6.74 (dt, *J* = 7.0, 1.6 Hz, 1H), 4.48 (t, *J* = 6.6 Hz, 2H), 4.19 - 4.09 (m, 1H), 4.01 - 3.91 (m, 1H), 3.40 - 3.31 (m, 2H), 3.20 (dd, J = 12.5, 5.1 Hz, 1H), 2.91 (ddd, *J* = 12.6, 9.1, 5.9 Hz, 2H), 2.75 - 2.65 (m, 1H), 2.51 (tdd, *J* = 6.8, 5.1, 3.2 Hz, 2H), 2.27 (dtd, *J* = 10.8, 6.5, 4.4 Hz, 1H), 2.09 - 1.96 (m, 1H), 1.96 - 1.84 (m, 1H), 1.80 - 1.66 (m, 2H), 1.70 - 1.53 (m, 9H), 1.47 - 1.33 (m, 1H), 1.22 (d, *J* = 6.5 Hz, 3H), 1.17 (d, *J* = 6.5 Hz, 3H).ESI-MS<br>Calculated for $C_{35}H_{46}N_4O_6S_2$ [M+H] $^+$ :682.89, measured : 682.93 |
| 115-017 | 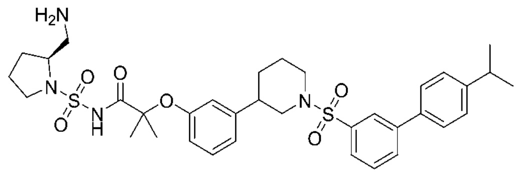 N-(2-(R)-(Aminomethyl)pyrrolidin-1-yl)sulfonyl)-2-(3-(1-((4'-isopropyl-[1,1'-biphenyl]-3-yl)sulfonylpi peridin-3-yl)phenoxy)-2-methylpropionamide 1H NMR(400 MHz, DMSO-d6) δ 7.93 (dd, J = 6.9, 1.5 Hz, 2H), 7.83 - 7.75 (m, 1H), 7.63 - 7.56 (m, 2H), 7.60 - 7.47 (m, 1H), 7.33 (d, J = 8.1 Hz, 2H), 7.25 - 7.17 (m, 1H), 7.00 (s, 1H), 6.96 - 6.88 (m, 1H), 6.79 (t, J = 2.2 Hz, 1H), 6.74 (dt, J = 7.0, 1.6 Hz, 1H), 4.48 (t, J = 6.6 Hz, 2H), 4.19 - 4.09 (m, 1H), 4.01 - 3.91 (m, 1H), 3.40 - 3.31 (m, 2H), 3.20 (dd, J = 12.5, 5.1 Hz, 1H), 2.91 (ddd, J = 12.6, 9.1, 5.9 Hz, 2H), 2.75 - 2.65 (m, 1H), 2.51 (tdd, J = 6.8, 5.1, 3.2 Hz, 2H), 2.27 (dtd, J = 10.8, 6.5, 4.4 Hz, 1H), 2.09 - 1.96 (m, 1H), 1.96 - 1.84 (m, 1H), 1.80 - 1.66 (m, 2H), 1.70 - 1.53 (m, 9H), 1.47 - 1.33 (m, 1H), 1.22 (d, J = 6.5 Hz, 3H), 1.17 (d, J = 6.5 Hz, 3H). ESI-MS<br>Calculated for C35H46N4O6S2[M+H] +:682.89, measured : 682.88 |
| 115-020 | 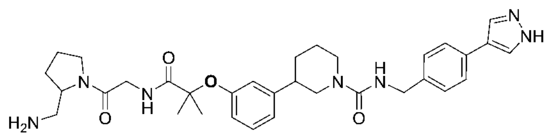 N-(4-(1H-pyrazol-4-yl)benzyl)-3-(3-((1-((2-(2-(aminomethyl)pyrrolidin-1-yl)-2-oxoethyl)amino)-2-me thyl-1-oxopropanoyl)oxy)phenyl)piperidine-1-carboxamide $^1$ H NMR(400 MHz, DMSO- *d* 6)δ 8.73 (d, *J* = 1.8 Hz, 2H), 8.28 (t, *J* = 5.3 Hz, 1H), 7.88 (t, *J* = 6.0 Hz, 1H), 7.67 - 7.59 (m, 2H), 7.30 (dt, *J* = 8.4, 1.2 Hz, 2H), 7.25 - 7.17 (m, 1H), 6.92 (dt, *J* = 7.9, 1.3 Hz, 1H), 6.79 (d, *J* = 1.3 Hz, 1H), 6.74 (dt, *J* = 7.0, 1.2 Hz, 1H), 4.48 (t, *J* = 6.8 Hz, 2H), 4.29 - 4.20 (m, 2H), 4.21 (d, *J* = 0.9 Hz, 1H), 4.13 (dt, *J* = 5.9, 1.0 Hz, 2H), 3.91 (ddd, *J* = 8.5, 4.7, 3.8 Hz, 1H), 3.83 - 3.68 (m, 2H), 3.51 (ddd, *J* = 12.3, 5.1, 3.2 Hz, 1H), 3.33 (dd, *J* = 12.3, 4.7 Hz, 1H), 2.76 (ddd, *J* = 12.4, 6.4, 3.6 Hz, 1H), 2.71 - 2.60 (m, 2H), 2.47 - 2.34 (m, 2H), 2.10 (ddt, *J* = 11.5, 6.4, 4.7 Hz, 1H), 2.01 - 1.70 (m, 4H), 1.74 - 1.62 (m, 1H), 1.62 (s, 3H), 1.57 (s, 3H), 1.62 - 1.48 (m, 1H). ESI-MS<br>Calculated for $C_{33}H_{43}N_7O_4$ [M+H] $^+$ :601.75, measured : 601.23 |

(continued)

| | |
|---|---|
| 115-021 | 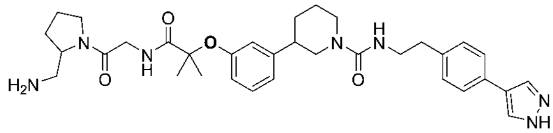<br>N-(4-(1H-pyrazol-4-yl)phenethyl)-3-(3-((1-((2-(2-(aminomethyl)pyrrolidin-1-yl)-2-oxoethyl)amino)-2 -methyl-1-oxopropanoyl)oxy)phenyl)piperidine-1-carboxamide<br>$^1$ H NMR(400 MHz, DMSO- $d_{6)}$δ 8.73 (d, *J* = 1.8 Hz, 2H), 8.28 (t, *J* = 5.3 Hz, 1H), 7.64 - 7.56 (m, 2H), 7.31 (d, *J* = 8.2 Hz, 2H), 7.25 - 7.17 (m, 1H), 6.92 (dd, *J* = 7.9, 1.3 Hz, 1H), 6.79 (d, *J* = 1.3 Hz, 1H), 6.74 (dt, *J* = 7.1, 1.2 Hz, 1H), 6.22 - 6.14 (m, 1H), 4.48 (t, *J* = 6.8 Hz, 2H), 4.29 - 4.20 (m, 2H), 4.21 (d, *J* = 0.9 Hz, 1H), 3.91 (ddd, *J* = 8.5, 4.7, 3.8 Hz, 1H), 3.83 - 3.68 (m, 2H), 3.51 (ddd, *J* = 12.3, 5.1, 3.2 Hz, 1H), 3.33 (dd, *J* = 12.3, 4.7 Hz, 1H), 3.11 (s, 2H), 2.76 (ddd, *J* = 12.5, 6.4, 3.6 Hz, 1H), 2.71 - 2.60 (m, 2H), 2.47 - 2.34 (m, 2H), 2.10 (ddt, *J* = 11.5, 6.5, 4.7 Hz, 1H), 2.01 - 1.82 (m, 2H), 1.78 (dtdd, *J* = 14.0, 6.9, 5.5, 3.5 Hz, 2H), 1.70 - 1.59 (m, 1H), 1.62 (s, 3H), 1.57 (s, 3H), 1.61 - 1.48 (m, 1H).<br>ESI-MS Calculated for$C_{34}H_{45}N_7O_4$ $[M+H]^+$ :615.77, measured: 616.35 |
| 115-022 | 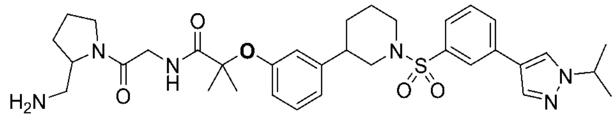<br>N-(2-(2-(Aminomethyl)pyrrolidin-1-yl)-2-oxoethyl)-2-(3-(1-((3-(1-isopropyl-1H-pyrazol-4-yl)phenyl)s ulfonyl)piperidin-3-yl)phenoxy)-2- methylpropanamide<br>$^1$ H NMR(400 MHz, DMSO- $d_{6)}$δ 9.04 (d, *J* = 1.7 Hz, 1H), 8.32 - 8.20 (m, 2H), 7.93 (dd, *J* = 7.0, 1.4 Hz, 2H), 7.79 (dt, *J* = 8.8, 1.5 Hz, 1H), 7.56 - 7.47 (m, 1H), 7.21 (t, *J* = 7.5 Hz, 1H), 6.92 (dd, *J* = 8.0, 1.4 Hz, 1H), 6.79 (d, *J* = 1.3 Hz, 1H), 6.74 (dt, *J* = 7.1, 1.2 Hz, 1H), 4.48 (t, *J* = 6.8 Hz, 2H), 4.34 (p, *J* = 4.4 Hz, 1H), 4.22 (dd, *J* = 5.1, 1.0 Hz, 2H), 3.91 (ddd, *J* = 8.5, 4.7, 3.8 Hz, 1H), 3.73 (ddd, *J* = 12.6, 5.1, 3.4 Hz, 1H), 3.51 (ddd, *J* = 12.3, 5.1, 3.2 Hz, 1H), 3.40 - 3.31 (m, 2H), 3.20 (dd, *J* = 12.5, 5.1 Hz, 1H), 2.90 (dd, *J* = 12.5, 5.1 Hz, 1H), 2.65 (dtd, *J* = 10.6, 6.8, 3.8 Hz, 1H), 2.50 (q, *J* = 5.4 Hz, 1H), 2.41 (dddd, *J* = 11.7, 10.2, 4.2, 3.2 Hz, 2H), 2.10 (ddt, *J* = 11.5, 6.4, 4.7 Hz, 1H), 1.96 - 1.84 (m, 2H), 1.76 (dddd, *J* = 13.1, 8.2, 4.2, 2.4 Hz, 1H), 1.67 - 1.53 (m, 8H), 1.46 - 1.33 (m, 1H), 1.32 (d, *J* = 4.4 Hz, 3H), 1.27 (d, *J* = 4.4 Hz, 3H).<br>ESI-MS Calculated for$C_{34}H_{46}N_6O_5S$ $[M+H]^+$ :650.84, measured: 650.65 |
| 115-023 | 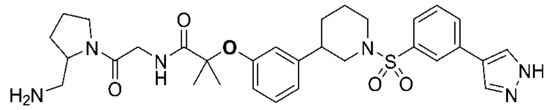<br>2-(3-(1-((3-(1H-pyrazol-4-yl)phenyl)sulfonyl)piperidin-3-yl)phenoxy)-N-(2-(2-(aminomethyl)pyrrolidi n-1-yl)-2-oxoethyl)-2-methylpropanamide<br>1H NMR(400 MHz, DMSO-d6) δ 8.73 (d, J = 1.8 Hz, 2H), 8.28 (t, J = 5.3 Hz, 1H), 7.93 (dd, J = 7.0, 1.4 Hz, 2H), 7.79 (dt, J = 8.8, 1.5 Hz, 1H), 7.56 - 7.47 (m, 1H), 7.21 (t, J = 7.5 Hz, 1H), 6.92 (dt, J = 8.0, 1.4 Hz, 1H), 6.79 (d, J = 1.3 Hz, 1H), 6.74 (dt, J = 7.1, 1.2 Hz, 1H), 4.48 (t, J = 6.8 Hz, 2H), 4.22 (dd, J = 5.1, 1.0 Hz, 2H), 3.91 (ddd, J = 8.5, 4.7, 3.8 Hz, 1H), 3.73 (ddd, J = 12.5, 5.1, 3.4 Hz, 1H), 3.51 (ddd, J = 12.3, 5.1, 3.2 Hz, 1H), 3.40 - 3.31 (m, 2H), 3.20 (dd, J = 12.5, 5.1 Hz, 1H), 2.90 (dd, J = 12.5, 5.1 Hz, 1H), 2.65 (dtd, J = 10.6, 6.8, 3.8 Hz, 1H), 2.50 (q, J = 5.4 Hz, 1H), 2.46 - 2.34 (m, 2H), 2.10 (ddt, J = 11.5, 6.4, 4.7 Hz, 1H), 1.98 - 1.84 (m, 2H), 1.81 - 1.69 (m, 1H), 1.67 - 1.53 (m, 8H), 1.46 - 1.33 (m, 1H). ESI-MS<br>Calculated for C31H40N6O5S[M+H] +:609.28, measured : 609.25 |

(continued)

| | |
|---|---|
| 115-024 | 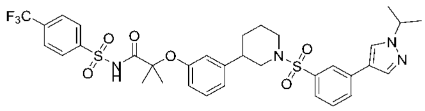 2-(3-(1-((3-(1-isopropyl-1H-pyrazol-4-yl)phenyl)sulfonyl)piperidin-3-yl)phenoxy)-2-methyl-N-((4-(trifluoromethyl)phenyl)sulfonyl)propionamide<br>1H NMR(400 MHz, DMSO-d6) δ 8.73 (d, J = 1.8 Hz, 2H), 7.93 (dd, J = 6.9, 1.5 Hz, 2H), 7.83 - 7.76 (m, 1H), 7.76 - 7.69 (m, 2H), 7.57 - 7.47 (m, 1H), 7.39 - 7.31 (m, 2H), 7.25 - 7.17 (m, 1H), 6.96 - 6.88 (m, 1H), 6.79 (t, J = 2.2 Hz, 1H), 6.74 (dt, J = 7.0, 1.6 Hz, 1H), 3.40 - 3.31 (m, 2H), 3.20 (dd, J = 12.5, 5.1 Hz, 1H), 2.90 (dd, J = 12.5, 5.1 Hz, 1H), 2.51 (p, J = 5.4 Hz, 1H), 1.96 - 1.84 (m, 1H), 1.67 - 1.53 (m, 8H), 1.47 - 1.33 (m, 1H). ESI-MS<br>Calculated for C34H37F3N4O6S2[M+H] +:719.21 measured : 719.16 |
| 115-025 | 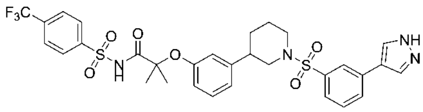 2-(3-(1-((3-(1H-pyrazol-4-yl)phenyl)sulfonyl)piperidin-3-yl)phenoxy)-2-methyl-N-((4-(trifluoromethy l)phenyl)sulfonyl)propionamide<br>1H NMR(400 MHz, DMSO-d6) δ 8.73 (d, J = 1.8 Hz, 2H), 7.93 (dd, J = 6.9, 1.5 Hz, 2H), 7.83 - 7.76 (m, 1H), 7.76 - 7.69 (m, 2H), 7.57 - 7.47 (m, 1H), 7.39 - 7.31 (m, 2H), 7.25 - 7.17 (m, 1H), 6.96 - 6.88 (m, 1H), 6.79 (t, J = 2.2 Hz, 1H), 6.74 (dt, J = 7.0, 1.6 Hz, 1H), 3.40 - 3.31 (m, 2H), 3.20 (dd, J = 12.5, 5.1 Hz, 1H), 2.90 (dd, J = 12.5, 5.1 Hz, 1H), 2.51 (p, J = 5.4 Hz, 1H), 1.96 - 1.84 (m, 1H), 1.67 - 1.53 (m, 8H), 1.47 - 1.33 (m, 1H).<br>ESI-MS Calculated forC31H31F3N4O6S2[M+H] +:677.18, measured: 677.15 |
| 115-026 | 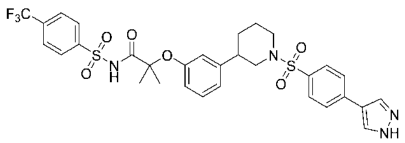 2-(3-(1-((4-(1H-pyrazol-4-yl)phenyl)sulfonyl)piperidin-3-yl)phenoxy)-2-methyl-N-((4-(trifluoromethy l)phenyl)sulfonyl)propionamide<br>1H NMR(400 MHz, DMSO-d6) δ 8.73 (d, J = 1.8 Hz, 2H), 7.92 - 7.84 (m, 2H), 7.77 - 7.69 (m, 2H), 7.69 - 7.61 (m, 2H), 7.39 - 7.31 (m, 2H), 7.25 - 7.17 (m, 1H), 6.96 - 6.88 (m, 1H), 6.79 (t, J = 2.2 Hz, 1H), 6.74 (dt, J = 7.0, 1.6 Hz, 1H), 3.40 - 3.31 (m, 2H), 3.20 (dd, J = 12.5, 5.1 Hz, 1H), 2.90 (dd, J = 12.5, 5.1 Hz, 1H), 2.51 (p, J = 5.4 Hz, 1H), 1.96 - 1.84 (m, 1H), 1.67 - 1.53 (m, 8H), 1.47 - 1.33 (m, 1H).<br>ESI-MS Calculated forC31H31F3N4O6S2[M+H] +:677.18, measured: 677.34 |
| 115-027 | 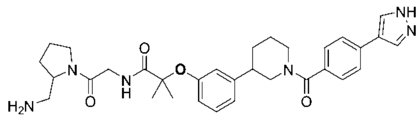 2-(3-(1-(4-(1H-pyrazol-4-yl)benzoyl)piperidin-3-yl)phenoxy)-N-(2-(2-(aminomethyl)pyrrolidin-1-yl)-2 -oxoethyl)-2-methylpropanamide<br>1H NMR(400 MHz, DMSO-d6) δ 8.73 (d, J = 1.8 Hz, 2H), 8.28 (t, J = 5.3 Hz, 1H), 8.09 - 8.01 (m, 2H), 7.93 - 7.85 (m, 2H), 7.21 (t, J = 7.5 Hz, 1H), 6.92 (dt, J = 8.1, 1.4 Hz, 1H), 6.79 (d, J = 1.3 Hz, 1H), 6.74 (dt, J = 7.0, 1.2 Hz, 1H), 4.48 (t, J = 6.8 Hz, 2H), 4.29 - 4.20 (m, 2H), 4.21 (d, J = 0.9 Hz, 1H), 3.91 (ddd, J = 8.5, 4.7, 3.8 Hz, 1H), 3.73 (ddd, J = 12.5, 5.1, 3.4 Hz, 1H), 3.51 (ddd, J = 12.3, 5.1, 3.2 Hz, 1H), 3.32 (ddd, J = 12.5, 6.1, 4.0 Hz, 2H), 3.22 (ddd, J = 12.5, 6.1, 3.5 Hz, 1H), 2.65 (ddt, J = 9.9, 7.1, 3.6 Hz, 2H), 2.47 - 2.34 (m, 2H), 2.10 (ddt, J = 11.5, 6.4, 4.7 Hz, 1H), 2.01 - 1.82 (m, 2H), 1.86 - 1.70 (m, 2H), 1.70 - 1.59 (m, 1H), 1.62 (s, 3H), 1.57 (s, 3H), 1.61 - 1.48 (m, 1H). ESI-MS<br>Calculated for C32H40N6O4[M+H] +:573.71, measured: 573.57 |

(continued)

| | |
|---|---|
| 115-028 | 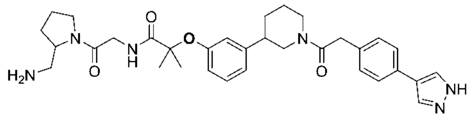<br>2-(3-(1-(2-(4-(1H-pyrazol-4-yl)phenyl)acetyl)piperidin-3-yl)phenoxy)-N-(2-(2-(aminomethyl)pyrrolidin-1-yl)-2-oxoethyl)-2-methylpropanamide<br>1H NMR(400 MHz, DMSO-d6) δ 8.73 (d, J = 1.8 Hz, 2H), 8.28 (t, J = 5.3 Hz, 1H), 7.64 - 7.56 (m, 2H), 7.33 (dt, J = 8.4, 1.2 Hz, 2H), 7.21 (t, J = 7.5 Hz, 1H), 6.92 (dt, J = 7.9, 1.3 Hz, 1H), 6.79 (d, J = 1.2 Hz, 1H), 6.74 (dt, J = 7.0, 1.2 Hz, 1H), 4.48 (t, J = 6.8 Hz, 2H), 4.25 - 4.15 (m, 3H), 3.91 (ddd, J = 8.5, 4.7, 3.8 Hz, 1H), 3.83 - 3.67 (m, 3H), 3.56 - 3.46 (m, 2H), 2.76 (ddd, J = 12.3, 6.3, 3.5 Hz, 1H), 2.71 - 2.60 (m, 2H), 2.47 - 2.34 (m, 2H), 2.10 (ddt, J = 11.5, 6.4, 4.7 Hz, 1H), 2.01 - 1.85 (m, 2H), 1.89 - 1.76 (m, 1H), 1.80 - 1.72 (m, 1H), 1.76 - 1.48 (m, 9H).<br>ESI-MS Calculated forC33H42N6O4[M+H] +:587.33, measured: 587.27 |
| 115-029 | 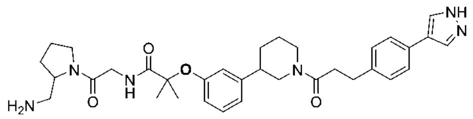<br>2-(3-(1-(3-(4-(1H-pyrazol-4-yl)phenyl)propionyl)piperidin-3-yl)phenoxy)-N-(2-(2-(aminomethyl)pyrrolidin-1-yl)-2-oxoethyl)-2-methylpropionamide<br>1H NMR(400 MHz, DMSO-d6) δ 8.73 (d, J = 1.8 Hz, 2H), 8.28 (t, J = 5.3 Hz, 1H), 7.64 - 7.56 (m, 2H), 7.37 - 7.30 (m, 2H), 7.21 (t, J = 7.5 Hz, 1H), 6.92 (dd, J = 7.9, 1.3 Hz, 1H), 6.79 (d, J = 1.3 Hz, 1H), 6.74 (dt, J = 7.0, 1.2 Hz, 1H), 4.48 (t, J = 6.8 Hz, 2H), 4.25 - 4.15 (m, 3H), 3.91 (ddd, J = 8.5, 4.7, 3.8 Hz, 1H), 3.83 - 3.68 (m, 2H), 3.56 - 3.46 (m, 2H), 2.89 (dd, J = 8.4, 7.2 Hz, 2H), 2.76 (ddd, J = 12.3, 6.3, 3.5 Hz, 1H), 2.71 - 2.60 (m, 4H), 2.47 - 2.34 (m, 2H), 2.10 (ddt, J = 11.5, 6.5, 4.7 Hz, 1H), 2.01 - 1.85 (m, 2H), 1.89 - 1.76 (m, 1H), 1.80 - 1.72 (m, 1H), 1.76 - 1.48 (m, 9H).<br>ESI-MS Calculated forC34H44N6O4[M+H] +:601.35, measured: 601.39 |
| 115-030 | 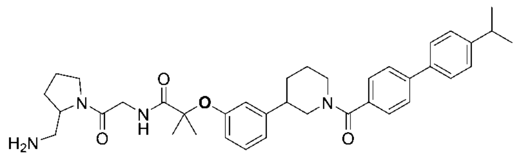<br>2-(3-(1-(4-(4'-isopropyl-[1,1'-biphenyl-4-yl)benzoyl)piperidin-3-yl)phenoxy)-N-(2-(2-(aminomethyl)p yrrolidin-1-yl)-2-oxoethyl)-2-methylpropanamide<br>$^{1}$H NMR(400 MHz, DMSO- $d_{6)}$δ 8.28 (t, *J* = 5.3 Hz, 1H), 8.09 - 8.01 (m, 2H), 7.87 - 7.79 (m, 2H), 7.63 - 7.55 (m, 2H), 7.33 (d, *J* = 8.1 Hz, 2H), 7.25 - 7.17 (m, 1H), 6.92 (dd, *J* = 8.0, 1.4 Hz, 1H), 6.79 (d, *J* = 1.3 Hz, 1H), 6.74 (dt, *J* = 7.1, 1.2 Hz, 1H), 4.48 (t, *J* = 6.8 Hz, 2H), 4.29 - 4.20 (m, 2H), 4.21 (d, *J* = 0.9 Hz, 1H), 3.91 (ddd, *J* = 8.5, 4.7, 3.8 Hz, 1H), 3.73 (ddd, *J* = 12.6, 5.1, 3.4 Hz, 1H), 3.51 (ddd, *J* = 12.3, 5.1, 3.2 Hz, 1H), 3.32 (ddd, *J* = 12.5, 6.1, 4.0 Hz, 2H), 3.22 (ddd, *J* = 12.6, 6.1, 3.5 Hz, 1H), 2.93 (hept, *J* = 6.3 Hz, 1H), 2.66 (ddd, *J* = 10.9, 6.5, 3.9 Hz, 2H), 2.41 (dddd, J = 11.8, 10.2, 4.2, 3.2 Hz, 2H), 2.10 (ddt, *J* = 11.6, 6.4, 4.7 Hz, 1H), 2.01 - 1.82 (m, 2H), 1.86 - 1.75 (m, 1H), 1.80 - 1.67 (m, 1H), 1.70 - 1.59 (m, 1H), 1.62 (s, 3H), 1.57 (s, 3H), 1.61 - 1.48 (m, 1H), 1.22 (d, *J* = 6.5 Hz, 3H), 1.17 (d, *J* = 6.5 Hz, 3H).<br>ESI-MS<br>Calculated for $C_{38}H_{48}N_{4}O_{4}$ $[M+H]^{+}$ :625.83, measured : 625.89 |

(continued)

| | |
|---|---|
| 115-031 | 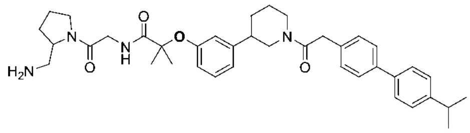<br>**N-**(2-(2-(Aminomethyl)pyrrolidin-1-yl)-2-oxoethyl)-2-(3-(1-(2-(4'-isopropyl-[1,1'-biphenyl]-4-yl)acetyl) piperidin-3-yl)phenoxy)-2-methylpropanamide<br>1H NMR(400 MHz, DMSO-d6) δ 8.28 (t, J = 5.3 Hz, 1H), 7.63 - 7.55 (m, 4H), 7.33 (dd, J = 8.4, 1.7 Hz, 4H), 7.25 - 7.17 (m, 1H), 6.92 (dd, J = 7.9, 1.3 Hz, 1H), 6.79 (d, J = 1.3 Hz, 1H), 6.74 (dt, J = 7.1, 1.2 Hz, 1H), 4.48 (t, J = 6.8 Hz, 2H), 4.25 - 4.15 (m, 3H), 3.91 (ddd, J = 8.5, 4.7, 3.8 Hz, 1H), 3.83 - 3.67 (m, 3H), 3.56 - 3.46 (m, 2H), 2.93 (p, J = 6.8 Hz, 1H), 2.76 (ddd, J = 12.3, 6.3, 3.5 Hz, 1H), 2.71 - 2.60 (m, 2H), 2.47 - 2.34 (m, 2H), 2.10 (ddt, J = 11.6, 6.5, 4.7 Hz, 1H), 2.01 - 1.85 (m, 2H), 1.89 - 1.76 (m, 1H), 1.77 (ddd, J = 8.9, 4.2, 2.3 Hz, 1H), 1.76 - 1.48 (m, 9H), 1.22 (d, J = 6.5 Hz, 3H), 1.17 (d, J = 6.5 Hz, 3H).<br>ESI-MS Calculated forC39H50N404[M+H] +:639.38, measured: 639.49 |
| 115-047 | 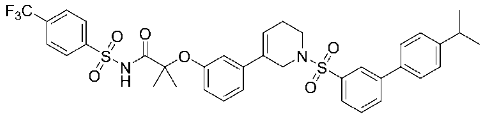<br>2-(3-(1-((4'-isopropyl-[1,1'-biphenyl]-3-yl)sulfonyl)-1,2,5,6-tetrahydropyridin-3-yl)phenoxy)-2-meth yl-N-((4-(trifluoromethyl)phenyl)sulfonyl)propanamide $^{1}$ H NMR(400 MHz, DMSO- *d* $_{6)}$δ 7.93 (dd, *J* = 6.9, 1.5 Hz, 2H), 7.83 - 7.76 (m, 1H), 7.76 - 7.69 (m, 2H), 7.63 - 7.56 (m, 2H), 7.56 - 7.48 (m, 1H), 7.45 (t, *J* = 7.7 Hz, 1H), 7.39 - 7.29 (m, 4H), 7.06 (t, *J* = 1.8 Hz, 1H), 6.81 (dt, *J* = 7.4, 1.4 Hz, 1H), 6.73 (dt, *J* = 7.9, 1.2 Hz, 1H), 6.08 (td, *J* = 4.1, 1.1 Hz, 1H), 3.33 (s, 1H), 3.00 (t, *J* = 5.2 Hz, 2H), 2.93 (p, *J* = 6.8 Hz, 1H), 2.30 (dtd, *J* = 6.5, 5.4, 4.9, 1.3 Hz, 2H), 1.60 (s, 5H), 1.20 (d, *J* = 6.5 Hz, 6H). ESI-MS Calculated for $C_{37}H_{37}F_{3}N_{2}O_{6}S_{2}$ [M+H] $^{+}$ :727.75, measured : 727.53 |
| 115-049 | 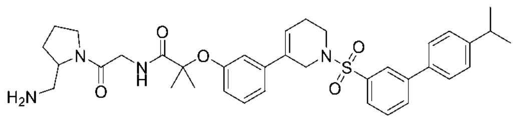<br>N-(2-(2-(Aminomethyl)pyrrolidin-1-yl)-2-oxoethyl)-2-(3-(1-((4'-isopropyl-[1,1'-biphenyl]-3-yl)sulfon yl)-1,2,5,6-tetrahydropyridin-3-yl)phenoxy)-2-methylpropanamide<br>1H NMR(400 MHz, DMSO-d6) δ 8.28 (t, J = 5.3 Hz, 1H), 7.93 (dd, J = 6.9, 1.5 Hz, 2H), 7.83 - 7.75 (m, 1H), 7.63 - 7.56 (m, 2H), 7.56 - 7.48 (m, 1H), 7.45 (t, J = 7.7 Hz, 1H), 7.33 (d, J = 8.1 Hz, 2H), 7.06 (t, J = 1.8 Hz, 1H), 6.81 (dt, J = 7.4, 1.5 Hz, 1H), 6.73 (dt, J = 7.9, 1.2 Hz, 1H), 6.08 (td, J = 4.1, 1.1 Hz, 1H), 4.48 (t, J = 6.8 Hz, 2H), 4.22 (dd, J = 5.1, 1.0 Hz, 2H), 3.91 (ddd, J = 8.5, 4.7, 3.8 Hz, 1H), 3.73 (ddd, J = 12.6, 5.1, 3.4 Hz, 1H), 3.51 (ddd, J = 12.3, 5.1, 3.2 Hz, 1H), 3.00 (t, J = 5.2 Hz, 2H), 2.93 (p, J = 6.8 Hz, 1H), 2.65 (dtd, J = 10.6, 6.8, 3.8 Hz, 1H), 2.46 - 2.34 (m, 2H), 2.30 (dtd, J = 6.5, 5.4, 4.9, 1.3 Hz, 2H), 2.10 (ddt, J = 11.6, 6.4, 4.7 Hz, 1H), 1.96 - 1.84 (m, 1H), 1.76 (dddt, J = 11.8, 10.0, 8.2, 4.0 Hz, 1H), 1.62 (s, 3H), 1.57 (s, 3H), 1.22 (d, J = 6.5 Hz, 3H), 1.17 (d, J = 6.5 Hz, 3H). ESI-MS<br>Calculated for C37H46N4O5S[M+H] +:659.32, measured : 659.37 |

(continued)

| | |
|---|---|
| 115-052 | 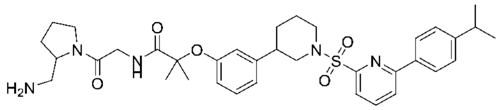<br>N-(2-(2-(aminomethyl)pyrrolidin-1-yl)-2-oxoethyl)-2-(3-(1-((6-(4-isopropylphenyl)pyridin-2-yl)sulfo nyl) piperidin-3-yl)phenoxy)-2-methylpropanamide<br>1H NMR(400 MHz, DMSO-d6) δ 8.28 (t, J = 5.3 Hz, 1H), 8.17 - 8.09 (m, 2H), 7.88 - 7.79 (m, 2H), 7.66 - 7.57 (m, 1H), 7.37 - 7.29 (m, 2H), 7.25 - 7.17 (m, 1H), 6.96 - 6.88 (m, 1H), 6.79 (t, J = 2.2 Hz, 1H), 6.74 (dt, J = 7.0, 1.6 Hz, 1H), 4.48 (t, J = 6.8 Hz, 2H), 4.22 (dd, J = 5.1, 1.0 Hz, 2H), 3.91 (ddd, J = 8.5, 4.7, 3.8 Hz, 1H), 3.73 (ddd, J = 12.6, 5.1, 3.4 Hz, 1H), 3.51 (ddd, J = 12.3, 5.1, 3.2 Hz, 1H), 3.40 - 3.31 (m, 2H), 3.20 (dd, J = 12.5, 5.1 Hz, 1H), 2.91 (ddd, J = 12.6, 9.1, 5.9 Hz, 2H), 2.65 (dtd, J = 10.6, 6.8, 3.8 Hz, 1H), 2.50 (q, J = 5.4 Hz, 1H), 2.46 - 2.34 (m, 2H), 2.10 (ddt, J = 11.5, 6.4, 4.7 Hz, 1H), 1.96 - 1.82 (m, 2H), 1.82 - 1.69 (m, 1H), 1.67 - 1.53 (m, 8H), 1.47 - 1.33 (m, 1H), 1.22 (d, J = 6.5 Hz, 3H), 1.17 (d, J = 6.5 Hz, 3H). ESI-MS<br>Calculated for C36H47N5O5S[M+H] +:662.33, measured : 662.39 |
| 115-055 | 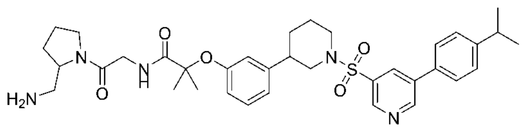<br>**N-**(2-(2-(Aminomethyl)pyrrolidin-1-yl)-2-oxoethyl)-2-(3-(1-((5-(4-isopropylphenyl)pyridin-3-yl)sulfonyl ) piperidin-3-yl)phenoxy)-2-methylpropanamide<br>1H NMR(400 MHz, DMSO-d6) δ 8.28 (t, J = 5.3 Hz, 1H), 8.17 - 8.09 (m, 2H), 7.88 - 7.79 (m, 2H), 7.66 - 7.57 (m, 1H), 7.37 - 7.29 (m, 2H), 7.25 - 7.17 (m, 1H), 6.96 - 6.88 (m, 1H), 6.79 (t, J = 2.2 Hz, 1H), 6.74 (dt, J = 7.0, 1.6 Hz, 1H), 4.48 (t, J = 6.8 Hz, 2H), 4.22 (dd, J = 5.1, 1.0 Hz, 2H), 3.91 (ddd, J = 8.5, 4.7, 3.8 Hz, 1H), 3.73 (ddd, J = 12.6, 5.1, 3.4 Hz, 1H), 3.51 (ddd, J = 12.3, 5.1, 3.2 Hz, 1H), 3.40 - 3.31 (m, 2H), 3.20 (dd, J = 12.5, 5.1 Hz, 1H), 2.91 (ddd, J = 12.6, 9.1, 5.9 Hz, 2H), 2.65 (dtd, J = 10.6, 6.8, 3.8 Hz, 1H), 2.50 (q, J = 5.4 Hz, 1H), 2.46 - 2.34 (m, 2H), 2.10 (ddt, J = 11.5, 6.4, 4.7 Hz, 1H), 1.96 - 1.82 (m, 2H), 1.82 - 1.69 (m, 1H), 1.67 - 1.53 (m, 8H), 1.47 - 1.33 (m, 1H), 1.22 (d, J = 6.5 Hz, 3H), 1.17 (d, J = 6.5 Hz, 3H). ESI-MS<br>Calculated for C36H47N5O5S[M+H] +:662.33, measured: 662.45 |

## Test Example

### I. Test Method

#### 1) CCK8

1. SW480 cell plating

Cells were digested, counted, and plated in a 96-well plate (flat-bottomed, transparent) with 10,000 cells/100 μl DMEM (10% FBS) per well .

2. Observe the cell status on the next day and start adding drugs after the cells have fully adhered to the wall;
3. Dilute the compound in DMEM (2% FBS) in a serial dilution format to concentrations of 20 μM, 10 μM, 5 μM, 2.5 μM, 1.25 μM, 0.625 μM, 0.3125 μM, 0.15625 μM, and 0 μM (with equal volume of DMSO).
4. Add 100 μl of the above compounds at different concentrations to each well. Repeat for each concentration gradient in duplicate. Leave three blank wells (add only DMEM 2% FBS culture medium without cells) .
5. Incubate at 37°C for 24 hours .
6. Add 10 μl of CCK-8 Enhanced Solution to each well. Since the amount of CCK-8 added to each well is relatively small, there may be errors due to the reagent sticking to the well wall. It is recommended to gently tap the culture plate after adding the reagent to help mix it.
7. Incubate in the incubator for 0.5-4 hours: The amount of formazan formed varies depending on the cell type. In most cases, incubation for 1 hour is sufficient. If color development is insufficient, continue incubation to confirm optimal conditions.
8. Measure the absorbance at 450nm and 600nm (excluding interference from the reagent background color and the

absorbance of the well plate itself);
9. The final absorbance value is OD450nm-OD600nm, and the inhibition rate is calculated;

$$\text{Inhibition rate} = [(Ac - As) / (Ac - Ab)] \times 100\%$$

As: absorbance of the experimental well (culture medium containing cells, CCK-8, and drug to be tested);
Ac: absorbance of control wells (culture medium containing cells, CCK-8, and no test drug);
Ab: absorbance of blank wells (culture medium without cells, test drugs, or CCK-8).

(2) SRP test

1. Experimental Setup

1. The analyte is in powder form.
2. The temperature is 25°C.
3. The analytical equipment used was Biacore T200 instrument.

2. Sample Dilution

1. Ligand β-catenin was diluted with HEPES (pH 7.4) to 0.5 mg/mL
2. The analyte was dissolved in DMSO and diluted with 0.1% DMSO HEPES (pH 7.4)

3. Experimental Procedure

1. Start the Biacore T200 instrument according to standard operating procedures.
2. Prepare 500 ml of HEPES buffer (pH 7.4) and deionized water (filtered through a 0.22 μm membrane) for rinsing the injection needle.
3. Start installing the chip and install the CM5 chip according to the standard process.
4. Prepare to start the formal experiment. The buffer solution will flush the flow system inside the entire system at a high flow rate.
5. Choose the appropriate procedure based on the sample size.
6. Start the capture chip. Prepare a sufficient volume of ligand β-catenin, EDC/NHS, and blocking buffer. Initiate the coupling procedure with a 7-minute coupling time and a flow rate of 10 μl/min. The final ligand coupling volume is approximately 16,000 RU.
7. After the coupling is completed, start the sample detection and set the analyte binding time to 120 s.

The flow rate was 30 μL/min; the dissociation time was 200 s, the flow rate was 30 μL/min; the regeneration time was 30 s,
The flow rate was 30 μL/min.

8. Prepare the corresponding samples to be tested as required and start the automatic running program for testing.
9. Result analysis: Based on the running results, perform data fitting analysis to obtain the final affinity fitting KD value.

(3) Test results

The results of tests are shown in Table 1

**Table 1**

| Compound No. | Structure | SW480 IC50(μM) | Kd(nM) |
|---|---|---|---|
| 115-010 | 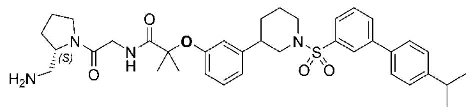 | 3.5599μM | 284nM |
| 115-011 | 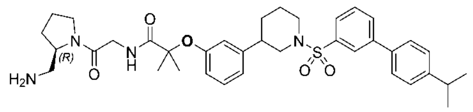 | 6.25μM~3.125μM | / |
| 115-012 | 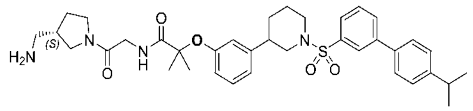 | <2.972μM | 760nM |
| 115-013 | 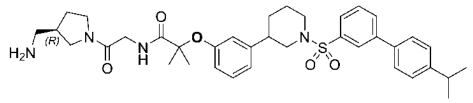 | 3.124μM | |
| 115-014 | 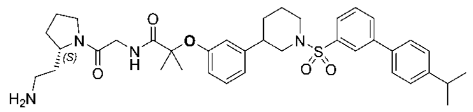 | 52.88μM | |
| 115-017 | 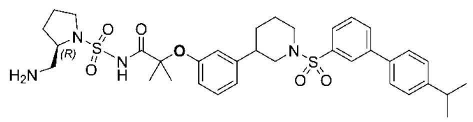 | 10.61μM | |
| | | | |
| 115-020 | 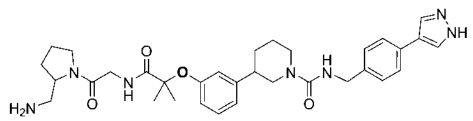 | Inactive | |
| 115-021 | 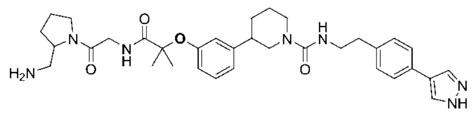 | Inactive | |
| 115-027 | 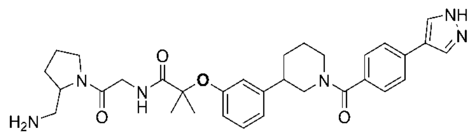 | 23.07μM | |
| 115-028 | 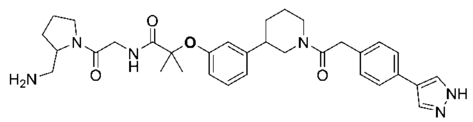 | 15.73μM | |
| 115-029 | 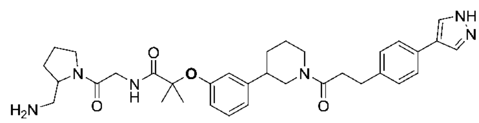 | 20.84μM | |
| 115-030 | 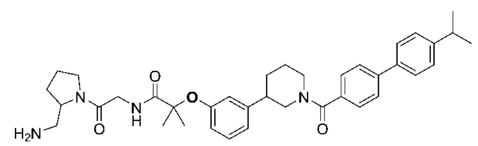 | 6.25μM~3.125μM | |

(continued)

| Compound No. | Structure | SW480 IC50(μM) | Kd(nM) |
|---|---|---|---|
| 115-031 | 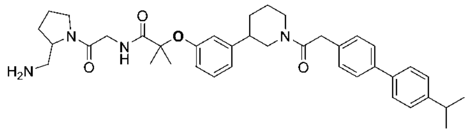 | 6.994μM | |
| 115-022 | 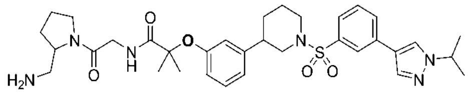 | 9.708μM | |
| 115-023 | 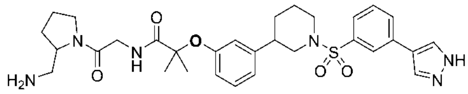 | 30.54μM | |
| | | | |
| 115-049 | 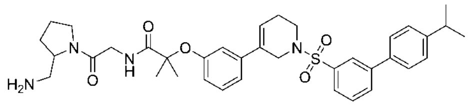 | ≤2.646μM | |
| | | | |
| 115-025 | 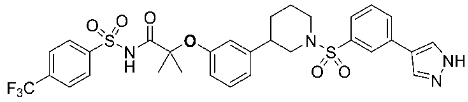 | Inactive | |
| 115-024 | 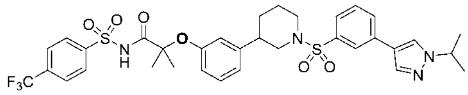 | 11.1μM | |
| 115-026 | 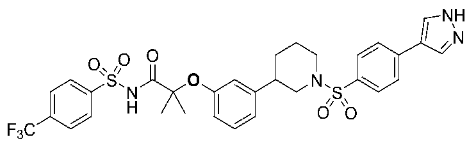 | Inactive | |
| 115-052 | 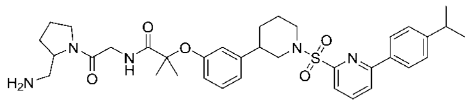 | ≤2.742μM | |
| 115-055 | 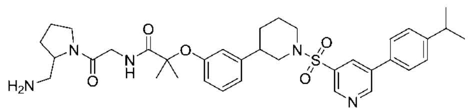 | 4.060μM | |
| | | | |
| 115 | 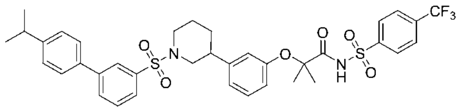 | 2.812μM | |

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound or a pharmaceutically acceptable salt thereof, or an isomer, solvate, crystal form or prodrug thereof, wherein the compound is represented by Formula I

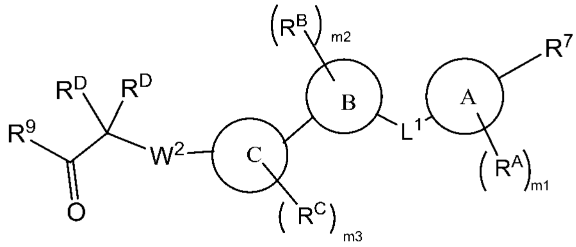

(I)

wherein

$R^9$ is

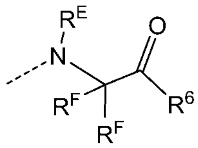

,

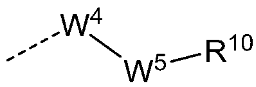

;

$W^4$ and $W^5$ are each independently selected from the group consisting of none, -O-, -S-, -C(O)-, -S(O)-, $-S(O)_2-$, $-N(R^{s1})-$, $-C(R^{s2})_2-$;

$R^{10}$ is H, OH, $R^6$, or is unsubstituted or substituted with one or more $R^H$ and is selected from the group consisting of $C_{1-6}$ alkyl, C3-10 cycloalkyl, 4- to 10-membered heterocycloalkyl, C3-10 cycloalkenyl, 4- to 10-membered heterocycloalkenyl, $C_{6-10}$ aryl, and 5- to 10-membered heteroaryl;

$R^H$ is selected from the group consisting of R, optionally substituted $C_{1-6}$haloalkyl ;

$R^E$ is selected from the group consisting of H, optionally substituted $C_{1-4}$alkyl, optionally substituted C3-10 cycloalkyl, and optionally substituted 4- to 10-membered heterocycloalkyl;

$R^F$ are each independently selected from the group consisting of H, optionally substituted $C_{1-4}$ alkyl , optionally substituted $_C$3-10 cycloalkyl, optionally substituted 4 to 10 membered heterocycloalkyl; or two $R^F$ and the carbon atom to which they are attached together form an optionally substituted C3-6 cycloalkyl or an optionally substituted 4 to 6 membered heterocyclyl;

$R^6$ is an optionally substituted group selected from the group consisting of $-OR^2$, $C_{3-12}$ cycloalkyl, a 4- to 10-membered heterocycloalkyl group connected to the rest of the group via a carbon atom on the ring, and $-NR^4R^5$ ;

$R^2$ is selected from the group consisting of H, optionally substituted $C_{1-6}$alkyl, optionally substituted C3-10 cycloalkyl, optionally substituted 4 to 8-membered heterocycloalkyl, optionally substituted $C_{6-10}$ aryl, optionally substituted 5 to 10-membered heteroaryl, optionally substituted C3-10 cycloalkenyl, optionally substituted 4 to 10-membered heterocycloalkenyl;

R4 and R5 are each independently H, $_{C1-6}$ alkyl, C3-10 cycloalkyl, 4- to 8- membered heterocycloalkyl, $C_{6-10}$ aryl, 5- to 10-membered heteroaryl, C3-10 cycloalkenyl, or 4- to 10-membered heterocycloalkenyl, which is optionally substituted or substituted by one or more (e.g., 1, 2, or 3) R3; or, R4 and R5, together with the nitrogen atom to which they are attached, form a ring optionally substituted or substituted by one or more (e.g., 1, 2, or 3) R3, which is selected from the group consisting of 4- to 10-membered heterocycloalkyl 、4- $^{to}$ 10 - membered heterocycloalkenyl, or 5- to 10-membered heteroaryl.

$R^3$ is each independently selected from the group consisting of R, $-OR^{31}$ , $-C_{1-4}$alkylene-$OR^{31}$ , $-N(R^{32})R^{33}$ , $-C_{1-4}$alkylene-$N(R^{31})R^{32}$ ;

$R^{31}$ is selected from the group consisting of H, optionally substituted $C_{1-4}$alkyl, $R^{34}$, $-C_{1-4}$alkylene-$R^{34}$ ;

$R^{32}$ is selected from the group consisting of H, optionally substituted $C_{1-4}$alkyl;

$R^{33}$ is selected from the group consisting of H, optionally substituted $C_{1-4}$alkyl, $R^{34}$, $-C_{1-4}$alkylene-$R^{34}$ ;

$R^{34}$ is selected from the group consisting of C3-10 cycloalkyl, 4- to 8-membered heterocycloalkyl, $C_{6-10}$ aryl, 5- to 10-membered heteroaryl, C3-10 cycloalkenyl, and 4- to 10-membered heterocycloalkenyl; wherein the cycloalkyl, heterocycloalkyl, aryl, heteroaryl, cycloalkenyl, and heterocycloalkenyl groups are optionally substituted with one or more groups selected from the group consisting of $-NH_2$, R;

$R^D$ are each independently selected from the group consisting of H, optionally substituted $C_{1-4}$alkyl; or, two $R^D{}_S$ and the carbon atom to which they are attached together form a group selected from the group consisting of optionally substituted C3-10 cycloalkyl, optionally substituted 4 to 10 membered heterocycloalkyl;
W2 is selected from the group consisting of -O-, -S-, and -N($R^{s1}$)-;
Ring C is an optionally substituted ring selected from the group consisting of $C_{6-10}$ aryl, and 5- to 10-membered heteroaryl;
m3=0, 1, 2, 3, or 4;
Each RC is independently $R^{C1}$ or $R^{s1}$ ;
Each $R^{C1}$ is independently selected from the group consisting of halogen, optionally substituted $C_{1-6}$alkyl, optionally substituted $C_{1-6}$haloalkyl, hydroxy, and optionally substituted $C_{1-6}$alkoxy, optionally substituted $C_{1-6}$haloalkoxy;
Ring B is an optionally substituted ring selected from the group consisting of $C_{3-12}$ cycloalkyl, 4-to 12-membered heterocycloalkyl, C3-10 cycloalkenyl, and 4- to 10-membered heterocycloalkyl;
Each RB is independently $R^{B1}$ , $R^{s1}$ or $R^{s2}$ ;
each $R^{B1}$ is independently selected from the group consisting of halogen, hydroxy, cyano,
optionally substituted $C_{1-6}$alkyl, optionally substituted $C_{1-6}$alkoxy, optionally substituted $C_{1-6}$alkylthio, optionally substituted C3-10 cycloalkyl, optionally substituted 4- to 10-membered heterocycloalkyl, optionally substituted C3-10 cycloalkenyl, optionally substituted 4- to 10-membered heterocycloalkenyl, optionally substituted $C_{6-10}$ aryl, and optionally substituted 5- to 10-membered heteroaryl;
m2=0, 1, 2, 3, or 4;
$L^1$ is -X-$(W^1)_{n1}$ -;
X is selected from the group consisting of -C(O)-, -S(O)-, and -$S(O)_2$-;
each $W^1$ is independently selected from the group consisting of none, -O-, -S-, -C(O)-, -S(O)-, -$S(O)_2$-, -N($R^1$)-, -N($R^{s1}$)-, -CH($R^8$)-, -C$(R^{s2})_2$-;
subscript n1 = 0, 1, 2, or 3;
Ring A is an optionally substituted ring selected from the group consisting of $C_{6-10}$ aryl; a 5- to 10-membered heteroaryl; a $C_{6-10\,\text{aryl substituted by a}}$ C3-10 cycloalkyl, a 4- to 10-membered heterocycloalkyl, a C3-10 cycloalkenyl, a 4- to 10-membered heterocycloalkenyl, a $C_{6-10}$ aryl or a 5-to 10-membered heteroaryl ; a 5- to 10-membered heteroaryl substituted by a C3-10 cycloalkyl, a 4- to 10-membered heterocycloalkyl, a C3-10 cycloalkenyl, a 4- to 10-membered heterocycloalkenyl, a $C_{6-10}$ aryl or a 5- to 10-membered heteroaryl; a $C_{6-10}$ aryl fused to $_a$ C3-10 cycloalkyl, a 4- to 10-membered heterocycloalkyl , $_a$ C3-10 cycloalkenyl, a 4- to 10-membered heterocycloalkenyl, a $C_{6-10}$ aryl or a 5- to 10-membered heteroaryl ; 3-10 -membered cycloalkyl, 4- to 10-membered heterocycloalkyl, C3-10 cycloalkenyl, 4- to 10-membered heterocycloalkenyl, $C_{6-10}$ aryl, or 5- to 10-membered heteroaryl-fused 5- to 10-membered heteroaryl;
m1=0, 1, 2, 3, or 4;
each $R^A$ is independently $R^{A1}$ , $R^{s1}$ or $R^{s2}$ ;
each $R^{A1}$ is independently selected from the group consisting of halogen, optionally substituted $C_{1-6}$alkyl, optionally substituted $C_{1-6}$haloalkyl, optionally substituted $C_{1-6}$alkoxy, optionally substituted $C_{1-6}$alkylthio, optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl; or, when two $R^{A1}$ are located on adjacent ring atoms, the two $R^{A1}$ and the ring atoms adjacent thereto together form a ring selected from the group consisting of optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl;
Each of $R^1$ and $R^8$ is independently selected from the group consisting of H, optionally substituted $C_{1-6}$alkyl, optionally substituted $C_{3-6}$ cycloalkyl, halogen, optionally substituted $C_{1-6}$haloalkyl, optionally substituted $C_{1-6\text{alkoxy}}$ , optionally substituted $C_{1-6}$haloalkoxy (-$OC_{1-6}$haloalkyl), optionally substituted $C_{1-6}$alkyl-$OC_{1-6}$alkylene, optionally substituted $C_{1-6}$haloalkyl-$OC_{1-6}$alkylene, optionally substituted $C_{1-6}$haloalkyl-$SC_{1-6}$alkylene, optionally substituted $C_{1-6}$aminoalkyl, optionally substituted C3-10 cycloalkyl, optionally substituted 4 to 10 membered heterocycloalkyl, optionally substituted $C_{6-10}$ aryl, optionally substituted 5 to 10 membered heteroaryl, optionally substituted C3-10 cycloalkenyl, optionally substituted 4 to 10 membered heterocycloalkenyl, optionally substituted C3-10 cycloalkyl-C $_{R1}$ or $^{R8}$ , together with $R^{s1}$ or $R^{s2}$ on ring $_A$ , form an optionally substituted $_{C4-10}$ cycloalkyl or $_{4-10}$ heterocycloalkyl ;
$R^7$ is an optionally substituted group selected from the group consisting of none, $C_{1-6}$alkyl, C3-10 cycloalkyl, 4 to 10-membered heterocycloalkyl, C3-10 cycloalkenyl, 4 to 10-membered heterocycloalkenyl, $C_{6-10}$ aryl, and 5 to 10-membered heteroaryl;
$R^{s1}$ are each independently selected from the group consisting of H, optionally substituted $C_{1-4}$alkyl, optionally

substituted C $_{3-6}$ cycloalkyl, and optionally substituted 4- to 6-membered heterocyclyl;

$R^{s2}$ are each independently selected from the following group: H, optionally substituted $C_{1-4}$alkyl, optionally substituted C $_{3-6}$ cycloalkyl, optionally substituted 4 to 6-membered heterocyclyl; or two $R^{s2}$ and the carbon atom to which they are attached together form an optionally substituted C $_{3-6}$ cycloalkyl or an optionally substituted 4 to 6-membered heterocyclyl;

Unless otherwise defined, the optional substitution refers to unsubstituted or one or more (such as 1, 2, 3 or 4) hydrogen atoms in the group are replaced by an optional Rsubstituent;

R is each independently selected from the group consistingof D, Halogen, C1-6-alkyl,

C1-6-haloalkyl, C1-6-hydroxyalkyl, C2-6 vinyl, C2-6 alkynyl-CN, -OR', $-NO_2$, -NR'R", -SR', -OC(O)R', -C(O)R', $-CO_2R'$, -CONR', -OC(O)NR'R", -NR"C(O)R', -NR"-C(O)NR'R", $-NR"C(O)_2R'$ , -S(O)R', $-S(O)_2R'$, $-S(O)_2NR'R"$ , $NR"S(O)_2R'$, C3-10 cycloalkyl optionally substituted with one or more R'", 4 to 10 membered heterocycloalkyl optionally substituted with one or -more R‴, C3-10 cycloalkyl optionally substituted with one or more R', C3-10 cycloalkyl optionally substituted with one or more R', C3-10 cycloalkyl optionally substituted with one or more R', C3-10 cycloalkyl optionally substituted with one or more R', -C3-10 cycloalkyl optionally substituted with one or more R' , C3-10 cycloalkyl optionally substituted with one or more R', C3-10 cycloalkyl optionally substituted with one or more R' $-C_{1-4}$alkylene C3-10 cycloalkyl optionally substituted by one or more R‴, $-C_{1-4}$alkylene- 4 to 10 -membered heterocycloalkyl optionally substituted by one or more R‴, $C_{1-4}$alkylene-$C_{6-10}$ aryl optionally substituted by one or more R' ", $-C_{1-4}$ alkylene - 5 to 10 -membered heteroaryl optionally substituted by one or more R'" ;

each R' is independently selected from the group consisting of H, D, $C_{1-6}$ alkyl , $C_{1-6}$ haloalkyl, C3-10 cycloalkyl optionally substituted with one or more R‴, 4- to 10-membered heterocycloalkyl optionally substituted with one or more R‴, $C_{6-10}$ aryl optionally substituted with one or more R‴, 5- to 10-membered heteroaryl optionally substituted with one or more R‴, $-C_{1-4}$ alkylene-C3-10 cycloalkyl optionally substituted with one or more R‴ , $-C_{1-4}$ alkylene- 4- to 10- membered heterocycloalkyl optionally substituted with one or more R‴, $-C_{1-4}$ alkylene-$C_{6-10}$ aryl optionally substituted with one or more R‴ , $-C_{1-4}$ alkylene- 5- to 10 -membered heteroaryl optionally substituted with one or more R‴ ;

Each R" is selected from the group consisting of H, D, $C_{1-4}$alkyl, $C_{1-4}$haloalkyl, and C $_{3-4}$ cycloalkyl;

Each R" is independently selected from the group consisting of D, halogen, hydroxy, nitro, CN, C1-6 alkyl, and C1-6 haloalkyl.

**2.** The compound according to claim 1, wherein the compound is as shown in Formula I-1 or Formula I-2

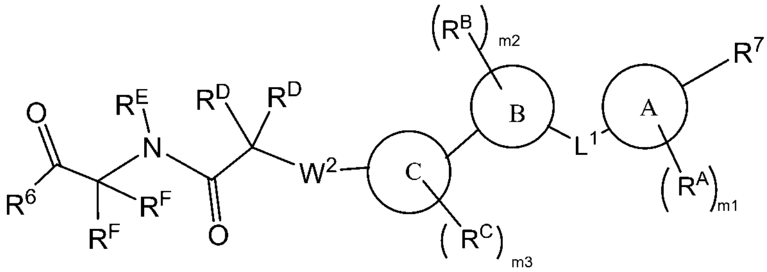

(I-1)

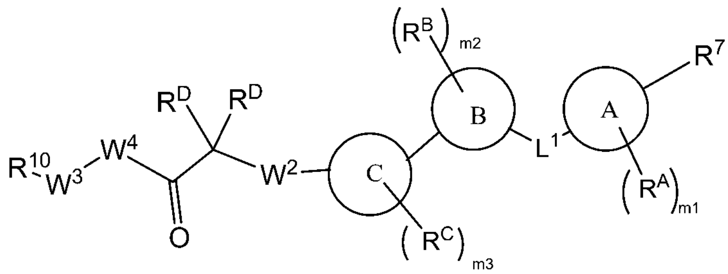

(I-2).

**3.** The compound of claim 1, wherein

(i) Ring B is

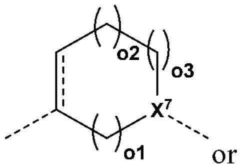 or 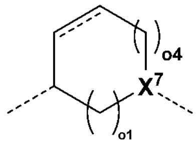 ;

wherein, 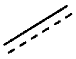 is a single bond or a double bond, X7 is N or CH, o1 is 1 or 2, o2 is 0, 1, 2 or 3, o3 is 0 or 1, and o4 is 0, 1, 2 or 3; and/or

(ii) Ring C is

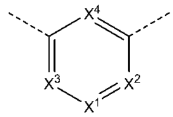 ;

wherein $X^1$ , $X^2$, $X^3$ and $X^4$ are each independently selected from the group consisting of CH and N.

**4.** The compound according to any one of claims 1 to 3, **characterized in that** $R^6$ is $-NR^4R^5$ , $-NR^4R^5$ is a 4- to 10-membered heterocycloalkyl substituted by one or more $R^3$; or, $W^4$ is $-N(R^{s1})-$; $W^3$ is selected from the group consisting of -C(O)-, -S(O)-, and $-S(O)_2-$, and $R^{10}$ is unsubstituted or substituted by one or more RH and selected from the group consisting of a $C_{6-10}$ aryl group, and a 5-to 10-membered heteroaryl group.

**5.** The compound according to any one of claims 1 to 3, wherein the compound has one or more of the following characteristics

(i) Ring A is a ring selected from the group consisting of:

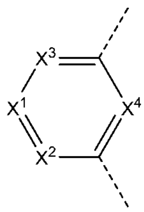 , 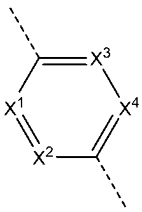 ;

wherein $X^1$ , $X^2$, $X^3$ and $X^4$ are each independently selected from the group consisting of CH, N;

(ii) $R^7$ is selected from the group consisting of:

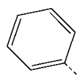 , 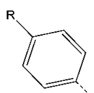 , 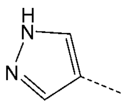 , 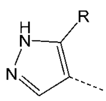 , 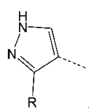 , 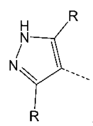 , 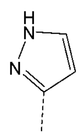 , 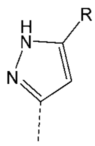 , 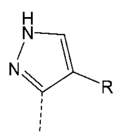 , 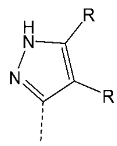 ,

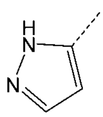 , 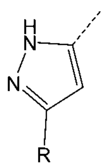 , 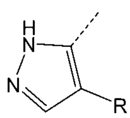 , 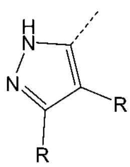 , 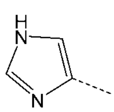 , 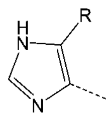 , 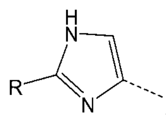 , 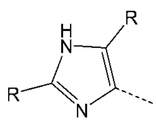 ,

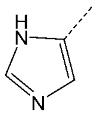 , 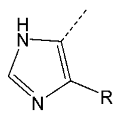 , 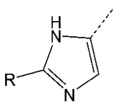 , 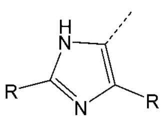 , 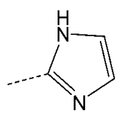 , 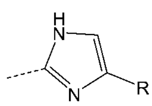 , 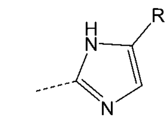

and

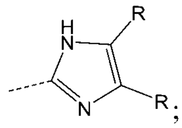

In $R^7$, each **R** is independently selected from the group consisting of $C_{1-6}$alkyl, -NR'R", wherein each R' is independently selected from the group consisting of H, $C_{1-6}$alkyl, and each R" is selected from the group consisting of H, $C_{1-4}$alkyl.

6. The compound according to claim 1 or 2, wherein the compound is selected from Table AI, Table BI and Table CI.

7. A compound or a pharmaceutically acceptable salt thereof, or an isomer, solvate, crystal form or prodrug thereof, wherein the compound is represented by Formula II

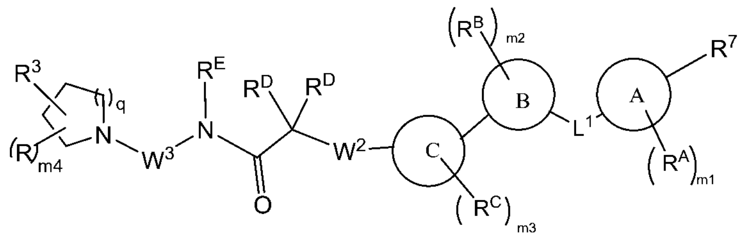

(II)

wherein

q is 0, 1, 2, or 3;
$R^3$ is selected from the group consisting of H, $-OR^{31}$, $-C_{1-4}$alkylene-$OR^{31}$, $-N(R^{32})R^{33}$, $-C_{1-4}$alkylene-$N(R^{31})R^{32}$;
m4 is 0, 1, 2, 3, 4, 5, 6 or 7;
$R^{31}$ is selected from the group consisting of H, optionally substituted $C_{1-4}$alkyl, $R^{34}$, $-C_{1-4}$alkylene-$R^{34}$;
$R^{32}$ is selected from the group consisting of H, optionally substituted $C_{1-4}$alkyl;
$R^{33}$ is selected from the group consisting of H, optionally substituted $C_{1-4}$alkyl, $R^{34}$, $-C_{1-4}$alkylene-$R^{34}$;
$R^{34}$ is selected from the group consisting of C3-10 cycloalkyl, 4- to 8-membered heterocycloalkyl, $C_{6-10}$ aryl, 5- to 10-membered heteroaryl, C3-10 cycloalkenyl, and 4- to 10-membered heterocycloalkenyl; wherein the cycloalkyl, heterocycloalkyl, aryl, heteroaryl, cycloalkenyl, and heterocycloalkenyl groups are optionally substituted with one or more groups selected from the group consisting of $-NH_2$, R;
W3 is selected from the group consisting of -C(O)-, -S(O)-, $-S(O)_{2-}$, $-C(RF)_{2-}$ C(O)-, $-C(RF)_{2-}$ S (O)-, $-C(RF)_{2-}$ $S(O)_{2-}$;
RF are each independently selected from the group consisting of H, optionally substituted $C_{1-4}$ alkyl, optionally substituted $_C$3-10 cycloalkyl, optionally substituted 4 to 10 membered heterocycloalkyl; or two RFs and the carbon atom to which they are attached together form an optionally substituted C3-6 cycloalkyl or an optionally substituted 4 to 6 membered heterocyclyl;
$R^E$ is selected from the group consisting of H, optionally substituted $C_{1-4}$alkyl, optionally substituted C3-10 cycloalkyl, and optionally substituted 4- to 10-membered heterocycloalkyl;
$R^D$ are each independently selected from the group consisting of H, optionally substituted $C_{1-4}$alkyl; or, two $R^D{}_S$ and the carbon atom to which they are attached together form a group selected from the group consisting of optionally substituted C3-10 cycloalkyl, optionally substituted 4 to 10 membered heterocycloalkyl;
W2 is selected from the group consisting of -O-, -S-, and $-N(R^{s1})$-;
Ring C is optionally substituted with a ring selected from the group consisting of $C_{6-10}$ aryl, and 5- to 10-membered heteroaryl;
m3=0, 1, 2, 3, or 4;
Each RC is independently $R^{C1}$ or $R^{s1}$;
Each $R^{C1}$ is independently selected from the group consisting of halogen, optionally substituted $C_{1-6}$alkyl, optionally substituted $C_{1-6}$haloalkyl, hydroxy, and optionally substituted $C_{1-6}$alkoxy, optionally substituted $C_{1-6}$haloalkoxy;
Ring B is an optionally substituted ring selected from the group consisting of $C_{3-12}$ cycloalkyl, 4-to 12-membered

heterocycloalkyl, C3-10 cycloalkenyl, and 4- to 10-membered heterocycloalkyl;

Each RB is independently $R^{B1}$, $R^{s1}$ or $R^{s2}$ ;

each $R^{B1}$ is independently selected from the group consisting of halogen, hydroxy, cyano,

optionally substituted $C_{1-6}$alkyl, optionally substituted $C_{1-6}$alkoxy, optionally substituted $C_{1-6}$alkylthio, optionally substituted C3-10 cycloalkyl, optionally substituted 4- to 10-membered heterocycloalkyl, optionally substituted C3-10 cycloalkenyl, optionally substituted 4- to 10-membered heterocycloalkenyl, optionally substituted $C_{6-10}$ aryl, and optionally substituted 5- to 10-membered heteroaryl;

m2=0, 1, 2, 3, or 4;

L $^{1}$ is -X-(W $^{1}$)$_{n1}$ -;

X is selected from the group consisting of -C(O)-, -S(O)-, and -$S(O)_2$-;

Each W $^{1}$ is independently selected from the group consisting of none, -O-, -S-, -C(O)-, -S(O)-, -$S(O)_2$-, -N($R^{s1}$ )-, -C($R^{s2}$)$_2$-;

Subscript n1 = 0, 1, 2, or 3;

Ring A is an optionally substituted ring selected from the group consisting of $C_{6-10}$ aryl; a 5- to 10-membered heteroaryl; a $C_{6-10\,aryl\,substituted\,by}$ a C3-10 cycloalkyl, a 4- to 10-membered heterocycloalkyl, a C3-10 cycloalkenyl, a 4- to 10-membered heterocycloalkenyl, a $C_{6-10}$ aryl or a 5-to 10-membered heteroaryl ; a 5- to 10-membered heteroaryl substituted by a C3-10 cycloalkyl, a 4- to 10-membered heterocycloalkyl, a C3-10 cycloalkenyl, a 4- to 10-membered heterocycloalkenyl, a $C_{6-10}$ aryl or a 5- to 10-membered heteroaryl; a $C_{6-10}$ aryl fused to $_{a}$ C3-10 cycloalkyl, a 4- to 10-membered heterocycloalkyl , $_{a}$ C3-10 cycloalkenyl, a 4- to 10-membered heterocycloalkenyl, a $C_{6-10}$ aryl or a 5- to 10-membered heteroaryl ; 3-10 -membered cycloalkyl, 4- to 10-membered heterocycloalkyl, C3-10 cycloalkenyl, 4- to 10-membered heterocycloalkenyl, $C_{6-10}$ aryl, or 5- to 10-membered heteroaryl-fused 5- to 10-membered heteroaryl;

m1=0, 1, 2, 3, or 4;

Each $R^{A}$ is independently $R^{A1}$ , $R^{s1}$ or $R^{s2}$;

each $R^{A1}$ is independently selected from the group consisting of halogen, optionally substituted $C_{1-6}$alkyl, optionally substituted $C_{1-6}$haloalkyl, optionally substituted $C_{1-6}$alkoxy, optionally substituted $C_{1-6}$alkylthio, optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl; or, when two $R^{A1}$ are located on adjacent ring atoms, the two $R^{A1}$ and the ring atoms adjacent thereto together form a ring selected from the group consisting of optionally substituted C3-10 cycloalkyl, optionally substituted 4 to 10 membered heterocycloalkyl, optionally substituted C3-10 cycloalkenyl, optionally substituted 4 to 10 membered heterocycloalkenyl, optionally substituted $C_{6-10}$ aryl, optionally substituted 5 to 10 membered heteroaryl;

$R^{7}$ is an optionally substituted group selected from the group consisting of none, $C_{1-6}$alkyl, C3-10 cycloalkyl, 4 to 10-membered heterocycloalkyl, C3-10 cycloalkenyl, 4 to 10-membered heterocycloalkenyl, $C_{6-10}$ aryl, and 5 to 10-membered heteroaryl;

$R^{s1}$ are each independently selected from the group consisting of H, optionally substituted $C_{1-4}$alkyl, optionally substituted C $_{3-6}$ cycloalkyl, and optionally substituted 4- to 6-membered heterocyclyl;

$R^{s2}$ are each independently selected from the following group: H, optionally substituted $C_{1-4}$alkyl, optionally substituted C $_{3-6}$ cycloalkyl, optionally substituted 4 to 6-membered heterocyclyl; or two $R^{s2}$ and the carbon atom to which they are attached together form an optionally substituted C $_{3-6}$ cycloalkyl or an optionally substituted 4 to 6-membered heterocyclyl;

unless otherwise defined, the optional substitution refers to unsubstituted or one or more (such as 1, 2, 3 or 4) hydrogen atoms in the group are replaced by an optional **R** substituent;

R is each independently selected from the group consistingof D, Halogen, C1-6-alkyl, C1-6-haloalkyl, C1-6-hydroxyalkyl, C2-6 vinyl, C2-6 alkynyl-CN, -OR', -$NO_2$, -NR'R", -SR', -OC(O)R', -C(O)R', -$CO_2$R', -CONR', -OC(O)NR'R", -NR"C(O)R', -NR"-C(O)NR'R", -NR"C(O)$_2$R' , -S(O)R', -S(O)$_2$R', -S(O)$_2$NR'R" , NR"S(O)$_2$R', C3-10 cycloalkyl optionally substituted with one or more R‴, 4 to 10 membered heterocycloalkyl optionally substituted with one or -$_{more\ R'''}$ , C3-10 cycloalkyl optionally substituted with one or more R', C3-10 cycloalkyl optionally substituted with one or more R', C3-10 cycloalkyl optionally substituted with one or more R', C3-10 cycloalkyl optionally substituted with one or more R', -C3-10 cycloalkyl optionally substituted with one or more R' , C3-10 cycloalkyl optionally substituted with one or more R', C3-10 cycloalkyl optionally substituted with one or more R' -$C_{1-4}$alkylene-C3-10 cycloalkyl optionally substituted by one or more R‴, -$C_{1-4}$alkylene- 4 to 10 -membered heterocycloalkyl optionally substituted by one or more R‴, -$C_{1-4}$alkylene-$C_{6-10}$ aryl optionally substituted by one or more R' ", -$C_{1-4}$ alkylene - 5 to 10 -membered heteroaryl optionally substituted by one or more R‴ ;

each R' is independently selected from the group consisting of H, D, $C_{1-6}$ alkyl , $C_{1-6}$ haloalkyl, C3-10 cycloalkyl optionally substituted with one or more R‴, $_{4-}$ to 10-membered heterocycloalkyl optionally substituted with one or

more R''', $C_{6-10}$ aryl optionally substituted with one or more R''', 5- to 10-membered heteroaryl optionally substituted with one or more R''', -$C_{1-4}$ alkylene-C3-10 cycloalkyl optionally substituted with one or more R''', -$C_{1-4}$ alkylene- 4- to 10- membered heterocycloalkyl optionally substituted with one or more R''', -$C_{1-4}$ alkylene-$C_{6-10}$ aryl optionally substituted with one or more R''', -$C_{1-4}$ alkylene- 5- to 10 -membered heteroaryl optionally substituted with one or more R''';
Each R" is selected from the group consisting of H, D, $C_{1-4}$alkyl, $C_{1-4}$haloalkyl, and C $_{3-4}$ cycloalkyl;
Each R" is independently selected from the group consisting of D, halogen, hydroxy, nitro, CN, C1-6 alkyl , and C1-6 haloalkyl .

**8.** The compound of claim 1, wherein

the compound is shown in formula II-1

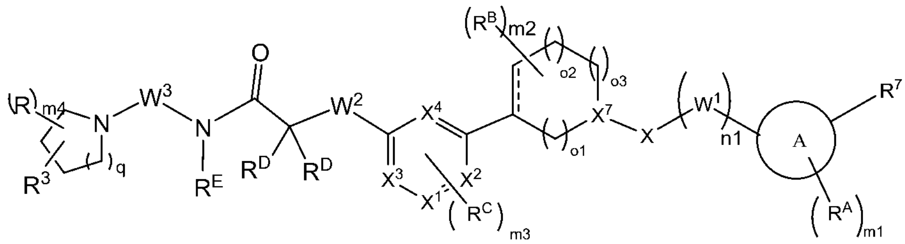
(II-1)

in,

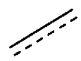

is a single bond or a double bond; X $^7$ is N or CH; o1 is 1 or 2, o2 is 0, 1, 2 or 3, and o3 is 0 or 1;
$X^1$ , $X^2$, $X^3$ and $X^4$ are each independently selected from the group consisting of CH, N; or,
the compound is shown in formula II-2

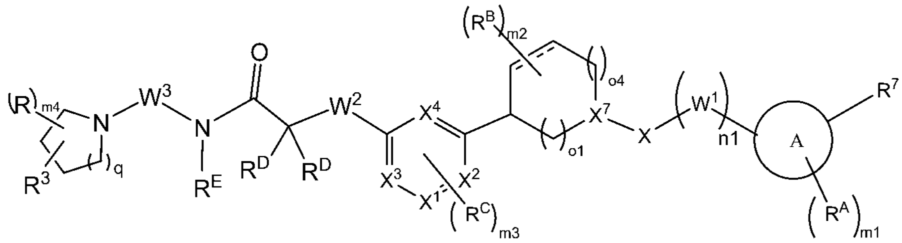
(II-2);

in,

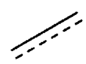

is a single bond or a double bond; X $^7$ is N or CH; o1 is 1 or 2, and o4 is 0, 1, 2 or 3;
$X^1$ , $X^2$, $X^3$ and $X^4$ are each independently selected from the group consisting of CH, N.

**9.** The compound according to claim 7 or 8,

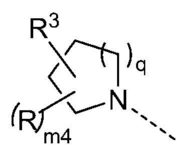

wherein

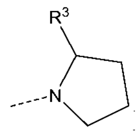

R3 is $-N(R^{32})R^{33}$ or $-C_{1-4}$alkylene $-N(R^{31})R^{32}$; $R^{32}$ and $R^{33}$ are each independently selected from the following group: H, $C_{1-4}$alkyl.

**10.** The compound according to claim 7 or 8, wherein the compound has one or more of the following characteristics:

(a) $R^7$ is selected from the group consisting of:

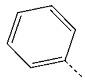, 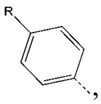, 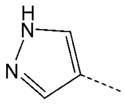, 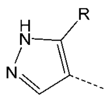, 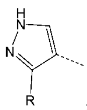, 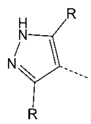, 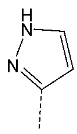, 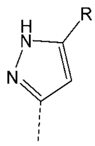, 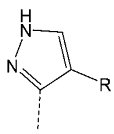, 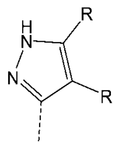,

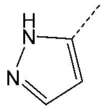, 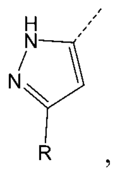, 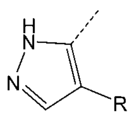, 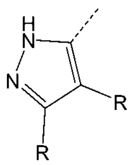, 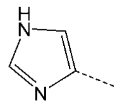, 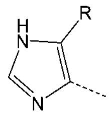, 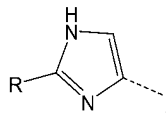, 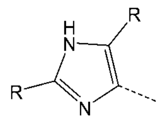,

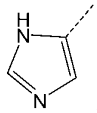, 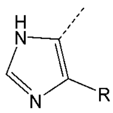, 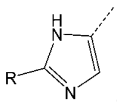, 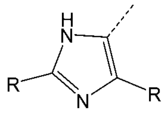, 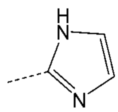, 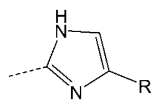, 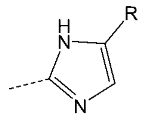

and

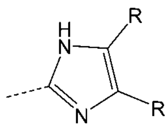;

(b) in $R^7$, each R is independently selected from the group consisting of $C_{1-6}$alkyl, -NR'R", wherein each R' is independently selected from the group consisting of H, $C_{1-6}$alkyl, and each R" is selected from the group consisting of H, $C_{1-4}$alkyl;
(c) Ring A is a ring selected from the group consisting of:

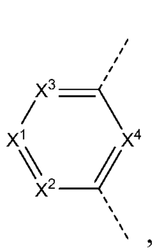, 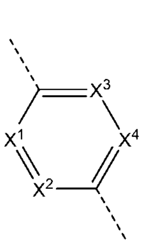;

wherein $X^1$, $X^2$, $X^3$ and $X^4$ are each independently selected from the group consisting of CH, N;
(d) each $R^A$ is independently H, $C_{1-4}$alkyl or $R^{A1}$; and $R^{A1}$ is selected from the group consisting of halogen, optionally substituted $C_{1-6}$alkyl, optionally substituted $C_{1-6}$haloalkyl, and optionally substituted $C_{1-6}$alkoxy;
(e) $W^2$ is -O- or $-N(R^s)-$.

**11.** The compound of claim 1, wherein the compound is selected from Table A-II, Table AB-II, and Table C-II.

**12.** A pharmaceutical composition, which consists of

(i) a compound according to any one of claims 1 to 11 or a pharmaceutically acceptable salt thereof, or an isomer, solvate, crystal form or prodrug thereof; and
(ii) a pharmaceutically acceptable carrier or excipient.

**13.** Use of a compound according to any one of claims 1 to 11 or a pharmaceutically acceptable salt thereof, or an isomer, solvate, crystal form or prodrug thereof in the preparation of a medicament for treating or preventing a disease associated with BCL9/β-catenin interaction.

**14.** The use according to claim 14, wherein the disease associated with the interaction between BCL9 and β-catenin comprises cancer, tumor, or a combination thereof.

**15.** Use of the compound according to any one of claims 1 to 11 or a pharmaceutically acceptable salt thereof, or an isomer, solvate, crystal form or prodrug thereof in the preparation of a medicament for treating or preventing fibrosis or a disease related thereto.

## INTERNATIONAL SEARCH REPORT

International application No. **PCT/CN2024/070917**

**A. CLASSIFICATION OF SUBJECT MATTER**

C07D401/12(2006.01)i; C07D211/22(2006.01)i; C07D211/96(2006.01)i; A61K31/44(2006.01)i; A61K31/454(2006.01)i; A61P35/00(2006.01)i; A61P11/00(2006.01)i; A61P1/16(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT; CNKI; VEN; WOTXT; EPTXT; USTXT; STN; 万方, WANFANG: 江苏明生聚太生物科技有限公司, 王英才, 陈一鸣, 朱文华, B细胞淋巴瘤, 连环蛋白, 抑制剂, 哌啶, 肿瘤, 癌症, 纤维化, BCL9, B-cell lymphoma 9, catenin, Wnt, inhibitor, tumor, cancer, piperidin+, fibro+, 结构式, structure formula

**C. DOCUMENTS CONSIDERED TO BE RELEVANT**

| Category* | Citation of document, with indication, where appropriate, of the relevant passages | Relevant to claim No. |
|---|---|---|
| PX | CN 116162056 A (SHANGHAI INSTITUTE OF PHARMACEUTICAL INDUSTRY et al.) 26 May 2023 (2023-05-26)<br>claims 1-5 and 8-10 | 1-6, 12-15 |
| PX | CN 116444420 A (SHANGHAI INSTITUTE OF PHARMACEUTICAL INDUSTRY CO., LTD.et al.) 18 July 2023 (2023-07-18)<br>claims 1-9 and 11-15 | 1-6, 12-15 |
| PX | ZHANG, Hao et al. "Discovery of Novel 3-Phenylpiperidine Derivatives Targeting the β-Catenin/B-Cell Lymphoma 9 Interaction as a Single Agent and in Combination with the Anti-PD-1 Antibody for the Treatment of Colorectal Cancer"<br>*J. Med. Chem.*, Vol. 66, No. 2, 11 January 2023 (2023-01-11),<br>ISSN: 0022-2623,<br>pages 1349-1379, in particular Table 3 | 1-6, 12-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 March 2024** | **24 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/070917**

**C. DOCUMENTS CONSIDERED TO BE RELEVANT**

| Category* | Citation of document, with indication, where appropriate, of the relevant passages | Relevant to claim No. |
|---|---|---|
| X | WANG, Zhen et al. "Discovery of 2-(3-(3-Carbamoylpiperidin-1-yl)phenoxy)acetic Acid Derivatives as Novel Small-Molecule Inhibitors of the β-Catenin/B-Cell Lymphoma 9 Protein-Protein Interaction"<br>*J. Med. Chem.,* Vol. 64, 27 April 2021 (2021-04-27),<br>ISSN: 0022-2623,<br>abstract, page 5887, left column, last paragraph, Tables 1, 3, and 4 | 1-6, 12-15 |
| A | WANG, Zhen et al. "Discovery of 2-(3-(3-Carbamoylpiperidin-1-yl)phenoxy)acetic Acid Derivatives as Novel Small-Molecule Inhibitors of the β-Catenin/B-Cell Lymphoma 9 Protein-Protein Interaction"<br>*J. Med. Chem.,* Vol. 64, 27 April 2021 (2021-04-27),<br>ISSN: 0022-2623,<br>pages 5886-5904 | 7-11 |
| A | WO 2021055936 A1 (H. LEE MOFFITT CANCER CENTER AND RESEARCH INSTITUTE, INC.) 25 March 2021 (2021-03-25)<br>entire document | 1-15 |

Form PCT/ISA/210 (second sheet) (July 2022)

**INTERNATIONAL SEARCH REPORT**
**Information on patent family members**

International application No.
**PCT/CN2024/070917**

| Patent document cited in search report | | | Publication date (day/month/year) | Patent family member(s) | | | Publication date (day/month/year) |
|---|---|---|---|---|---|---|---|
| CN | 116162056 | A | 26 May 2023 | None | | | |
| CN | 116444420 | A | 18 July 2023 | None | | | |
| WO | 2021055936 | A1 | 25 March 2021 | US | 2022411372 | A1 | 29 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **BERGE, SM et al.** Pharmaceutical Salts. *Journal of Pharmaceutical Science*, 1977, vol. 66, 1-19